# EUROPEAN PATENT SPECIFICATION

(11) **EP 2 413 953 B1**
(45) Date of publication and mention of the grant of the patent: **08.11.2017**
(21) Application number: 10712860.5
(22) Date of filing: 02.04.2010
(51) Int. Cl.: A61K 38/17

(54) **METHODS FOR PREPARING AND USING MULTICHAPERONE-ANTIGEN COMPLEXES**
METHODEN ZUR HERSTELLUNG UND VERWENDUNG VON MULTICHAPERON-ANTIGEN-KOMPLEXEN
PROCÉDÉS DE PRÉPARATION ET D'UTILISATION DE COMPLEXES CHAPERONS MULTIPLES-ANTIGÈNE

(30) Priority: 03.04.2009 US 211850 P
(43) Date of publication of application: 08.02.2012
(73) Proprietor: Agenus Inc., Lexington, MA 02421 (US)
(72) Inventor: LECLAIR, Kenneth, P., Needham MA 02494 (US); TOMLINSON, Andrew, J., Wayland MA 01778 (US)
(74) Representative: Potter Clarkson LLP
(86) International application number: PCT/US2010/029803
(87) International publication number: WO 2010/115118

(56) References cited:
- WO-A1-02/28407
- WO-A2-01/23421
- WO-A2-2004/035602
- CN-A- 1 640 887
- CN-A- 101 297 966
- KING FRANK W ET AL: "Co-chaperones Bag-1, Hop and Hsp40 regulate Hsc70 and Hsp90 interactions with wild-type or mutant p53", EMBO (EUROPEAN MOLECULAR BIOLOGY ORGANIZATION) JOURNAL, vol. 20, no. 22, 15 November 2001 (2001-11-15), pages 6297-6305, XP002639944, ISSN: 0261-4189
- CARRIGAN PATRICIA E ET AL: "Domain : domain interactions within Hop, the Hsp70/Hsp90 organizing protein, are required for protein stability and structure", PROTEIN SCIENCE, vol. 15, no. 3, March 2006 (2006-03), pages 522-532, XP002639945, ISSN: 0961-8368
- ARNOLD-SCHILD DANIELE ET AL: "One-step single-chain Fv recombinant antibody-based purification of gp96 for vaccine development", CANCER RESEARCH, AMERICAN ASSOCIATION FOR CANCER REREARCH, US, vol. 60, no. 15, 1 August 2000 (2000-08-01), pages 4175-4178, XP002175252, ISSN: 0008-5472 cited in the application
- GRANER M ET AL: "Immunoprotective activities of multiple chaperone proteins isolated from murine B-cell leukemia/lymphoma.", CLINICAL CANCER RESEARCH : AN OFFICIAL JOURNAL OF THE AMERICAN ASSOCIATION FOR CANCER RESEARCH MAR 2000 LNKD- PUBMED:10741715, vol. 6, no. 3, March 2000 (2000-03), pages 909-915, XP002639946, ISSN: 1078-0432
- YI ZENG ET AL: "Chaperone-rich cell lysates, immune activation and tumor vaccination", CANCER IMMUNOLOGY, IMMUNOTHERAPY, SPRINGER, BERLIN, DE, vol. 55, no. 3, 1 March 2006 (2006-03-01), pages 329-338, XP019333212, ISSN: 1432-0851, DOI: DOI:10.1007/S00262-005-0694-1
- GONG JIANLIN ET AL: "A Heat Shock Protein 70-Based Vaccine with Enhanced Immunogenicity for Clinical Use", JOURNAL OF IMMUNOLOGY, vol. 184, no. 1, January 2010 (2010-01), pages 488-496, XP002639947, ISSN: 0022-1767
- DATABASE MEDLINE [Online] US NATIONAL LIBRARY OF MEDICINE (NLM), BETHESDA, MD, US; May 2009 (2009-05), ZHAO JIAN-GANG ET AL: "[Preparation of anticolon carcinoma vaccine with rich chaperone peptides and study on its anticancer efficacy].", XP002639948, Database accession no. NLM19434541 & ZHONGHUA WEI CHANG WAI KE ZA ZHI = CHINESE JOURNAL OF GASTROINTESTINAL SURGERY MAY 2009 LNKD- PUBMED:19434541, vol. 12, no. 3, May 2009 (2009-05), pages 290-293, ISSN: 1671-0274

### SEQUENCE LISTING

<110> Antigenics, Inc.
   LeClair, Kenneth P.
   Tomlinson, Andrew J.
<120> METHODS FOR PREPARING AND USING
   MULTICHAPERONE ANTIGEN COMPLEXES
<130> 8449-513-228
<140>
   <141>
<150> 61/211,850
   <151> 2009-04-03
<160> 10
<170> FastSEQ for Windows Version 4.0
<210> 1
   <211> 124
   <212> PRT
   <213> Homo sapiens
<220>
   <223> human HOP TPR1
<400> 1
   Met Glu Gln Val Asn Glu Leu Lys Glu Lys Gly Asn Lys Ala Leu Ser
    1               5                  10                  15
   Val Gly Asn Ile Asp Asp Ala Leu Gln Cys Tyr Ser Glu Ala Ile Lys
               20                  25                  30
   Leu Asp Pro His Asn His Val Leu Tyr Ser Asn Arg Ser Ala Ala Tyr
           35                  40                  45
   Ala Lys Lys Gly Asp Tyr Gln Lys Ala Tyr Glu Asp Gly Cys Lys Thr
       50                  55                  60
   Val Asp Leu Lys Pro Asp Trp Gly Lys Gly Tyr Ser Arg Lys Ala Ala
   65                  70                  75                  80
   Ala Leu Glu Phe Leu Asn Arg Phe Glu Glu Ala Lys Arg Thr Tyr Glu
                   85                  90                  95
   Glu Gly Leu Lys His Glu Ala Asn Asn Pro Gln Leu Lys Glu Gly Leu
               100                 105                 110
   Gln Asn Met Glu Ala Arg His His His His His Gln
           115                 120
<210> 2
   <211> 137
   <212> PRT
   <213> Homo sapiens
<220>
   <223> human HOP TPR2a
<400> 2
   Met Lys Gln Ala Leu Lys Glu Lys Glu Leu Gly Asn Asp Ala Tyr Lys
    1               5                  10                  15
   Lys Lys Asp Phe Asp Thr Ala Leu Lys His Tyr Asp Lys Ala Lys Glu
               20                  25                  30
   Leu Asp Pro Thr Asn Met Thr Tyr Ile Thr Asn Gln Ala Ala Val Tyr
           35                  40                  45
   Phe Glu Lys Gly Asp Tyr Asn Lys Cys Arg Glu Leu Cys Glu Lys Ala
       50                  55                  60
   Ile Glu Val Gly Arg Glu Asn Arg Glu Asp Tyr Arg Gln Ile Ala Lys
   65                  70                  75                  80
   Ala Tyr Ala Arg Ile Gly Asn Ser Tyr Phe Lys Glu Glu Lys Tyr Lys
                   85                  90                  95
   Asp Ala Ile His Phe Tyr Asn Lys Ser Leu Ala Glu His Arg Thr Pro
               100                 105                 110
   Asp Val Leu Lys Lys Cys Gln Gln Ala Glu Lys Ile Leu Lys Glu Gln
           115                 120                 125
   Glu Arg Leu His His His His His Gln
       130                 135
<210> 3
   <211> 358
   <212> PRT
   <213> Homo sapiens
<220>
   <223> human HOP TPR1/2a
<400> 3
   Met Glu Gln Val Asn Glu Leu Lys Glu Lys Gly Asn Lys Ala Leu Ser
    1               5                  10                  15
   Val Gly Asn Ile Asp Asp Ala Leu Gln Cys Tyr Ser Glu Ala Ile Lys
               20                  25                  30
   Leu Asp Pro His Asn His Val Leu Tyr Ser Asn Arg Ser Ala Ala Tyr
           35                  40                  45
   Ala Lys Lys Gly Asp Tyr Gln Lys Ala Tyr Glu Asp Gly Cys Lys Thr
       50                  55                  60
   Val Asp Leu Lys Pro Asp Trp Gly Lys Gly Tyr Ser Arg Lys Ala Ala
   65                  70                  75                  80
   Ala Leu Glu Phe Leu Asn Arg Phe Glu Glu Ala Lys Arg Thr Tyr Glu
                   85                  90                  95
   Glu Gly Leu Lys His Glu Ala Asn Asn Pro Gln Leu Lys Glu Gly Leu
               100                 105                 110
   Gln Asn Met Glu Ala Arg Leu Ala Glu Arg Lys Phe Met Asn Pro Phe
           115                 120                 125
   Asn Met Pro Asn Leu Tyr Gln Lys Leu Glu Ser Asp Pro Arg Thr Arg
       130                 135                 140
   Thr Leu Leu Ser Asp Pro Thr Tyr Arg Glu Leu Ile Glu Gln Leu Arg
   145                 150                 155                 160
   Asn Lys Pro Ser Asp Leu Gly Thr Lys Leu Gln Asp Pro Arg Ile Met
                   165                 170                 175
   Thr Thr Leu Ser Val Leu Leu Gly Val Asp Leu Gly Ser Met Asp Glu
               180                 185                 190
   Glu Glu Glu Ile Ala Thr Pro Pro Pro Pro Pro Pro Pro Lys Lys Glu
           195                 200                 205
   Thr Lys Pro Glu Pro Met Glu Glu Asp Leu Pro Glu Asn Lys Lys Gln
       210                 215                 220
   Ala Leu Lys Glu Lys Glu Leu Gly Asn Asp Ala Tyr Lys Lys Lys Asp
   225                 230                 235                 240
   Phe Asp Thr Ala Leu Lys His Tyr Asp Lys Ala Lys Glu Leu Asp Pro
                   245                 250                 255
   Thr Asn Met Thr Tyr Ile Thr Asn Gln Ala Ala Val Tyr Phe Glu Lys
               260                 265                 270
   Gly Asp Tyr Asn Lys Cys Arg Glu Leu Cys Glu Lys Ala Ile Glu Val
           275                 280                 285
   Gly Arg Glu Asn Arg Glu Asp Tyr Arg Gln Ile Ala Lys Ala Tyr Ala
       290                 295                 300
   Arg Ile Gly Asn Ser Tyr Phe Lys Glu Glu Lys Tyr Lys Asp Ala Ile
   305                 310                 315                 320
   His Phe Tyr Asn Lys Ser Leu Ala Glu His Arg Thr Pro Asp Val Leu
                   325                 330                 335
   Lys Lys Cys Gln Gln Ala Glu Lys Ile Leu Lys Glu Gln Glu Arg Leu
               340                 345                 350
   His His His His His His
           355
<210> 4
   <211> 125
   <212> PRT
   <213> Homo sapiens
<220>
   <223> human HOP TPR2b
<400> 4
   Ala Tyr Ile Asn Pro Asp Leu Ala Leu Glu Glu Lys Asn Lys Gly Asn
    1              5                   10                  15
   Glu Cys Phe Gln Lys Gly Asp Tyr Pro Gln Ala Met Lys His Tyr Thr
               20                  25                  30
   Glu Ala Ile Lys Arg Asn Pro Lys Asp Ala Lys Leu Tyr Ser Asn Arg
           35                  40                  45
   Ala Ala Cys Tyr Thr Lys Leu Leu Glu Phe Gln Leu Ala Leu Lys Asp
       50                  55                  60
   Cys Glu Glu Cys Ile Gln Leu Glu Pro Thr Phe Ile Lys Gly Tyr Thr
   65                  70                  75                  80
   Arg Lys Ala Ala Ala Leu Glu Ala Met Lys Asp Tyr Thr Lys Ala Met
                   85                  90                  95
   Asp Val Tyr Gln Lys Ala Leu Asp Leu Asp Ser Ser Cys Lys Glu Ala
               100                 105                 110
   Ala Asp Gly Tyr Gln Arg Cys Met Met Ala Gln Tyr Asn
           115                 120                 125
<210> 5
   <211> 375
   <212> DNA
   <213> Artificial Sequence
<220>
   <223> cDNA construct of HOP TPR1
<400> 5
   atggagcagg tcaatgagct gaaggagaaa ggcaacaagg ccctgagcgt gggtaacatc 60
   gatgatgcct tacagtgcta ctccgaagct attaagctgg atccccacaa ccacgtgctg 120
   tacagcaacc gttctgctgc ctatgccaag aaaggagact accagaaggc ttatgaggat 180
   ggctgcaaga ctgtcgacct aaagcctgac tggggcaagg gctattcacg aaaagcagca 240
   gctctagagt tcttaaaccg ctttgaagaa gccaagcgaa cctatgagga gggcttaaaa 300
   cacgaggcaa ataaccctca actgaaagag ggtttacaga atatggaggc caggcaccac 360
   catcaccatc agtag                                                  375
<210> 6
   <211> 414
   <212> DNA
   <213> Artificial Sequence
<220>
   <223> cDNA construct of HOP TPR2a
<400> 6
   atgaagcagg cactgaaaga aaaagagctg gggaacgatg cctacaagaa gaaagacttt 60
   gacacagcct tgaagcatta cgacaaagcc aaggagctgg accccactaa catgacttac 120
   attaccaatc aagcagcggt atactttgaa aagggcgact acaataagtg ccgggagctt 180
   tgtgagaagg ccattgaagt ggggagagaa aaccgagaag actatcgaca gattgccaaa 240
   gcgtatgctc gaattggcaa ctcctacttc aaagaagaaa agtacaagga tgccatccat 300
   ttctataaca agtctctggc agagcaccga accccagatg tgctcaagaa atgccagcag 360
   gcagagaaaa tcctgaagga gcaagagcgg ctgcaccacc atcaccatca gtag       414
<210> 7
   <211> 1077
   <212> DNA
   <213> Artificial Sequence
<220>
   <223> cDNA construct of HOP TPR1/2a
<400> 7
   atggagcagg tcaatgagct gaaggagaaa ggcaacaagg ccctgagcgt gggtaacatc 60
   gatgatgcct tacagtgcta ctccgaagct attaagctgg atccccacaa ccacgtgctg 120
   tacagcaacc gttctgctgc ctatgccaag aaaggagact accagaaggc ttatgaggat 180
   ggctgcaaga ctgtcgacct aaagcctgac tggggcaagg gctattcacg aaaagcagca 240
   gctctagagt tcttaaaccg ctttgaagaa gccaagcgaa cctatgagga gggcttaaaa 300
   cacgaggcaa ataaccctca actgaaagag ggtttacaga atatggaggc caggttggca 360
   gagagaaaat tcatgaaccc tttcaacatg cctaatctgt atcagaagtt ggagagtgat 420
   cccaggacaa ggacactact cagtgatcct acctaccggg agctgataga gcagctacga 480
   aacaagcctt ctgacctggg cacgaaacta caagatcccc ggatcatgac cactctcagc 540
   gtcctccttg gggtcgatct gggcagtatg gatgaggagg aagagattgc aacacctcca 600
   ccaccacccc ctcccaaaaa ggagaccaag ccagagccaa tggaagaaga tcttccagag 660
   aataagaagc aggcactgaa agaaaaagag ctggggaacg atgcctacaa gaagaaagac 720
   tttgacacag ccttgaagca ttacgacaaa gccaaggagc tggaccccac taacatgact 780
   tacattacca atcaagcagc ggtatacttt gaaaagggcg actacaataa gtgccgggag 840
   ctttgtgaga aggccattga agtggggaga gaaaaccgag aagactatcg acagattgcc 900
   aaagcgtatg ctcgaattgg caactcctac ttcaaagaag aaaagtacaa ggatgccatc 960
   catttctata acaagtctct ggcagagcac cgaaccccag atgtgctcaa gaaatgccag 1020
   caggcagaga aaatcctgaa ggagcaagag cggctgcacc accatcacca tcactag    1077
<210> 8
   <211> 543
   <212> PRT
   <213> Homo sapiens
<220>
   <223> human HOP protein (full length)(GenBank Accession No. P31948)
<400> 8
   Met Glu Gln Val Asn Glu Leu Lys Glu Lys Gly Asn Lys Ala Leu Ser
    1               5                  10                  15
   Val Gly Asn Ile Asp Asp Ala Leu Gln Cys Tyr Ser Glu Ala Ile Lys
               20                  25                  30
   Leu Asp Pro His Asn His Val Leu Tyr Ser Asn Arg Ser Ala Ala Tyr
           35                  40                  45
   Ala Lys Lys Gly Asp Tyr Gln Lys Ala Tyr Glu Asp Gly Cys Lys Thr
       50                  55                  60
   Val Asp Leu Lys Pro Asp Trp Gly Lys Gly Tyr Ser Arg Lys Ala Ala
   65                  70                  75                  80
   Ala Leu Glu Phe Leu Asn Arg Phe Glu Glu Ala Lys Arg Thr Tyr Glu
                   85                  90                  95
   Glu Gly Leu Lys His Glu Ala Asn Asn Pro Gln Leu Lys Glu Gly Leu
               100                 105                 110
   Gln Asn Met Glu Ala Arg Leu Ala Glu Arg Lys Phe Met Asn Pro Phe
           115                 120                 125
   Asn Met Pro Asn Leu Tyr Gln Lys Leu Glu Ser Asp Pro Arg Thr Arg
       130                 135                 140
   Thr Leu Leu Ser Asp Pro Thr Tyr Arg Glu Leu Ile Glu Gln Leu Arg
   145                 150                 155                 160
   Asn Lys Pro Ser Asp Leu Gly Thr Lys Leu Gln Asp Pro Arg Ile Met
                   165                 170                 175
   Thr Thr Leu Ser Val Leu Leu Gly Val Asp Leu Gly Ser Met Asp Glu
               180                 185                 190
   Glu Glu Glu Ile Ala Thr Pro Pro Pro Pro Pro Pro Pro Lys Lys Glu
           195                 200                 205
   Thr Lys Pro Glu Pro Met Glu Glu Asp Leu Pro Glu Asn Lys Lys Gln
       210                 215                 220
   Ala Leu Lys Glu Lys Glu Leu Gly Asn Asp Ala Tyr Lys Lys Lys Asp
   225                 230                 235                 240
   Phe Asp Thr Ala Leu Lys His Tyr Asp Lys Ala Lys Glu Leu Asp Pro
                   245                 250                 255
   Thr Asn Met Thr Tyr Ile Thr Asn Gln Ala Ala Val Tyr Phe Glu Lys
               260                 265                 270
   Gly Asp Tyr Asn Lys Cys Arg Glu Leu Cys Glu Lys Ala Ile Glu Val
           275                 280                 285
   Gly Arg Glu Asn Arg Glu Asp Tyr Arg Gln Ile Ala Lys Ala Tyr Ala
       290                 295                 300
   Arg Ile Gly Asn Ser Tyr Phe Lys Glu Glu Lys Tyr Lys Asp Ala Ile
   305                 310                 315                 320
   His Phe Tyr Asn Lys Ser Leu Ala Glu His Arg Thr Pro Asp Val Leu
                   325                 330                 335
   Lys Lys Cys Gln Gln Ala Glu Lys Ile Leu Lys Glu Gln Glu Arg Leu
               340                 345                 350
   Ala Tyr Ile Asn Pro Asp Leu Ala Leu Glu Glu Lys Asn Lys Gly Asn
           355                 360                 365
   Glu Cys Phe Gln Lys Gly Asp Tyr Pro Gln Ala Met Lys His Tyr Thr
       370                 375                 380
   Glu Ala Ile Lys Arg Asn Pro Lys Asp Ala Lys Leu Tyr Ser Asn Arg
   385                 390                 395                 400
   Ala Ala Cys Tyr Thr Lys Leu Leu Glu Phe Gln Leu Ala Leu Lys Asp
                   405                 410                 415
   Cys Glu Glu Cys Ile Gln Leu Glu Pro Thr Phe Ile Lys Gly Tyr Thr
               420                 425                 430
   Arg Lys Ala Ala Ala Leu Glu Ala Met Lys Asp Tyr Thr Lys Ala Met
           435                 440                 445
   Asp Val Tyr Gln Lys Ala Leu Asp Leu Asp Ser Ser Cys Lys Glu Ala
       450                 455                 460
   Ala Asp Gly Tyr Gln Arg Cys Met Met Ala Gln Tyr Asn Arg His Asp
   465                 470                 475                 480
   Ser Pro Glu Asp Val Lys Arg Arg Ala Met Ala Asp Pro Glu Val Gln
                   485                 490                 495
   Gln Ile Met Ser Asp Pro Ala Met Arg Leu Ile Leu Glu Gln Met Gln
               500                 505                 510
   Lys Asp Pro Gln Ala Leu Ser Glu His Leu Lys Asn Pro Val Ile Ala
           515                 520                 525
   Gln Lys Ile Gln Lys Leu Met Asp Val Gly Leu Ile Ala Ile Arg
       530                 535                 540
<210> 9
   <211> 104
   <212> PRT
   <213> Homo sapiens
<220>
   <223> DP1 of human HOP protein
<400> 9
   Leu Ala Glu Arg Lys Phe Met Asn Pro Phe Asn Met Pro Asn Leu Tyr
    1               5                  10                  15
   Gln Lys Leu Glu Ser Asp Pro Arg Thr Arg Thr Leu Leu Ser Asp Pro
               20                  25                  30
   Thr Tyr Arg Glu Leu Ile Glu Gln Leu Arg Asn Lys Pro Ser Asp Leu
           35                  40                  45
   Gly Thr Lys Leu Gln Asp Pro Arg Ile Met Thr Thr Leu Ser Val Leu
       50                  55                  60
   Leu Gly Val Asp Leu Gly Ser Met Asp Glu Glu Glu Glu Ile Ala Thr
   65                  70                  75                  80
   Pro Pro Pro Pro Pro Pro Pro Lys Lys Glu Thr Lys Pro Glu Pro Met
                   85                  90                  95
   Glu Glu Asp Leu Pro Glu Asn Lys
               100
<210> 10
   <211> 66
   <212> PRT
   <213> Homo sapiens
<220>
   <223> DP2 of human HOP protein
<400> 10
   Arg His Asp Ser Pro Glu Asp Val Lys Arg Arg Ala Met Ala Asp Pro
    1               5                  10                  15
   Glu Val Gln Gln Ile Met Ser Asp Pro Ala Met Arg Leu Ile Leu Glu
               20                  25                  30
   Gln Met Gln Lys Asp Pro Gln Ala Leu Ser Glu His Leu Lys Asn Pro
           35                  40                  45
   Val Ile Ala Gln Lys Ile Gln Lys Leu Met Asp Val Gly Leu Ile Ala
       50                  55                  60
   Ile Arg
   65

## Description

### 1. INTRODUCTION

The present disclosure relates to methods for preparing and using multichaperone-antigen complexes.

### 2. BACKGROUND

### 2.1. HEAT SHOCK PROTEINS

Heat shock proteins (HSPs), also referred to as HSPs, stress proteins, or chaperones, were first identified as proteins synthesized by cells in response to heat shock. HSPs have been classified into five families, based on molecular weight, HSP100, HSP90, HSP70, HSP60, and smHSP. Many members of these families were found subsequently to be induced in response to other stressful stimuli including nutrient deprivation, metabolic disruption, oxygen radicals, and infection with intracellular pathogens (see Welch, May 1993, Scientific American 56-64; Young, 1990, Annu. Rev. Immunol. 8:401-420; Craig, 1993, Science 260:1902-1903; Gething et al., 1992, Nature 355:33-45; and Lindquist et al., 1988, Annu. Rev. Genetics 22:631-677). These families also contain constitutively expressed homologs of the induced proteins.

Studies on the cellular response to heat shock and other physiological stresses revealed that the HSPs are involved not only in cellular protection against these adverse conditions, but also in essential biochemical and immunological processes in unstressed cells. HSPs accomplish different kinds of chaperoning functions. For example, members of the HSP70 family, located in the cell cytoplasm, nucleus, mitochondria, or endoplasmic reticulum (Lindquist et al., 1988, Ann. Rev. Genetics 22:631-677), are involved in the presentation of antigens to the cells of the immune system (Srivastava Ann. Rev. Immunol 2002, 20:395-425) and are also involved in the transfer, folding and assembly of proteins in normal cells. HSPs are capable of binding proteins or peptides, and releasing the bound proteins or peptides in the presence of adenosine triphosphate (ATP) or acidic conditions (Udono and Srivastava, 1993, J. Exp. Med. 178:1391-1396).

The realization that HSPs play a role in immunity generated interest in their use to modulate the immune response. For example, the use of heat shock proteins as adjuvants to stimulate an immune response was proposed by Young in PCT International Application Pub. No. WO 94/29459 and by Edgington in Bio/Technol. 13:1442-1444 (1995), and references infra. One of the best known adjuvants, Freund's complete adjuvant, contains a mixture of heat shock proteins derived from mycobacteria, the genus of the bacterium which causes tuberculosis. Freund's complete adjuvant is generally useful for boosting the immune response to non-mycobacterial antigens. A number of references suggest, inter alia, the use of isolated mycobacterial heat shock proteins for a similar purpose, including vaccination against tuberculosis itself (Lukacs et al., 1993, J. Exp. Med. 178:343-348; Lowrie et al., 1994, Vaccine 12:1537-1540; Silva and Lowrie, 1994, Immunology 82:244-248; Lowrie et al., 1995, J. Cell. Biochem. Suppl. 0(19b):220; Retzlaff et al., 1994, Infect. Immun. 62:5689-5693; PCT International Application Pub. No. WO 94/11513 by the Medical Research Council, Colston et al., inventors; PCT International Application Pub. No. WO 93/1771 by Biocine Sclavo Spa, Rappuoli et al., inventors).

Another approach is to produce covalent complexes of HSP and peptide antigen. For example, a synthetic peptide comprising multiple iterations of the malarial antigen, asparagine-alanine-asparagine-proline, was chemically cross-linked to glutaraldehyde-fixed mycobacterial HSP65 or HSP70 and demonstrated to induce antibodies against the antigen in the absence of adjuvant. A similar effect was observed using HSP from the bacterium *Escherichia coli.* Cross-linking of synthetic peptide to heat shock protein and possibly glutaraldehyde fixation was required for antibody induction. (See Del Guidice, Experientia 50:1061-1066 (1994); Barrios et al., Clin. Exp. Immunol. 98:224-228, 229-233 (1994); Barrios et al., Eur. J. Immunol. 22:1365-1372 (1992)). Alternatively, the HSP can be covalently linked to an antigen by producing a fusion protein as described by Young in European Patent No. EP0700445, also published as PCT International Application Pub. No. WO 94/29459. Young describes an effective amount of HSPs for use as vaccines or adjuvants to elicit specific immunity to the HSPs, or to substances conjugated to them, is in the range of 0.1 to 1000 micrograms of HSP per injection, citing Lussow, A.R., et al., Eur. J. Immun., 21:2297-2302 (1991) and Barrios, C. et al., Eur. J. Immun., 22:1365-1372 (1992).

Additionally, autologous, or even endogenous, heat shock proteins can be used to elicit a specific immune response against a target antigen. For example, Srivastava describes noncovalent complexes of HSPs and peptide, purified from cancer cells, that can be used for the treatment and prevention of cancer in PCT International Application Pub. Nos. WO 96/10411, published April 11, 1996 and WO 97/10001, published March 20, 1997; and also in U.S. Patent Nos. 5,750,119, and 5,837,251 issued May 12, 1998 and November 17, 1998, respectively. Similarly, noncovalent complexes of HSPs and peptide, purified from pathogen-infected cells, have been described for use in the treatment and prevention of infection caused by the pathogen, such as a virus or bacteria, in PCT International Application Pub. No. WO 95/24923, published September 21, 1995. The HSP-antigen complexes can also be prepared in vitro and used for the treatment and prevention of cancer and infectious diseases as described in PCT International Application Pub. No. WO 97/100000, published March 20, 1997, and in U.S. Patent No. 6,030,618 issued February 29, 2000. Srivastava also describes the use of HSP-antigen complexes for sensitizing antigen presenting cells in vitro for use in adoptive immunotherapy in PCT International Application Pub. No. WO 97/10002, published March 20, 1997, and in U.S. Patent No. 5,985,270, issued November 16, 1999.

### 2.2. HSP70/HSP90 ORGANIZING PROTEINS (HOPS)

HSP70/HSP90 Organizing Protein (also known as HOP, STI1, STIP1, p60) was first identified in immunoaffinity purification of HSP90 from chicken oviduct cytosol (Smith et al., 1993, Mol. & Cell. Biology, 13:869-876). This protein was similarly co-purified with HSP90 from different chicken tissues, and rabbit, rat, xenopus and human tissue lysates (Smith et al., 1993, Mol. & Cell. Biology, 13:869-876). Consistent with its functionality (mediation of HSP70 and HSP90 interaction), HSP70 was also detected in these immunoprecipitates (Smith et al., 1993, Mol. & Cell. Biology, 13:869-876). Subsequently, HOP was shown to stimulate folding of thermally denatured firefly luciferase by HSP70 (Johnson et al., 1998, JBC, 273: 3679-3686). This reaction was aided by the addition of HSP90, an experiment that confirmed that HOP provides a physical link between both chaperones (Johnson et al., 1998, JBC, 273:3679-3686). Although somewhat controversial HOP has also been reported to be an essential component in the assembly of steroid receptor complexes, with its addition increasing steroid binding activity of *in vitro* models (reviewed in Pratt et al., 2003, EXP. Biol. Med. 228:111-133). The HOP ortholog STI1 is expressed in yeast (Nicolet et al., 1998, Mol. Cell. Biol. 9:3638-3646).

Structurally, HOP is comprised of three-tetratricopeptide repeat (TPR) and two aspartic acid/proline rich (DP) domains (TPR1-DP1-TPR2a-TPR2b-DP2), with a molecular weight of 62 kDa (Scheufler et al., 2000, Cell. 101: 199-210; Carrigan et al., 2004, JBC, 279:16185-16193; Cortajarena et al., 2006, Protein Science. 15:1193-1198; Flom et al., 2007, Biochem, J. 404:159-167). This protein forms a homodimer through interactions within the TPR2a domain (Cortajarena et al., 2006, Protein Science, 15:1193-1198). Although there is reported cooperation by several domains, TPR1 has been shown to bind HSP70, and TPR2a interacts with HSP90 (Scheufler et al., 2000, Cell. 101:199-210; Carrigan et al., 2004, JBC. 279: 16185-16193; Nelson et al., 2003, Cell Stress and Chaperones, 8:125-133). Both of these TPR domains form structures that comprise seven alpha helices that together give rise to a grove that interacts with the carboxy-terminal GPTIEEVD sequence of HSP70 (TPR1) or the carboxy-terminal MEEVD sequence of HSP90 (TPR2a) through carboxylate clamps (Scheufler et al., 2000, Cell. 101:199-210; Carrigan et al., 2004, JBC. 279:16185-16193). It has been shown that HOP selectively binds the ADP-bound state of HSP70 and stimulates ATPase activity of this chaperone, which implies that this adaptor molecule interacts with the substrate bound chaperone (Johnson et al., 1998, JBC. 273:3679-3686; Wegele et al., 2006 J. Mol. Biol. 356:802-811). In contrast, HOP inhibits ATP binding and ATPase activity of HSP90, and efficiently prevents binding of the co-chaperone p23 to HSP90 (reviewed in Pratt et al., 2003, EXP. Biol. Med. 228:111-133). Combined these data indicate that HOP facilitates HSP90 binding to HSP70/HSP40 complexes that have bound a target protein and transfer of such substrates to HSP90 (Pratt et al., 2003, EXP. Biol. Med. 228:111-133; Wegele et al., 2006 J. Mol. Biol. 356:802-811).
WO 2004/035602A2 relates to a method of improving or prolonging a subject's immune response to a vaccine composition comprising heat shock protein (HSP)-peptide complexes or alpha-2-macro globulin (alpha2M)-peptide complexes.

WO 01/23421A2 relates to pharmaceutical compositions comprising a stress protein complex and related molecules encoding or cells presenting such a complex.

CN 101297966A relates to a tumour vaccine of intestinal cancer, which is a compound rich in chaperone molecules-antigenic peptide, and a preparation method thereof. The tumour vaccine is a compound which is rich in protein molecules combined with antigenic peptide, and the compound is the tumour vaccine of the intestinal cancer.

King et al, 2001 (EMBO J, 20(22): 6297-6305) relates to identification of intermediate reactions that lead to the assembly of molecular chaperone complexes with wild-type or mutant p53R175H protein.

Carrigan et al, 2006 (Protein Science, 15(3): 522-532) relates to Hop global conformation being destabilised by single point mutations in carboxylate clamp positions at pH 5, while the structure of individual TPR domains is unaffected. It also relates to binding of peptides corresponding to the C termini of Hsp70 and Hsp90 altering the global conformation of wild-type Hop, whereas peptide binding does not alter conformation of individual TPR domains.

Graner et al, 2000 (Clin. Cancer Res., 6(3): 909-915) relates to a method for purification of the chaperone proteins grp94/gp96, HSP90, HSP70, and calreticulin from harvested A20 murine leukemia/lymphoma tumor material.

Zeng et al, 2006 (Cancer Immunol. Immunother., 55(3): 329-338) relates to use of a free-solution-isoelectric focusing technique (FS-IEF) to obtain chaperone-rich cell lysates (CRCL) fractions from clarified tumor homogenates.

WO 02/28407A1 relates to a method of purifying heat shock protein complexes using heparin affinity chromatography.

Gong et al, 2010 (J. Immunol., 184(1): 488-496) relates to an approach to preparation of HSP-based vaccines using DC/tumor fusion technology and gentle and rapid isolation of HSP peptide complexes.

Gang et al, 2009 (Chinese Journal of Gastrointestinal Surgery, 12(3): 290-293) relates to preparation of anticolon carcinoma vaccine with rich chaperone peptides and study on its anticancer efficacy.

### 3. SUMMARY OF THE DISCLOSURE

The present invention is defined in the accompanying claims and relates to methods for preparing and using multichaperone-antigen complexes.

Embodiments of the invention are described in the following numbered paragraphs:
(1) A method for preparing multichaperone-antigen complexes which comprise a combination of at least two different heat shock proteins selected from the group consisting of HSP40, HSP70, HSP90, HSP110, HIP, BIP, and calreticulin, said method comprising the steps of:
   (a) contacting a biological sample with a solid phase to which HOP affinity molecules are covalently bound, under conditions such that multichaperone-antigen complexes in the biological sample bind said HOP affinity molecules, wherein the HOP affinity molecules comprise HOP TPR1, having an amino acid sequence as set forth in SEQ ID NO: 1, HOP TPR2a, having an amino acid sequence as set forth in SEQ ID NO: 2, HOP TPR1/2a, having an amino acid sequence as set forth in SEQ ID NO: 3, or a combination or variant of any one or more of the foregoing, wherein a variant is a HOP affinity molecule defined above containing deletions, insertions, substitutions or other modifications relative to the native HOP affinity molecule sequence and retains the specificity of the native HOP affinity molecule to bind heat shock proteins;
   (b) removing unbound components in the biological sample away from the solid phase;
   (c) eluting multichaperone-antigen complexes from the solid phase; and
   (d) recovering the eluted multichaperone-antigen complexes.
(2) The method of paragraph 1, wherein the biological sample is a mammalian cell extract, a human cell extract, a tumor cell extract, an infected cell extract, an extract of an engineered cell, or wherein the biological sample is flow-through resulting from contacting a tumor cell extract, a pathogen-infected cell extract or an extract of cells transfected with and expressing a nucleic acid encoding a tumor associated antigen or a tumor specific antigen or infectious disease antigen, containing cellular proteins, with a solid phase to which is bound a binding partner for a heat shock protein.
(3) The method of paragraph 2, wherein said solid phase to which is bound said binding partner is an anti-gp96 immunoaffinity column and said heat shock protein to which said binding partner binds is gp96.
(4) The method of any one of paragraphs 1 to 3, wherein the multichaperone-antigen complexes comprise, human heat shock proteins.
(5) The method of any one of paragraphs 1 to 4, wherein the solid phase
   (i) comprises beads; or
   (ii) is a membrane; or
   (iii) has a surface comprising polycarbonate, polystyrene, polypropylene, polyethylene, glass, nitrocellulose, dextran, nylon, polyacrylamide or agarose; or
   (iv) is a mixed resin bed comprising a first bead/resin to which a HOP affinity molecule comprising HOP TPR1 or a variant thereof is covalently bound and a second bead/resin to which a HOP affinity molecule comprising HOP TPR1/2a or a variant thereof is covalently bound; or
   (v) is a mixed resin bed comprising (a) a HOP affinity molecule comprising HOP TPR1 or a variant thereof; and (b) a HOP affinity molecule comprising HOP TPR1/2a or a variant thereof; and wherein the eluting step comprises eluting with a buffered solution containing 20 mM Tris and 500 mM NaCl, at pH 9.
(6) The method of paragraph 5, wherein the beads of (i) are packed in a column or are magnetic.
(7) The method of paragraph 5, wherein the beads of (i) are used in a slurry.
(8) The method of any one of paragraphs 1 to 7, wherein said HOP affinity molecules
   (i) are attached via a bifunctional crosslinker to the solid phase; or
   (ii) comprise a HOP affinity fragment or variant thereof selected from the group consisting of HOP TPR1 or a variant thereof, HOP TPR2a or a variant thereof, HOP TPR1/2a or a variant thereof, and a combination of any one or more of the foregoing; or
   (iii) comprise a mammalian HOP affinity fragment or variant thereof; or
   (iv) comprise a human HOP affinity fragment or variant thereof; or
   (v) comprise a HOP affinity fragment or variant thereof that is present as a concatamer of two or more of HOP TPR1 or a variant thereof, HOP TPR2a or a variant thereof, and/or HOP TPR1/2a or a variant thereof; or
   (vi) comprise a HOP affinity fragment or variant thereof that is present as a fusion protein of two or more of HOP TPR1 or a variant thereof, HOP TPR2a or a variant thereof, and/or HOP TPR1/2a or a variant thereof; or
   (vii) do not comprise a wild-type HOP protein.
(9) The method of any one of paragraphs 1 to 8, wherein
   (i) the eluting step comprises eluting with a buffered solution containing 150 mM to 1.5M sodium chloride at pH 3 to pH 11; or
   (ii) the HOP affinity molecule comprises HOP TPR1 or a variant thereof and the eluting step comprises eluting with a buffered solution containing 500 mM NaCl at pH 9; or
   (iii) the HOP affinity molecule comprises HOP TPR2a or a variant thereof and the eluting step comprises eluting with a buffered solution containing 300 mM NaCl at pH 7.2; or
   (iv) the wherein the HOP affinity molecule comprises HOP TPR1/2a or a variant thereof and the eluting step comprises eluting with a buffered solution containing 500 mM NaCl at pH 7.2; or
   (v) the HOP affinity molecule comprises HOP TPR1/2a or a variant thereof and the eluting step comprises eluting with a buffered solution containing 500 mM NaCl at pH 9.
(10) The method of any one of paragraphs 1 to 9, further comprising combining the recovered multichaperone-antigen complexes with purified heat shock protein-antigen complexes or purified gp96-antigen complexes.
(11) A method comprising
   (i) contacting a first solid phase to which is bound a binding partner for a heat shock protein with a human tumor cell extract or human infected cell extract or an extract of cells transfected with and expressing a nucleic acid encoding a tumor associated antigen or a tumor specific antigen or infectious disease antigen under conditions such that gp96-antigen complexes in the extract bind the first solid phase, wherein the first solid phase is an anti-gp96 immunoaffinity column or an anti-gp96 scFv column;
   (ii) collecting the flow through from said first solid phase;
   (iii) washing said first solid phase;
   (iv) eluting gp96- antigen complexes from said first solid phase;
   (v) contacting said flow through collected in step (ii) with a second solid phase to which HOP affinity molecules are covalently bound, under conditions such that multichaperone-antigen complexes in the biological sample bind said HOP affinity molecules, wherein the multichaperone-antigen complexes comprise a combination of at least two different heat shock proteins selected from the group consisting of HSP40, HSP70, HSP90, HSP110, HIP, BIP, and calreticulin; and wherein the HOP affinity molecules comprise HOP TPR1, having an amino acid sequence as set forth in SEQ ID NO: 1, HOP TPR2a, having an amino acid sequence as set forth in SEQ ID NO: 2, HOP TPR1/2a, having an amino acid sequence as set forth in SEQ ID NO: 3, or a combination or variant of any one or more of the foregoing wherein a variant is a HOP affinity molecule defined above containing deletions, insertions, substitutions or other modifications relative to the native HOP affinity molecule sequence and retains the specificity of the native HOP affinity molecule to bind heat shock proteins;
   (vi) removing unbound components in the biological sample away from the second solid phase;
   (vii) eluting multichaperone-antigen complexes from the second solid phase; and
   (viii) combining said gp96-antigen complexes eluted in step (iv) with the multichaperone-antigen complexes eluted in step (vii).

Disclosed herein is a method for preparing multichaperone-antigen complexes comprising: (a) contacting a biological sample with a solid phase to which HOP affinity molecules are covalently bound, under conditions such that multichaperone-antigen complexes in the biological sample bind said HOP affinity molecules; (b) removing unbound components in the biological sample away from the solid phase; (c) eluting multichaperone-antigen complexes from the solid phase; and (d) recovering the eluted multichaperone-antigen complexes.

The HOP affinity molecules used in the methods described herein may comprise a HOP affinity fragment or variant thereof selected from the group consisting of HOP TPR1 (SEQ ID NO: 1) or a variant thereof, and a combination of any one or more of the foregoing. The HOP affinity molecules can comprise a mammalian HOP affinity fragment or variant thereof, and preferably they comprise a human HOP affinity fragment or variant thereof.

In a specific option, the HOP affinity molecules used in the methods described herein comprise a HOP affinity fragment or variant thereof that is present as a concatamer of two or more of HOP TPR1 or a variant thereof, HOP TPR2a or a variant thereof, and/or HOP TPR1/2a or a variant thereof. In a specific option, the HOP affinity molecules comprise a HOP affinity fragment or variant thereof that is present as a fusion protein of two or more of HOP TPR1 or a variant thereof, HOP TPR2a or a variant thereof, and/or HOP TPR1/2a or a variant thereof.

The biological sample that is used in the methods described herein can be a mammalian cell extract, and is preferably a human cell extract. The biological sample can also be a tumor cell extract and/or an infected cell extract, and can further be an extract of an engineered cell. In a specific option, the biological sample is flow-through resulting from contacting a tumor cell extract, a pathogen-infected cell extract or an extract of cells transfected with and expressing a nucleic acid encoding a tumor associated antigen or a tumor specific antigen or infectious disease antigen, containing cellular proteins, with a solid phase to which is bound a binding partner for a heat shock protein. In a specific option, the solid phase to which is bound said binding partner is an anti-gp96 immunoaffinity column and said heat shock protein is gp96.

The solid phase that is used in the methods described herein may comprise beads. The beads are preferably functionalized with a chemical reactive group (e.g., NHS, aldehyde, epoxy, azolactone) to attach the HOP affinity molecules to the solid phase. The beads can be packed in a column or they can be not packed in a column.
The beads can also be magnetic. In another specific option, the solid phase is a membrane. The solid phase may have a surface comprising polycarbonate, polystyrene, polypropylene, polyethylene, glass, nitrocellulose, dextram, nylon, polyacrylamide or agarose. The HOP affinity molecules may be attached via a bifunctional crosslinker to the solid phase.

The solid phase to which said HOP affinity molecules are covalently bond can be a mixed resin bed comprising a first bead/resin to which a HOP affinity molecule comprising HOP TPR1 or a variant thereof is covalently bound and a second bead/resin to which a HOP affinity molecule comprising HOP TPR1/2a or a variant thereof is covalently bound.

In an option, the eluting step performed in the methods described herein, comprises eluting with a buffered solution containing 150 mM to 1.5M sodium chloride at pH 3 to pH 11. The HOP affinity molecule may comprise HOP TPR1 or a variant thereof and the eluting step comprises eluting with a buffered solution containing 500 mM NaCl at pH 9. In a specific option, the HOP affinity molecule comprises HOP TPR2a or a variant thereof and the eluting step comprises eluting with a buffered solution containing 00 mM NaCl at pH 7.2. In a specific option, the HOP TPR1/2a or a variant thereof and the eluting step comprises eluting with a buffered solution containing 500 mM NaCl at pH 7.2. In another specific option, the HOP affinity molecule comprises HOP TPR1/2a or a variant thereof and the eluting step comprises eluting with a buffered solution containing 500 mM NaCl at pH 9. The solid phase may be a mixed resin bed comprising (a) a HOP affinity molecule comprising HOP TPR1 or a variant thereof; and (b) a HOP affinity molecule comprising HOP TPR1/2a or a variant thereof; and wherein the eluting step comprises eluting with a buffered solution containing 20 mM Tris and 500 mM NaCl, at pH 9.

The methods described herein further comprise combining the recovered multichaperone-antigen complexes with purified heat shock protein-antigen complexes. In one option, the methods described herein further comprise combining the recovered multichaperone-antigen complexes with purified gp96-antigen complexes.

In a specific option, the multichaperone-antigen complexes that are obtained by the methods described herein comprise a combination of at least two different heat shock proteins selected from the group consisting of HSP40, HSP70, HSP90, HSP110, HIP, BIP, and calreticulin. Human HSPs are generally preferred.

The multichaperone-antigen complexes may be purified, such that the HSPs that are present in the preparation containing the multichaperone-antigen complexes account for the majority of protein band intensity on an SDS-PAGE gel.

In a specific option there is herein disclosed a method for preparing multichaperone-antigen complexes comprising (a) contacting an anti-gp96 immunoaffinity column with a human tumor cell extract or human infected cell extract or an extract of cells transfected with and expressing a nucleic acid encoding a tumor associated antigen or a tumor specific antigen or infectious disease antigen under conditions such that gp96-antigen complexes in the extract bind the anti-gp96 immunoaffinity reagent; (b) collecting the flow through from said column; (c) washing said column; (d) eluting gp96- antigen complexes from said column; (e) contacting said flow through collected in step b with a solid phase to which HOP affinity molecules are covalently bound, under conditions such that multichaperone-antigen complexes in the biological sample bind said HOP affinity molecules; (f) removing unbound components in the biological sample away from the solid phase; (g) eluting multichaperone-antigen complexes from the solid phase; and (h) combining said gp96-antigen complexes eluted in step (d) with the multichaperone-antigen complexes eluted in step (g). In a preferred option, the anti-gp96 immunoaffinity column is an anti-gp96 scFv column.

Also disclosed herein is a composition comprising mammalian HOP affinity molecules covalently bound to a solid phase. In a specific option, the HOP affinity molecules in the composition comprise a HOP affinity fragment or variant thereof selected from the group consisting of HOP TPR1 or a variant thereof, HOP PRO2a or a variant thereof, HOP TPR1/2a or a variant thereof, and a combination of any one or more of the foregoing. In a specific embodiment, the HOP affinity molecules comprise a HOP affinity fragment or variant thereof that is present as a concatamer of two or more of HOP TPR1 or a variant thereof, HOP TPR2a or a variant thereof, and/or HOP TPR1/2a or a variant thereof. The HOP affinity molecules may comprise a HOP affinity fragment or variant thereof that is present as a fusion protein of two or more of HOP TPR1 or a variant thereof, HOP TPR2a or a variant thereof, and/or HOP TPR1/2a or a variant thereof. In one option, the HOP affinity molecules comprise a human HOP affinity fragment or variant thereof. In a specific option, the solid phase in the composition comprises beads. The beads can be packed in a column or not paced in a column. The beads can also be magnetic. In another option, the solid phase is a membrane. The solid phase may have a surface comprising polycarbonate, polystyrene, polypropylene, polyethylene, glass, nitrocellulose, dextran, nylon, polyacrylamide or agarose. In a specific option, HOP affinity molecules are via a bifunctional crosslinker to the solid phase.

The HOP affinity molecules in the composition may be noncovalently bound to mammalian multichaperone-antigen complexes. The multichaperone-antigen complexes can be a combination of at least two different heat shock proteins selected from the group consisting of HSP40, HSP70, HSP90, HSP110, HIP, BIP, and calreticulin. In one preferred option, the heat shock proteins are human heat shock proteins.

In a specific option, the solid phase in the composition is in contact with a cell extract. The cell extract can be a mammalian cell extract, and is preferably a human cell extract. The cell extract can also be a tumor cell extract and/or an infected cell extract, and can further be an extract of an engineered cell.

Also disclosed herein is a kit comprising in one or more containers a composition comprising mammalian HOP affinity molecules covalently bound to a solid phase.

Disclosed herein are pharmaceutical compositions comprising the multichaperone-antigen complexes obtained by the methods of the invention.

In one option, a pharmaceutical composition of the invention comprises (a) human multichaperone-antigen complexes and (b) mammalian HOP affinity molecules, with the proviso that the HOP affinity molecules comprise a HOP affinity fragment or variant thereof that is not present as a fusion protein fused to a protein sequence that is not a HOP affinity fragment or a variant thereof, and wherein the HOP affinity molecules do not comprise a wild-type HOP protein. The pharmaceutical composition may comprise multichaperone-antigen complexes that comprise a combination of at least two different heat shock proteins selected from the group consisting of HSP40, HSP70, HSP90, HSP110, HIP, BIP, and calreticulin. In a specific option, the HOP affinity molecules in the pharmaceutical composition comprise a HOP affinity fragment or variant thereof selected from the group consisting of HOP TPR1 (SEQ ID NO: 1) or a variant thereof, HOP TPR2a (SEQ ID NO: 2) or a variant thereof, HOP TPR1/2a (SEQ ID NO: 3) or a variant thereof, and a combination of any one or more of the foregoing. The HOP affinity molecules in the pharmaceutical composition may comprise a human HOP affinity fragment or variant thereof. In another specific embodiment, the HOP affinity molecules in the pharmaceutical composition comprise are present as concatamers of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof. The HOP affinity molecules in the pharmaceutical composition may be present as fusion proteins of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof.

In one option, a pharmaceutical composition of the invention comprises isolated human multichaperone-antigen complexes, wherein the human multichaperone-antigen complexes comprise the following heat shock proteins: HSP70, HSP90, and HSP110, with the proviso that gp96 is not present.

In a specific option, a pharmaceutical composition of the invention comprises isolated human multichaperone-antigen complexes, wherein the human multichaperone-antigen complexes comprise the following heat shock proteins: HSP70, HSP90, gp96 and HSP110, with the proviso that HSP60 is not present.

The pharmaceutical compositions disclosed herein may further comprise HSP-antigen complexes that are not part of the multichaperone-antigen complexes of the invention. In one option, a pharmaceutical composition may comprise the multichaperone-antigen complexes mixed with HSP-antigen complexes, preferably, the HSP-antigen complexes are not present in a noncovalent or covalent complex with the multichaperone-antigen complexes.

The pharmaceutical compositions disclosed herein may be purified, such that the HSPs that are present in the preparation containing the multichaperone-antigen complexes account for the majority of protein band intensity on an SDS-PAGE gel.

In one option, the pharmaceutical compositions of the invention further comprise a pharmaceutically acceptable carrier.

In a specific option, the pharmaceutical compositions provided herein comprise a therapeutically effective amount of said multichaperone-antigen complexes to treat cancer, wherein said multichaperone-antigen complexes comprise an epitope of a tumor-specfic antigen or a tumor-associated antigen.

The pharmaceutical compositions provided herein may comprise a therapeutically effective amount of said multichaperone-antigen complexes to treat an infectious disease, wherein said multichaperone-antigen complexes comprise an epitope that displays the antigenicity of an agent that causes said infections disease.

Also disclosed herein is a method of treating or preventing a type of cancer, comprising administering to a subject in need of such treatment or prevention any one of the pharmaceutical compositions provided herein, wherein the multichaperone-antigen complexes display the antigenicity of a tumor specific antigen or tumor associated antigen of the type of cancer being treated.

Disclosed herein is a method of treating or preventing a type of infectious disease, comprising administering to a subject in need of such treatment or prevention any one of the pharmaceutical compositions provided herein, wherein the multichaperone-antigen complexes display the antigenicity of an antigen of an infectious agent causing the type of infectious disease.

Also disclosed herein is a method of eliciting an immune response in a subject against an antigen comprising administering to the subject an immunogenic amount of the pharmaceutical composition of any one of the pharmaceutical compositions provided herein, wherein the multichaperone-antigen complexes comprise a peptide displaying antigenicity of said antigen.

### DESCRIPTION OF DRAWINGS

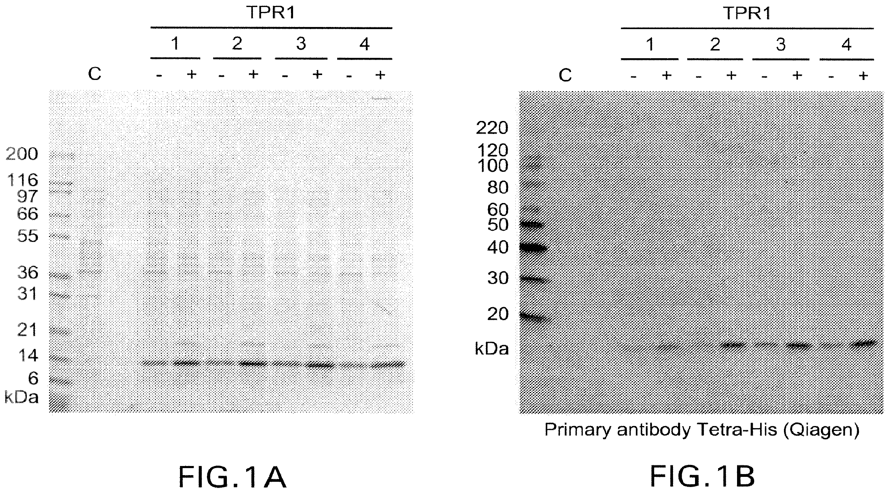

**FIGS. 1 A-B****:** HOP TRP1 Expression. **FIG. 1A** shows HOP TPR1 expression via SDS-PAGE analysis and **FIG. 1B** shows HOP TPR1 expression via western blot analysis, in non-transformed cells (C) and in four separate preparations (1, 2, 3, 4) of extracts of *E*. *coli* strain BL21 (DE3) cells that were transformed with HOP TPR1 (SEQ ID NO: 1) and were either induced to express HOP TPR1 (+) or uninduced (-).

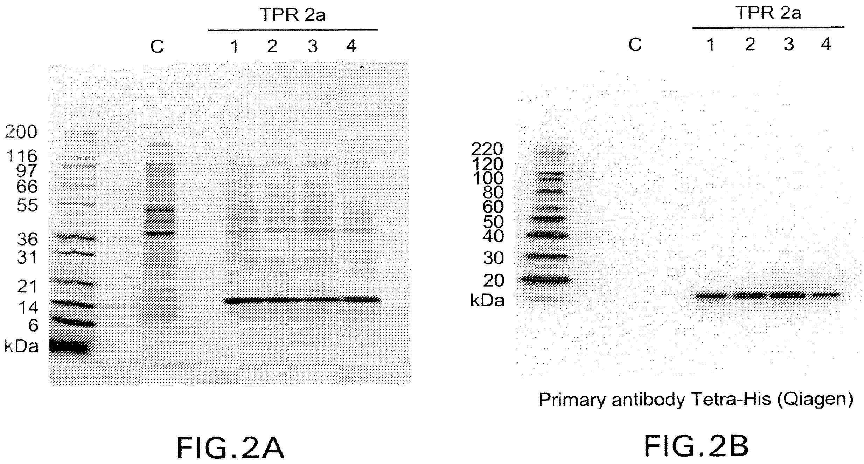

**FIGS. 1 A-B****:** HOP TPR2a Expression: **FIG. 2A** shows HOP TPR2a expression via SDS-PAGE analysis and **FIG. 2B** shows HOP TPR2a expression via western blot analysis, in non-transformed cells (C) and in four separate preparations (1, 2, 3, 4) of extracts of *E*. *coli* strain BL21(DE3) cells that were transformed with HOP TPR2a (SEQ ID NO: 2) and induced to express HOP TPR2a.

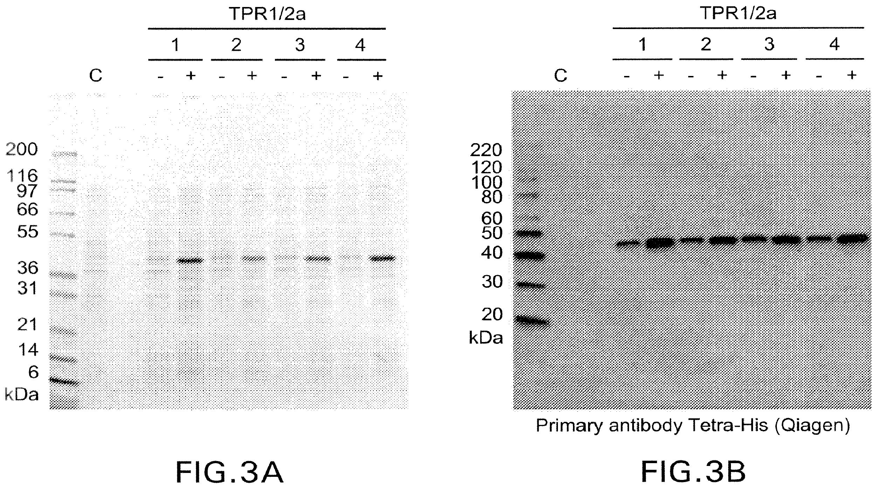

**FIGS. 3 A-B****:** HOP TPR1/2a Expression: **FIG. 3A** shows HOP TPR1/2a expression via SDS-PAGE analysis and **FIG. 3B** shows HOP TPR1/2a expression via western blot analysis, in non-transformed cells (C) and in four separate preparations (1, 2, 3, 4) of extracts of *E*. *coli* strain BL21(DE3) cells that were transformed with HOP TPR1/2a (SEQ ID NO: 3) and were either induced to express HOP TPR1/2a (+) or uninduced (-).

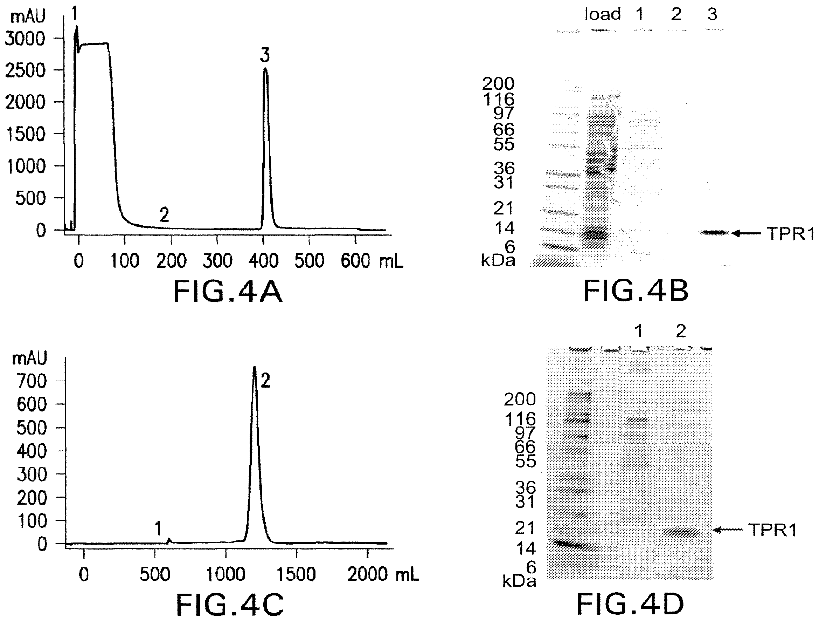

**FIGS. 4 A-D****:** Isolation of HOP TPR1 from *E. Coli* strain BL21(DE3) using metal affinity chromatography followed by gel filtration. **FIG. 4A** shows A UV chromatogram generated during the purification of HOP TPR1 protein by metal affinity chromatography. **FIG. 4B** shows SDS-PAGE analysis of HOP TPR1 protein isolated by metal affinity chromatography. **FIG. 4C** shows a UV chromatogram generated during the purification of HOP TPR1 protein by gel filtration. **FIG. 4D** shows SDS-PAGE analysis of HOP TPR1 protein isolated by gel filtration.

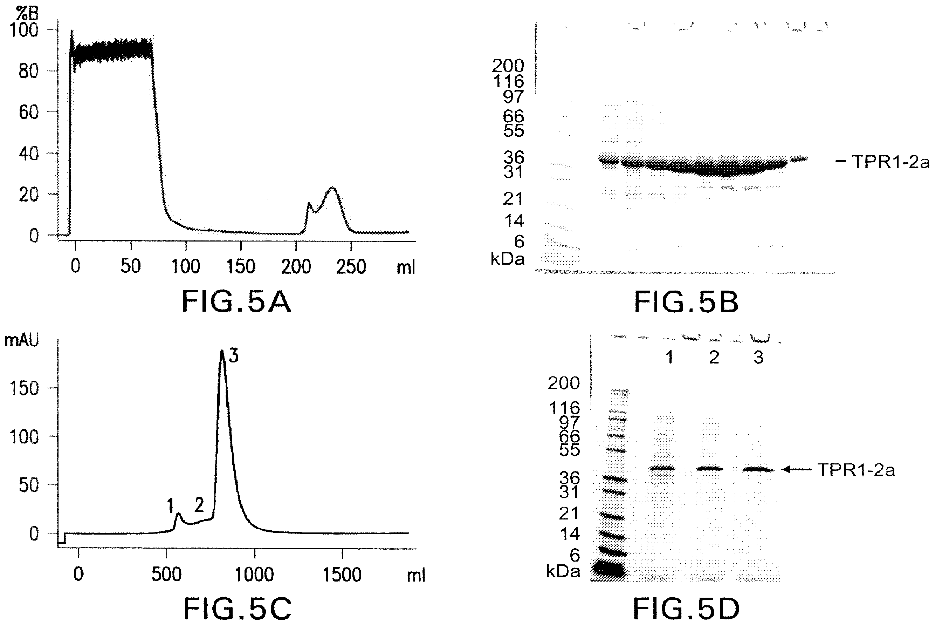

**FIGS. 5 A-D****:** Isolation of HOP TPR1/2a from *E. Coli* strain BL21(DE3) using metal affinity chromatography followed by gel filtration. **FIG. 5A** shows A UV chromatogram generated during the purification of HOP TPR1/2a protein by metal affinity chromatography. **FIG. 5B** shows SDS-PAGE analysis of HOP TPR1/2a protein isolated by metal affinity chromatography. **FIG. 5C** shows a UV chromatogram generated during the purification of HOP TPR1/2a protein by gel filtration. **FIG. 5D** shows SDS-PAGE analysis of HOP TPR1/2a protein isolated by gel filtration.

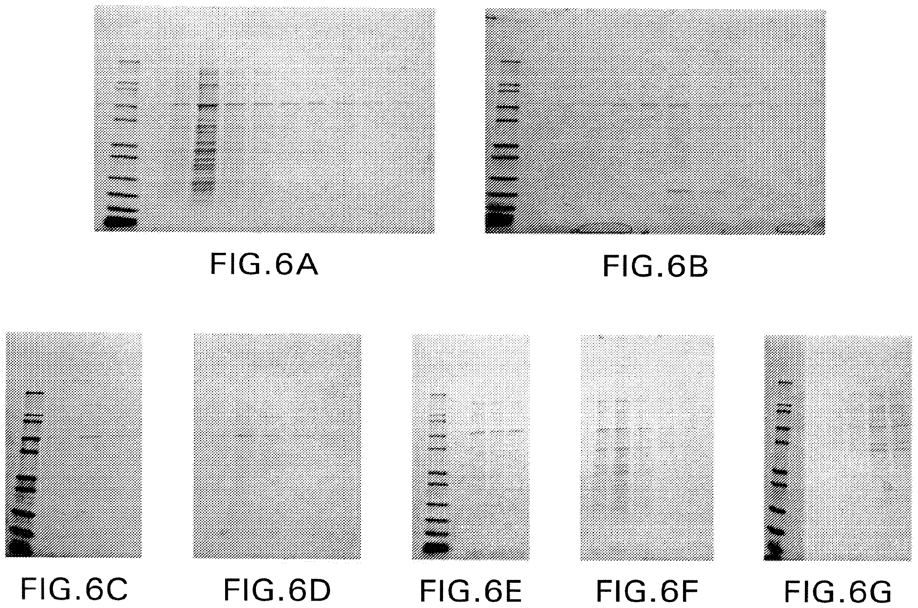

**FIGS. 6 A-G****:** Development of Resin Immobilized HOP TPR1 Elution Conditions. **FIG. 6A** shows SDS-PAGE analyses of proteins eluted with sodium phosphate (30 mM) buffer containing 1.5 mM magnesium chloride and 250 mM sodium chloride (pH 7.2) following isolation with resin immobilized HOP TPR1. **FIG. 6B** shows SDS-PAGE analyses of proteins eluted with sodium phosphate (30 mM) buffer containing 1.5 mM magnesium chloride and 500 mM sodium chloride (pH 7.2) following isolation with resin immobilized HOP TPR1. **FIG. 6C** shows SDS-PAGE analyses of proteins eluted with Tris buffer (20 mM) at pH 8.0 following isolation with resin immobilized HOP TPR1. **FIG. 6D** shows SDS-PAGE analyses of proteins eluted with sodium chloride (1.5 M) in pH 8.0 Tris buffer (20 mM) following isolation with resin immobilized HOP TPR1. **FIG. 6E** shows SDS-PAGE analyses of proteins eluted with Tris buffer (20 mM) at pH 9.0 following isolation with resin immobilized HOP TPR1. **FIG. 6F** shows SDS-PAGE analyses of proteins eluted with sodium chloride (1.5 M) in Tris buffer (20 mM) at pH 9.0 following isolation with resin immobilized HOP TPR1. **FIG. 6G** shows SDS-PAGE analyses of proteins eluted with sodium chloride (1.5 M) in Tris buffer (20 mM) at pH 11.0 (20 mM CAPs buffer) following isolation with resin immobilized HOP TPR1.

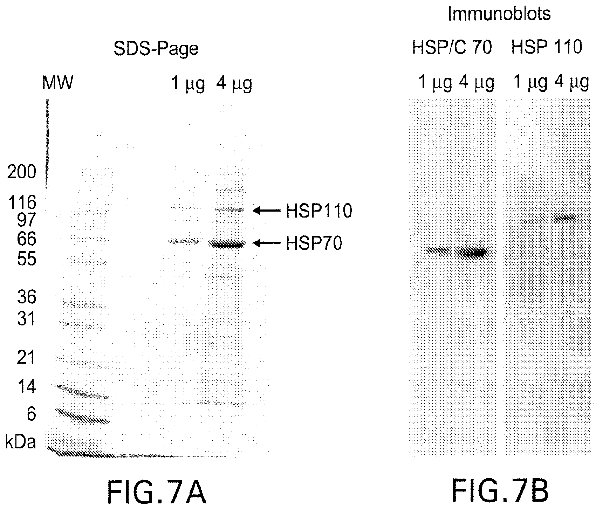

**FIGS. 7 A-B****:** Isolation of multichaperone-antigen complexes from mouse organs of tumor bearing mice using resin immobilized HOP TPR1. **FIG. 7A** shows SDS-PAGE analysis of the multichaperone-antigen complex isolated from 5 g of organ tissue harvested from tumor bearing mice using a 5 mL column of resin immobilized HOP TPR1. **FIG. 7B** Western blot analysis demonstrating isolation of HSP70 and HSP110 from 5 g of organ tissue harvested from tumor bearing mice using a 5 mL column of resin immobilized HOP TPR1. The amount of protein loaded into the gel was either 1 or 4 µg, as indicated in each blot.

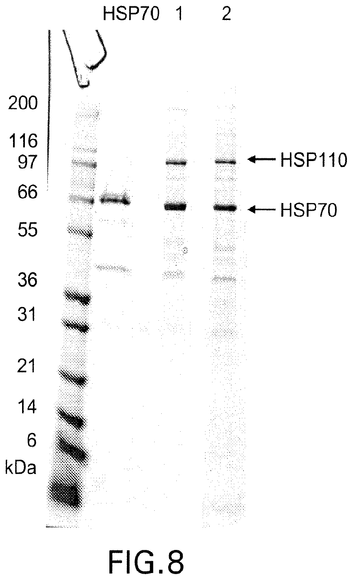

**FIG. 8****:** SDS-PAGE analysis of HOP TPR1 eluate from mouse methylcholanthrene-induced fibrosarcoma (Meth A). FIG. 8 shows SDS-PAGE analysis of eluate following isolation of multichaperone-antigen complexes from two separate preparations (1 and 2) from mouse Meth A ascites using resin immobilized HOP TPR1.

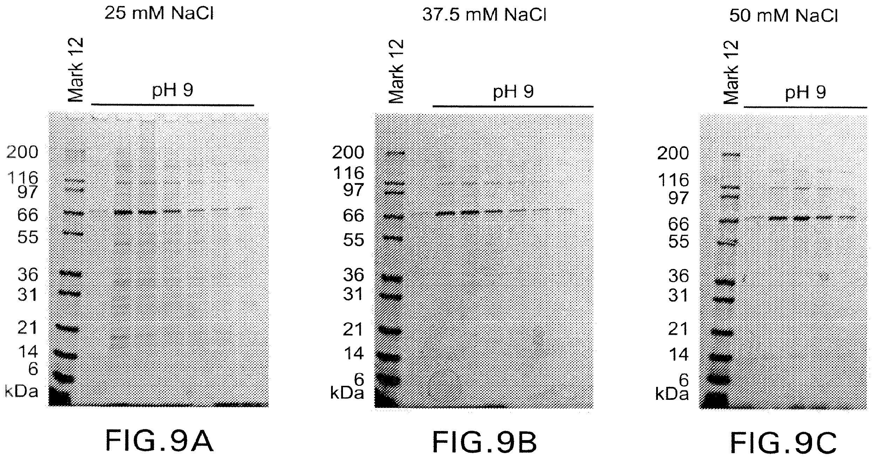

**FIGS. 9 A-C****:** HSP purity resulting from increasing sodium chloride concentration of the clarified homogenate. **FIG. 9A** shows SDS-PAGE analysis for protein fractions collected during experiments in which 25 mM of sodium chloride was added to the clarified homogenate. **FIG. 9B** shows SDS-PAGE analysis for protein fractions collected during experiments in which 37.5 mM of sodium chloride was added to the clarified homogenate. **FIG. 9C** shows SDS-PAGE analysis for protein fractions collected during experiments in which 50 mM of sodium chloride was added to the clarified homogenate.

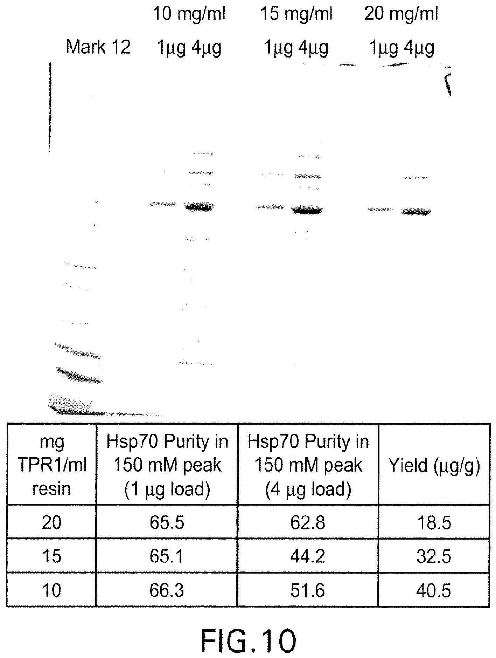

**FIGS. 10 A-B****:** Eluate yield and purity using varying amounts of HOP TPR1 immobilized on Resin. **FIG. 10A** shows SDS PAGE analysis of multichaperone-antigen eluates from experiments using resin loaded with 10, 15, and 20 mg/mL of HOP TPR1. **FIG. 10B** is a table showing protein yield and HSP70 purity in eluates obtained from experiments using resin loaded with 10, 15, and 20 mg/mL of HOP TPR1, as determined by laser densitometry of the SDS-PAGE gel image of FIG. 10A.

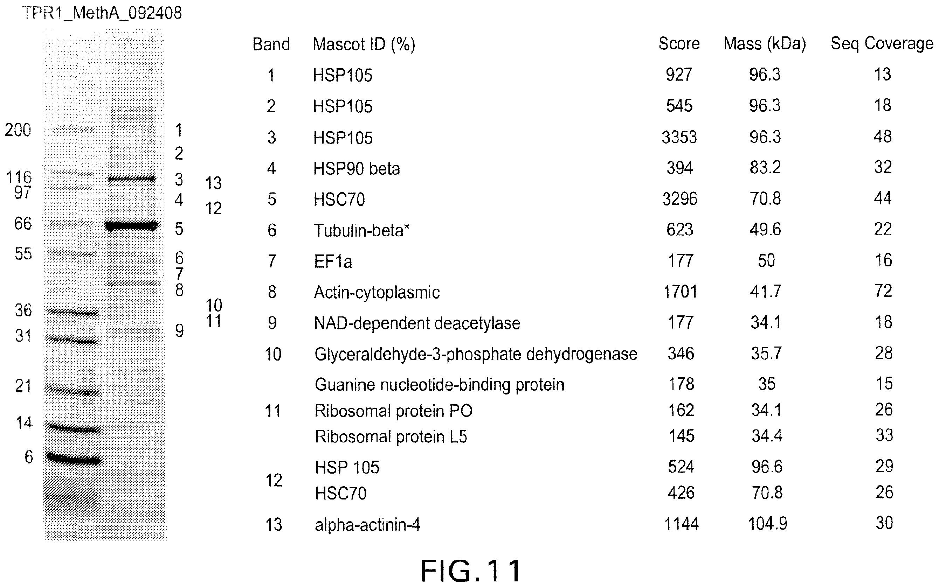

**FIG. 11****:** SDS-PAGE analysis of a mouse Meth A multichaperone-antigen complex preparation obtained using resin immobilized HOP TPR1 used for protein identification by LC/MS/MS of in gel trypsin digested protein bands. Abundant proteins in each gel slice were identified using the MASCOT® algorithm to search the SwissProt mouse protein database. Proteins identified include HSP70, HSP90, HSP110, tubulin, elongation factor 1a, actin, NAD dependent deacetylase, glyceraldehyde-3-phosphate dehydrogenase, guanine nucleotide binding protein, ribosomal proteins, and actinin.

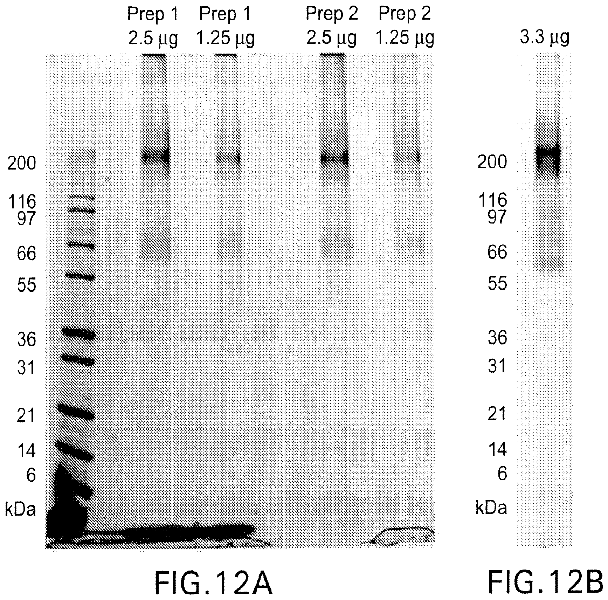

**FIGS. 12A****-B:** Identification of large macromolecular complexes isolated using resin immobilized HOP TPR1. **FIG. 12A** shows SDS-PAGE gel image of glutaraldehyde cross-linked HSPs isolated by resin immobilized HOP TPR1 from mouse organ tissues. **FIG. 12B** shows SDS-PAGE gel image of glutaraldehyde cross-linked HSPs isolated by TPR1 from the human leukemia cell line K562. The amount of protein loaded into the gel is indicated above each image.

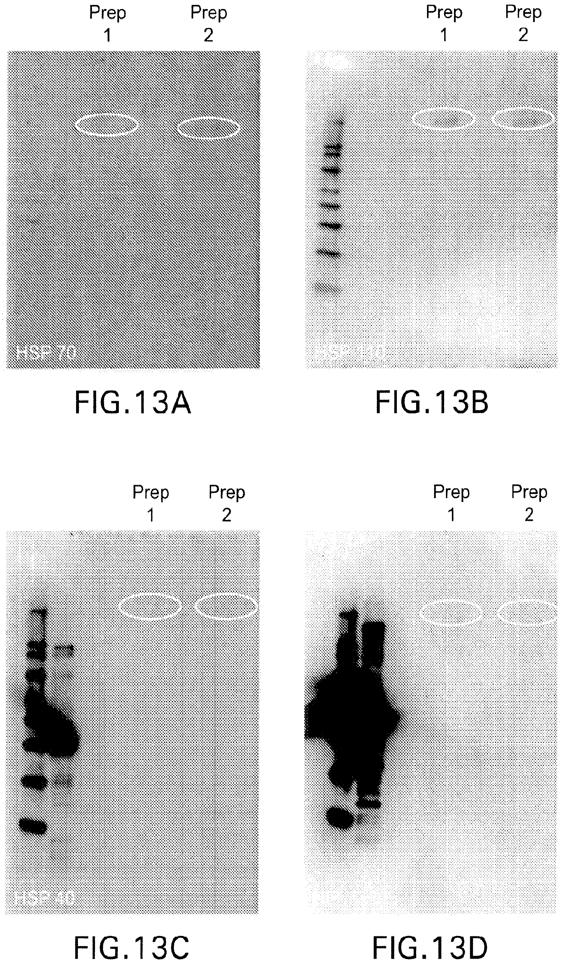

**FIGS. 13A****-D:** Identification HSP members of the multichaperone-antigen complexes isolated using resin immobilized HOP TPR1. **FIG. 13A** shows western blot analysis using primary antibodies against HSP70 to probe the composition of glutaraldehyde cross-linked protein bands transferred from the SDS-PAGE gel of FIG 12. **FIG. 13B** shows western blot analysis using primary antibodies against HSP110 to probe the composition of glutaraldehyde cross-linked protein bands transferred from the SDS-PAGE gel of FIG 12. **FIG. 13C** shows western blot analysis using primary antibodies against HSP40 to probe the composition of glutaraldehyde cross-linked protein bands transferred from the SDS-PAGE gel of FIG 12. **FIG. 13D** shows western blot analysis using primary antibodies against HIP to probe the composition of glutaraldehyde cross-linked protein bands transferred from the SDS-PAGE gel of FIG 12.

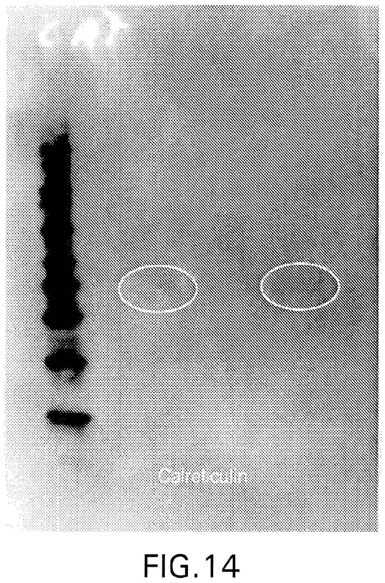

**FIG. 14****:** Detection of calreticulin via Western blot analysis of multichaperone-antigen complexes isolated using resin immobilized HOP TPR1 using a primary antibody against calreticulin to probe the composition of glutaraldehyde cross-linked protein bands transferred from the SDS-PAGE gel of FIG. 12.

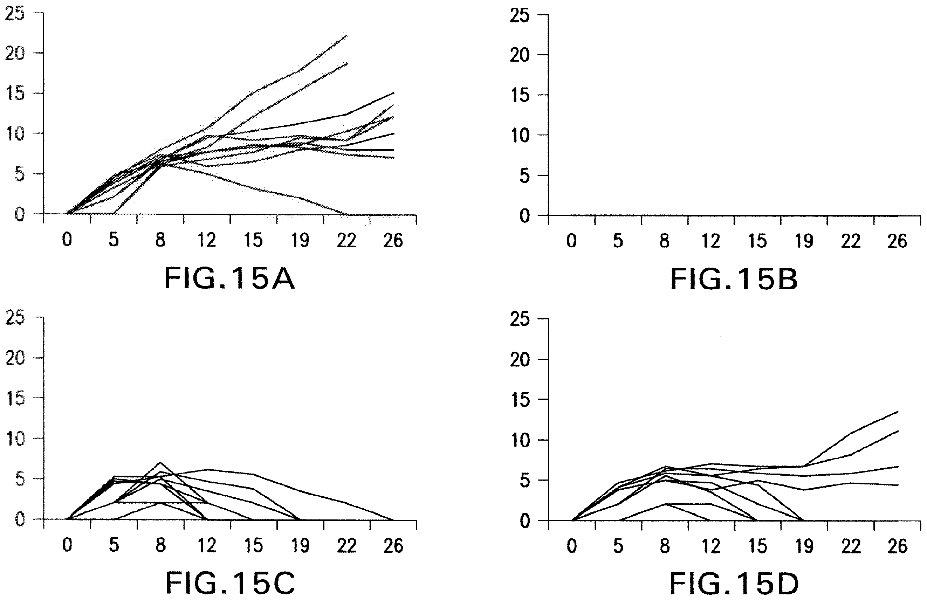

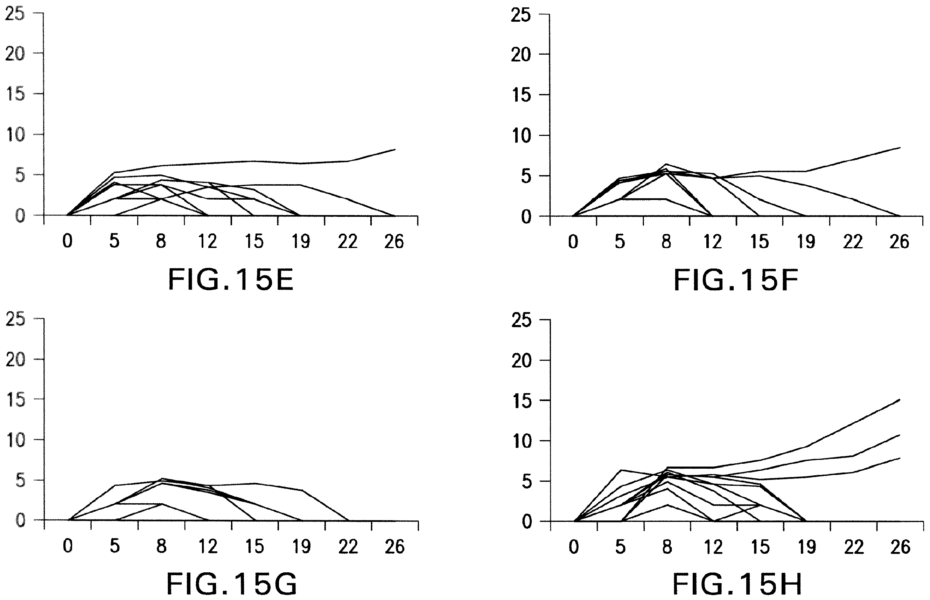

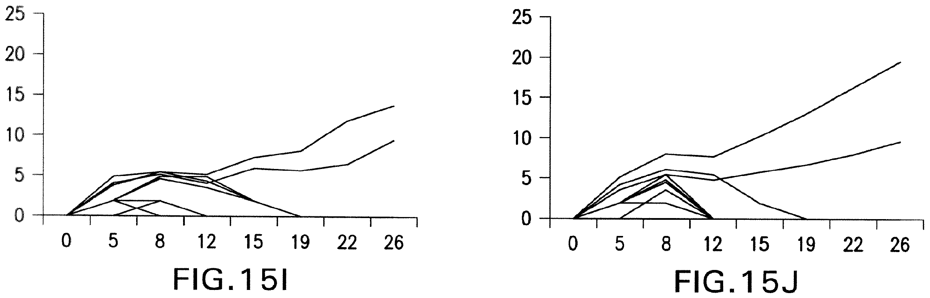

**FIGS. 15A****-J:** Graphs showing tumor rejection activity of the two preparations isolated by resin immobilized HOP TPR1; x-axis = number of days post tumor challenge; y-axis = tumor diameter (mm). **FIG. 15A** is a graph showing no tumor rejection activity in 10 mice vaccinated with the protein formulation buffer of 5 mM potassium phosphate with 9% (weight: volume) sucrose (pH 7.2). **FIG. 15B** is a graph showing tumor rejection activity in 10 mice vaccinated with 2 x 10⁷ irradiated Meth A cells. **FIG. 15C** is a graph showing tumor rejection activity in 10 mice vaccinated with 1.7 µg of the multichaperone fraction from preparation 1. **FIG. 15D** is a graph showing tumor rejection activity in 10 mice vaccinated with 5 µg of the multichaperone fraction from preparation 1. **FIG. 15E** is a graph showing tumor rejection activity in 10 mice vaccinated with 16.7 µg of the multichaperone fraction from preparation 1. **FIG. 15F** is a graph showing tumor rejection activity in 10 mice vaccinated with 1.7 µg of the multichaperone fraction from preparation 2. **FIG. 15G** is a graph showing tumor rejection activity in 10 mice vaccinated with 5 µg of the multichaperone fraction from preparation 2. **FIG. 15H** is a graph showing tumor rejection activity in 10 mice vaccinated with 16.7 µg of the multichaperone fraction from preparation 2. **FIG. 15I** is a graph showing tumor rejection activity in 10 mice vaccinated with 3 µg of a Meth A derived preparation of gp96. **FIG. 15J** is a graph showing tumor rejection activity in 10 mice vaccinated with a combined dose of 3 µg of a gp96 preparation and 5 µg of the multichaperone fraction from preparation 2.

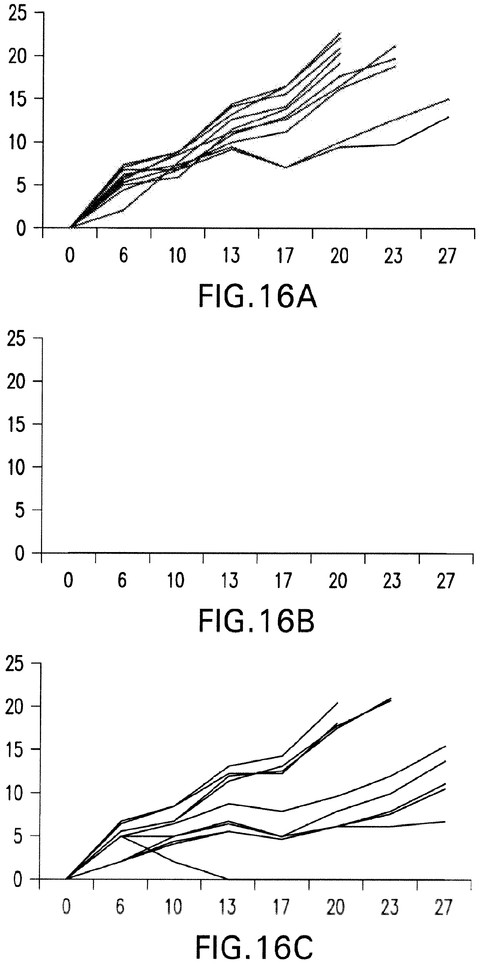

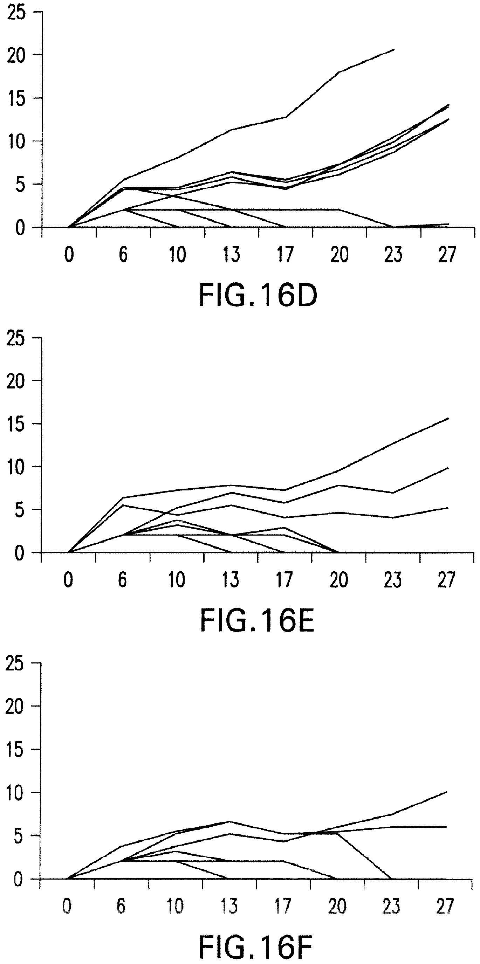

**FIGS. 16A****-F:** Results of a follow on assessment of tumor rejection activity of the TPR1 isolated multichaperone preparation (preparation 1 from FIG. 15) in the Meth A mouse model with vaccination of mice at lower doses; x-axis = number of days post tumor challenge; y-axis = tumor diameter (mm). **FIG. 16A** is a graph showing no tumor rejection activity in 10 mice vaccinated with the protein formulation buffer of 5 mM potassium phosphate with 9% (weight: volume) sucrose, (pH 7.2). **FIG. 16B** is a graph showing tumor rejection activity in 10 mice vaccinated with 2 x 10⁷ irradiated Meth A cells. **FIG. 16C** is a graph showing tumor rejection activity in 10 mice vaccinated with 0.1 µg of the multichaperone fraction. **FIG. 16D** is a graph showing tumor rejection activity in 10 mice vaccinated with 0.5 µg of the multichaperone fraction. **FIG. 16E** is a graph showing tumor rejection activity in 10 mice vaccinated with 1 µg of the multichaperone fraction; **FIG. 16F** is a graph showing tumor rejection activity in 10 mice vaccinated with 3 µg of the multichaperone fraction.

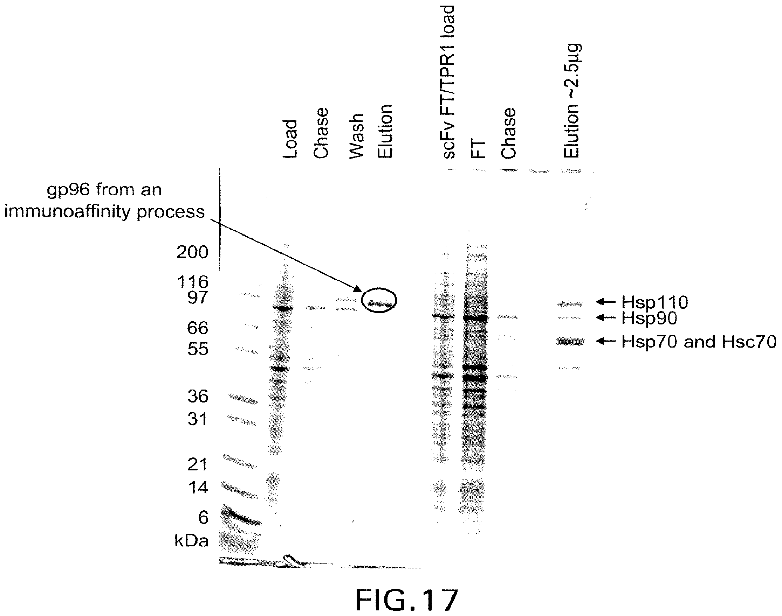

**FIG. 17****:** SDS-PAGE analysis of HSPs isolated from a 10 g pellet of the human tumor cell line K562 using resin immobilized HOP TPR1 at various points during the purification process.

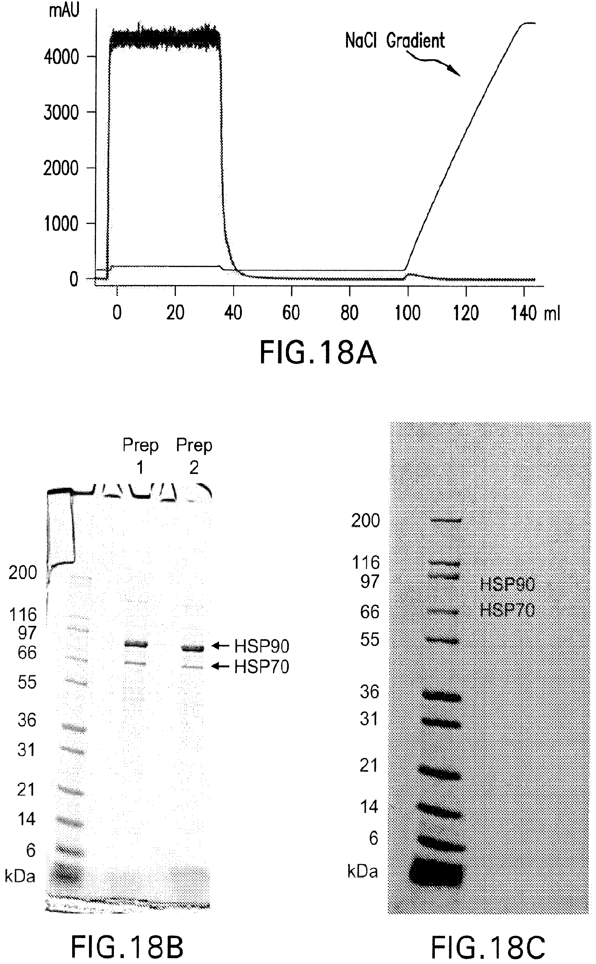

**FIGS. 18 A-C****:** Analysis of HOP TPR1/2a Eluate. **FIG. 18A** shows A UV chromatogram collected at a wavelength of 280 nm depicting isolation of the multichaperone product for resin immobilized HOP TPR1/2a. **FIG. 18B** shows SDS PAGE analysis of products collected from two separate multichaperone preparations isolated by TPR1/2a from mouse organ tissue. **FIG 18C****.** shows SDS-PAGE analysis of a pH 9.0 buffer eluate (20 mM Tris with 500 mM sodium chloride at pH 9.0) collected from a column of resin immobilized HOP TPR1/2a following elution of the multichaperone fraction that was eluted by 10 mM sodium phosphate, 500 mM sodium chloride (pH 7.2).

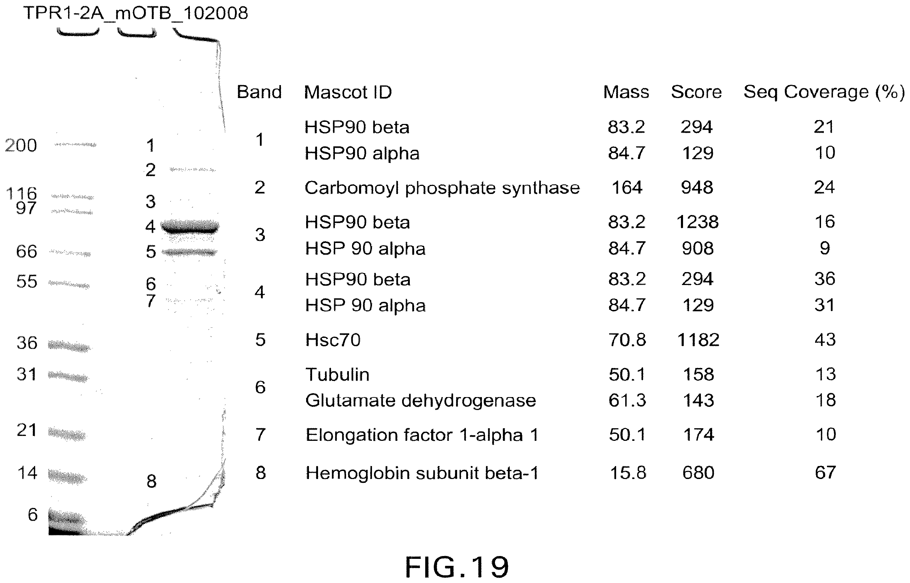

**FIG. 19** Identification of protein bands in multichaperone-antigen complexes isolated using resin immobilized HOP TPR1/2a. FIG. 19 shows SDS-PAGE analysis of mouse organ tissue preparation isolated by resin immobilized TPR1/2a that was used for protein identification by LC/MS/MS of in gel trypsin digested protein bands. Abundant proteins in each gel slice were identified using the MASCOT® algorithm to search the SwissProt mouse protein database. Proteins identified included HSP70, HSP90 alpha, HSP90 beta, tubulin, elongation factor 1a, carbamoyl phosphate synthase, glutamate dehydrogenase, and hemoglobin.

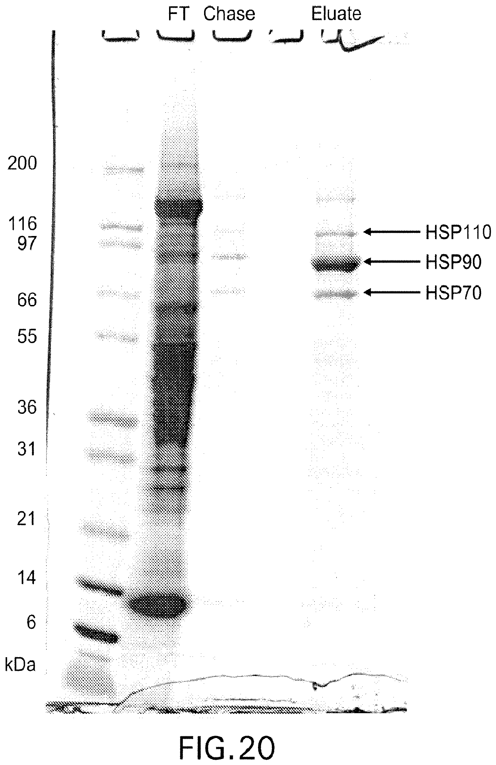

**FIG. 20****:** SDS-PAGE analysis of the flow-through (FT), chase, and eluate obtained using a mixed bed of HOP TPRl-Sepharose and HOP TPR1/2a-Sepharose loaded with a 5 g sample of organs harvested from tumor bearing mice.

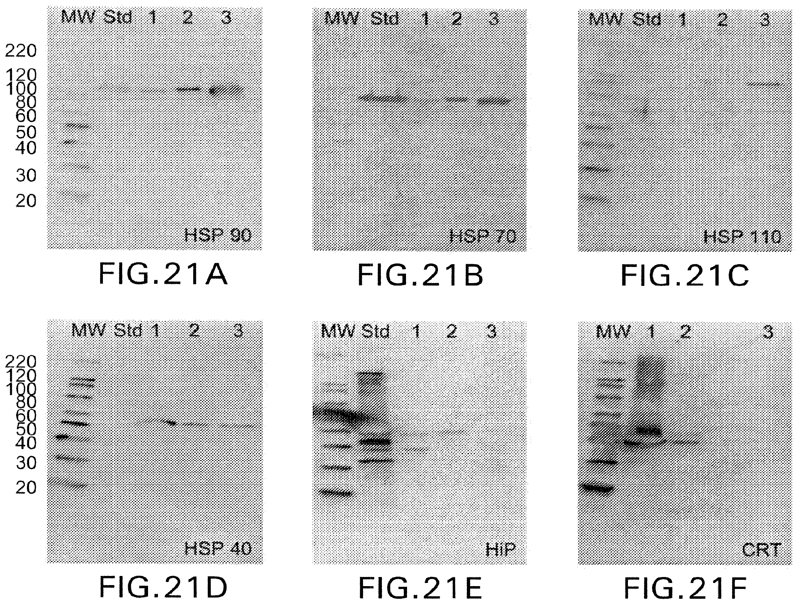

**FIGS. 21 A-F****:** Identification of HSPs by western blot analysis in the eluate isolated by a mixed bed of TPR1-Sepharose and TPR1/2a-Sepharose from a sample of organs harvested from tumor bearing mice. Lanes from left to right in each Western blot were (mw) molecular weight markers, (std) a HSP standard, (1) the flow through of the mixed bed resin, (2) the chase fraction through the mixed bed resin and (3) the eluate of the mixed bed resin. **FIG. 21A** shows western blot analysis using an HSP90 specific antibody. **FIG. 21B** shows western blot analysis using an HSP70 specific antibody. **FIG. 21C** shows western blot analysis using an HSP110 specific antibody. **FIG. 21D** shows western blot analysis using an HSP40 specific antibody. **FIG. 21E** shows western blot analysis using an HIP specific antibody. **FIG. 21F** shows western blot analysis using an calreticulin specific antibody.

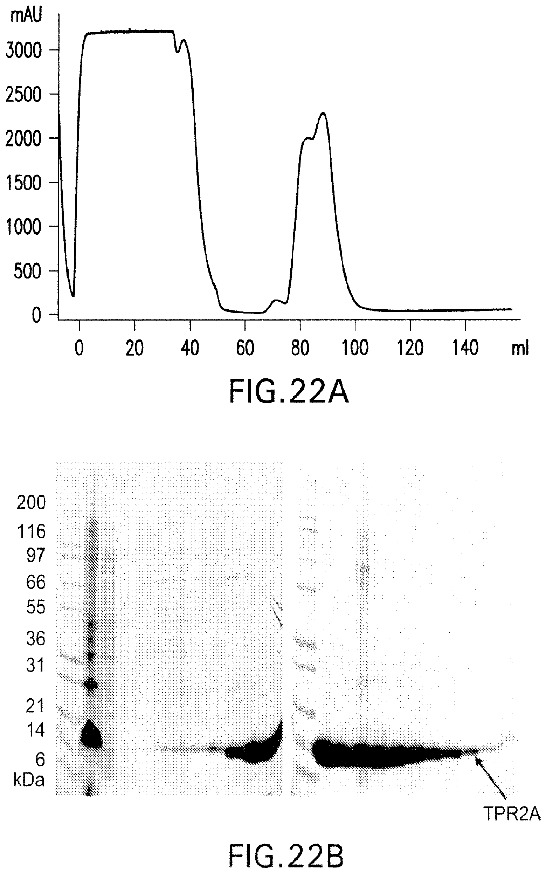

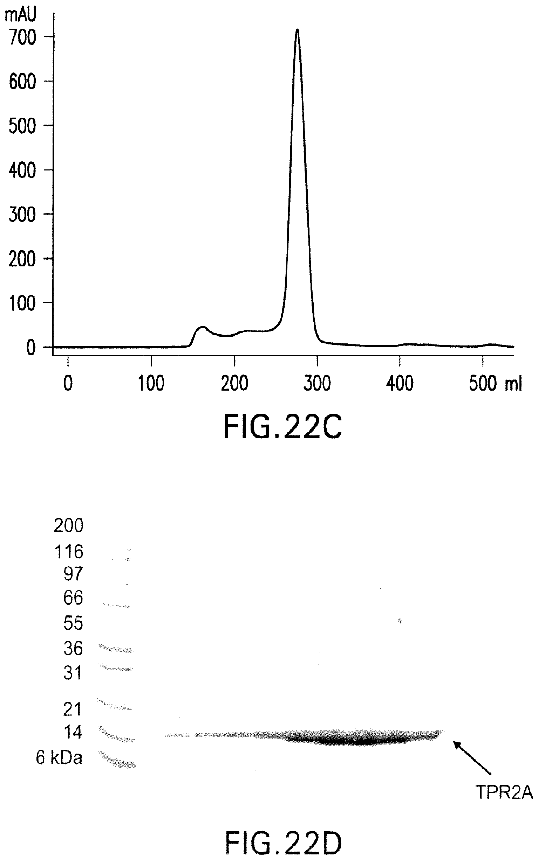

**FIGS. 22 A-D****:** Isolation of HOP TPR2a from *E. Coli* strain BL21(DE3) using metal affinity chromatography followed by gel filtration. **FIG. 22A** shows A UV chromatogram collected at a wavelength of 280 nm depicting isolation of the HOP TPR2a protein by metal affinity chromatography. **FIG. 22B** shows SDS PAGE analysis of the HOP TPR2a product isolated by metal affinity chromatography. **FIG. 22C** shows a UV chromatogram collected at a wavelength of 280 nm depicting isolation of the HOP TPR2a protein by gel filtration. **FIG. 22D** shows SDS-PAGE analysis of the TPR2a product isolated by gel filtration.

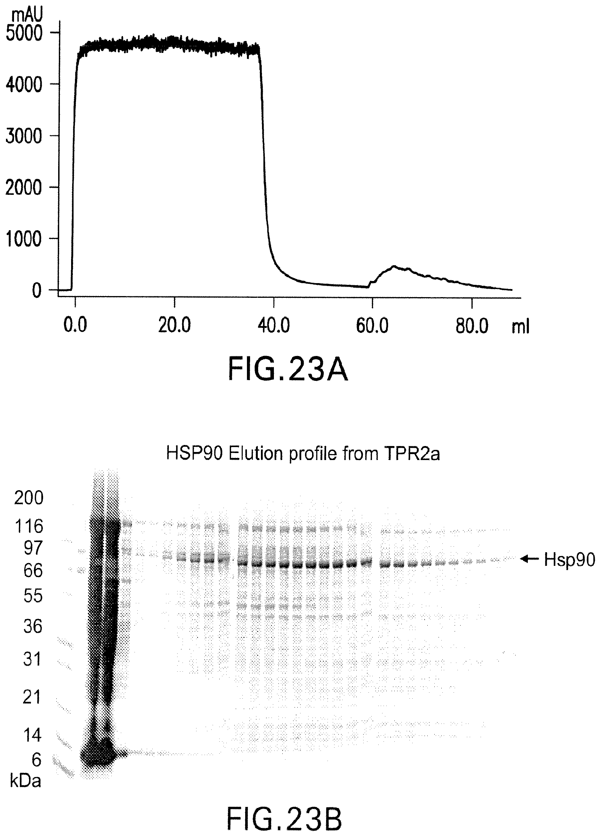

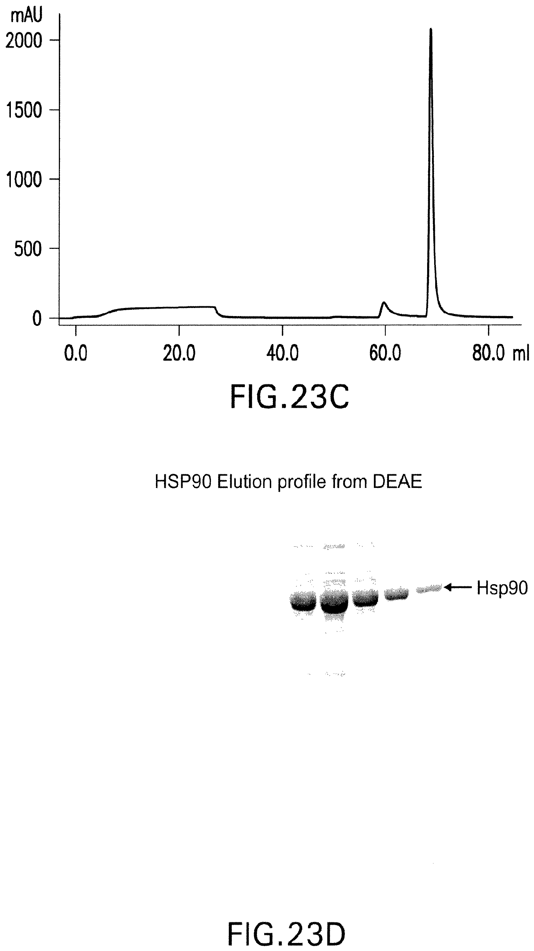

**FIGS. 23 A-D****:** Isolation of a HSP90 rich fraction from 20 g of mixed organ tissue harvested from tumor bearing mice by using a 12 mL column of resin immobilized HOP TPR2a followed by DEAE. **FIG. 23A** is a UV chromatogram at 280 nm showing isolation of the chaperone fraction from resin immobilized HOP TPR2a. **FIG. 23B** shows SDS-PAGE analysis of protein fractions collected from resin immobilized HOP TPR2a. **FIG. 23C** is a UV chromatogram at 280 nm showing isolation of the chaperone fraction from the DEAE column. **FIG. 23D** is an SDS-PAGE analysis of protein fractions collected from the DEAE column.

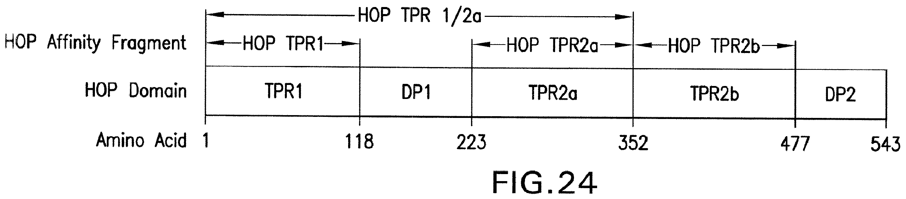

**FIG. 24** shows the domain structure of the human HOP protein.

### DETAILED DESCRIPTION

Disclosed herein are uses of HOP affinity molecules in affinity methods to isolate multichaperone (multi-HSP)-antigen complexes. As used herein the term "antigen" refers to an antigenic peptide or antigenic protein. Such complexes have use in therapy. For example, such complexes that are isolated from cancer cells or that comprise a protein or peptide that displays the antigenicity of a tumor-specific antigen or tumor-associated antigen, can be used to treat a cancer of the same type as the cancer cells, or a cancer displaying the antigenicity of the tumor-specific antigen or tumor-associated antigen, respectively. Also, such complexes that are isolated from infected cells, *i.e*., cells infected by a pathogen or infectious agent that causes an infectious disease, or comprising a protein or peptide that displays the antigencity of a pathogen or infectious agent that causes an infectious disease, can be used to treat the infectious disease. The HOP affinity molecules comprise one or more HOP affinity fragments, but preferably do not comprise wild-type HOP protein, and preferably the HOP affinity molecules do not contain as sequences adjacent to the HOP affinity fragments those sequences that flank the specified fragment in the native HOP protein. In an alternative option, the HOP affinity molecules comprise wild-type HOP protein.

The multichaperone-antigen complexes as disclosed herein are complexes collectively comprising more than one different HSP and more than one different antigen. In particular, as isolated from a cell, the multichaperone-antigen complexes disclosed herein may collectively comprise more than one different HSP and a heterogeneous population of antigens, which are noncovalently associated with the HSPs. The different HSPs in the multichaperone-antigen complexes are noncovalently bound to one or more components of the complex, such as other HSPs in the complex, in addition to being bound to the antigens with which they noncovalently associate. The identity of the different HSPs in the multichaperone-antigen complexes of the invention depend at least in part on the identity (and thus specificity of HSP binding) of the HOP affinity fragments present in the HOP affinity molecules used in the affinity methods of the invention to isolate the multichaperone-antigen complexes.

The multichaperone-antigen complexes as disclosed herein may comprise a combination of at least two different heat shock proteins selected from the group consisting of HSP40, HSP60, HSP70 (including hsc70 and hsp70, the constitutive and inducible forms, respectively), HSP90 (also known as HSP84/86 or HSP90α/β), HSP110 (also known as HSP105), HIP, BIP (also known as grp78), and calreticulin. The HSPs and/or antigens in the multichaperone-antigen complexes disclosed herein can be recombinant and/or endogenous (made intracellularly) with respect to the cell from which the complexes are isolated. In one option, the multichaperone-antigen complexes comprise mammalian HSPs, preferably human HSPs, isolated from mammalian or human cells. In a specific option, the multichaperone-antigen complexes comprise mammalian antigens, preferably human antigens. The multichaperone-antigen complexes may comprise non-human mammalian HSPs and human antigens. In another option, the multichaperone-antigen complexes comprise human mammalian HSPs and non-human mammalian antigens. In a preferred option, the multichaperone-antigen complexes comprise human HSPs and human antigens, most preferably endogenously (non-recombinantly) expressed in human cells from which the complexes are isolated. In another preferred option, the multichaperone-antigen complexes comprise mammalian HSPs and mammalian antigens, most preferably endogenously (non-recombinantly) expressed in mammalian cells from which the complexes are isolated.

### 5.1. Methods For Preparing Multichaperone Complexes

Disclosed herein are methods for preparing multichaperone-antigen complexes comprising (a) contacting a biological sample with a solid phase to which HOP affinity molecules are covalently bound, under conditions such that multichaperone-antigen complexes in the biological sample bind said HOP affinity molecules; (b) removing unbound components in the biological sample away from the solid phase; (c) eluting multichaperone-antigen complexes from the solid phase; and (d) recovering the eluted multichaperone-antigen complexes.

### 5.1.1. HOP Affinity Molecules and HOP Affinity Fragments

As used herein, the term "HOP affinity molecule" refers to a molecule that comprises one or more HOP affinity fragments, or one or more variants thereof, but not wild-type HOP protein. Preferably the HOP affinity molecules do not contain as sequences adjacent to the HOP affinity fragments those sequences that flank the specified fragment in the native HOP protein.

As used herein, the term "HOP affinity fragment" refers to a fragment of a HOP protein that binds to one or more heat shock proteins. In one option, a HOP affinity fragment is a fragment of a mammalian HOP protein sequence. In a preferred option, a HOP affinity fragment is a fragment of the human HOP protein sequence (SEQ ID NO: 8). In another option, a HOP affinity fragment is a fragment of a non-human mammalian HOP protein sequence, from a non-primate (e.g., a camel, donkey, zebra, cow, pig, horse, goat, sheep, cat, dog, rat, and mouse) or a primate (e.g., a monkey and chimpanzee). The HOP affinity fragment can be any one of the following four fragments of the human HOP protein sequence, optionally further comprising adjacent DP region(s) (e.g., DP1 and/or DP2)(see Figure 24): (1) HOP TPR1, which consists of amino acid residues 1 to 118 of human HOP (SEQ ID NO: 1); (2) HOP TPR2a, which consists of amino acid residues 223 to 352 of human HOP (SEQ ID NO: 2); (3) HOP TPR1/2a, which consists of amino acid residues 1 to 352 of human HOP (SEQ ID NO: 3), and (4) HOP TPR2b, which consists of amino acid residues 353-477 of human HOP (SEQ ID NO: 4).

As used herein, the term "variants" in the context of variants of HOP affinity fragments refers to HOP affinity fragments that contain deletions, insertions, substitutions, or other modifications relative to native HOP affinity fragments, but that retain their specificity to bind to HSPs. The variants of HOP affinity fragments preferably have deletions, insertions, substitutions, and/or other modifications of not more than 5, 4, 3, 2, or 1 amino acid residues. In a specific option, the variant of a HOP affinity fragment has the native sequence of a HOP affinity fragment as specified above, except that 1 to 5 amino acids are added or deleted from the carboxy and or the amino end of the fragment (where the added amino acids are the flanking amino acid(s) present in the native HOP protein).

In a specific option, a variant of HOP TPR1 (SEQ ID NO: 1) comprises the following amino acid residues of HOP TPR1 (SEQ ID NO: 1): Lys 8, Asn 12, Asn 43, Lys 73, and Arg 77. In one option, a variant of HOP TPR1 (SEQ ID NO: 1)comprises amino acid residues 8 to 77 of HOP TPR1 (SEQ ID NO: 1). In another option, a variant of HOP TPR1 (SEQ ID NO: 1) comprises, or alternatively consists of, amino acid residues 4 to 105 of HOP TPR1 (SEQ ID NO: 1). In a specific option, a variant of HOP TPR1 (SEQ ID NO: 1) comprises, or alternatively consists of, amino acid residues 4 to 169 of HOP (SEQ ID NO: 8). In another option, a variant of HOP TPR1 (SEQ ID NO: 1) comprises, or alternatively consists of, amino acid residues 1 to 122 of HOP (SEQ ID NO: 8). A variant of HOP TPR1 (SEQ ID NO: 1) may comprise, or alternatively consist of, amino acid residues 1 to 115 of HOP TPR1 (SEQ ID NO: 1). In a specific option, a variant of HOP TPR1 (SEQ ID NO: 1) comprises, or alternatively consists of, amino acid residues 1 to 148 of HOP (SEQ ID NO: 8). In another option, the variant of HOP TPR1 (SEQ ID NO: 1) comprises the carboxylate clamp residues, which have been implicated in binding to HSPs (see Scheufler et al., 2000, Cell, 101: 199-210).

In a specific option, a variant of HOP TPR2a (SEQ ID NO: 2) comprises the following amino acid residues of HOP TPR2a (SEQ ID NO: 2): Lys 229, Asn 233, Asn 264, Lys 301, and Arg 305. In another option, a variant of HOP TPR2a (SEQ ID NO: 2) comprises, or alternatively consists of, amino acid residues 229 to 305 of HOP TPR2a (SEQ ID NO: 2). In a specific option, a variant of HOP TPR2a (SEQ ID NO: 2) comprises, or alternatively consists of, amino acid residues 225 to 333 of HOP TPR2a (SEQ ID NO: 2). In another option, a variant of HOP TPR2a (SEQ ID NO: 2) comprises, or alternatively consists of, amino acid residues 214 to 362 of HOP (SEQ ID NO: 8). In a specific option, a variant of HOP TPR2a (SEQ ID NO: 2) comprises, or alternatively consists of, amino acid residues 223 to 349 of HOP TPR2a (SEQ ID NO: 2). In one option, a variant of HOP TPR2a (SEQ ID NO: 2) comprises, or alternatively consists of, amino acid residues 211 to 352 of HOP (SEQ ID NO: 8). In a specific option, a variant of TPR2a (SEQ ID NO: 2) comprises, or alternatively consists of, amino acid residues 200 to 380 of HOP (SEQ ID NO: 8). In another option, the variant of HOP TPR2a (SEQ ID NO: 2) comprises the carboxylate clamp residues, which have been implicated in binding to HSPs (see Scheufler et al., 2000, Cell, 101: 199-210).

A variant of HOP TPR2b, (HOP TPR2b consists of amino acid residues 353 to 477 of human HOP (SEQ ID NO: 4)), may comprise amino acid residues Lys 429 and Arg 433. In a specific option, a variant of HOP TPR2b (SEQ ID NO: 4) comprises, or alternatively consists of, amino acid residues 429 to 433 of HOP TPR2b (SEQ ID NO: 4). In another option, a variant of HOP TPR2b (SEQ ID NO: 4) comprises, or alternatively consists of, amino acid residues 360 to 461 of HOP TPR2b (SEQ ID NO: 4). In another option, a variant of HOP TPR2b (SEQ ID NO: 4) comprises, or alternatively consists of, amino acid residues 360 to 531 of HOP (SEQ ID NO: 8). In one option, a variant of HOP TPR2b (SEQ ID NO: 4) comprises, or alternatively consists of, amino acid residues 349 to 481 of HOP (SEQ ID NO: 8). In another specific option, a variant of HOP TPR2b (SEQ ID NO: 4) comprises, or alternatively consists of, amino acid residues 381 to 537 of HOP (SEQ ID NO: 8). In another specific option, the variant of HOP TPR2b (SEQ ID NO: 4) comprises the carboxylate clamp residues, which have been implicated in binding to HSPs (see Carrigan et al., 2004, JBC, 279: 16185-16193).

A HOP affinity fragment may contain one or more DP domains. In a specific option, a HOP affinity fragment contains the DP1 domain of human HOP, which consist of amino acid residues 119 to 222 of human HOP (SEQ ID NO: 9). In another option, a HOP affinity fragment contains the DP2 domain of human HOP, which consists of amino acid residues 478 to 543 of human HOP (SEQ ID NO: 10).

A variant of HOP TPR1/2a (SEQ ID NO: 3) may comprise the following amino acid residues of HOP TPR1/2a (SEQ ID NO: 3); Lys 8, Asn 12, Asn 43, Lys 73, Arg 77, Lys 229, Asn 233, Asn 264, Lys 301, and Arg 305. In a specific option, a variant of HOP TPR1/2a (SEQ ID NO: 3) comprises, or alternatively consists of, amino acid residues 8 to 305 of HOP TPR1/2a (SEQ ID NO: 3). In another option, a variant of HOP TPR1/2a (SEQ ID NO: 3) comprises, or alternatively consists of, amino acid residues 4 to 169 of HOP TPR1/2a (SEQ ID NO: 3). In one option, a variant of HOP TPR1/2a (SEQ ID NO: 3) comprises the carboxylate clamp residues, which have been implicated in binding to HSPs (see Scheufler et al., 2000, Cell. 101: 199-210).

In the foregoing options or in another option, the variant of a HOP affinity fragment contains only conservative substitutions relative to the native HOP affinity fragment sequences, preferably not more than 5, 4, 3, 2, or 1 conservative substitutions, alone or in addition to the modifications described above. Conservative substitutions are those in which the amino acid sequence of a peptide is modified by replacing one or more amino acids with different amino acids which have similar chemical or structural characteristics, and which preferably do not significantly alter the biological function of the peptide. For example, an amino acid residue can be replaced with an amino acid residue having a side chain with a similar charge. Families of amino acid residues having side chains with similar charges have been defined in the art. These families include amino acids with basic side chains (e.g. , lysine, arginine, histidine), acidic side chains (e.g. , aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Also disclosed herein, in any of the foregoing options, the variant of a HOP affinity fragment can be not more than 118, 130, 352, 125, 150, 300, 400 or 543 amino acids in length.

In one embodiment, a HOP affinity molecule is a fusion protein, the HOP protein sequence of which comprises one or more of the foregoing three fragments or variants thereof: (1) HOP TPR1 (SEQ ID NO: 1); (2) HOP TPR2a (SEQ ID NO: 2), and (3) HOP TPR1/2a (SEQ ID NO: 3). In one embodiment, a HOP affinity molecule is a fusion protein that comprises a protein sequence other than a HOP affinity fragment or a variant thereof. For example, in one embodiment, a HOP affinity molecule is a fusion protein that comprises an affinity label. In another embodiment, a HOP affinity molecule does not comprise an affinity label. In another embodiment, a HOP affinity molecule is a fusion protein that comprises a protein sequence of a different HOP affinity fragment or a variant thereof. For example, in one embodiment, a HOP affinity molecule is a fusion protein that consists of HOP TPR1 (SEQ ID NO: 1) or a variant thereof and HOP TPR2a (SEQ ID NO: 2) or a variant thereof. In another embodiment, a HOP affinity molecule is a fusion protein that consists of HOP TPR1 (SEQ ID NO: 1) or a variant thereof and HOP TPR1/2a (SEQ ID NO: 3) or a variant thereof. In another embodiment, a HOP affinity molecule is a fusion protein that consists of HOP TPR2a (SEQ ID NO: 2) or a variant thereof and HOP TPR1/2a (SEQ ID NO: 3) or a variant thereof. In another embodiment, a HOP affinity molecule is a fusion protein that consists of HOP TPR1 (SEQ ID NO: 1) or a variant thereof, HOP TPR2a (SEQ ID NO: 2) or a variant thereof, and HOP TPR1/2a (SEQ ID NO: 3) or a variant thereof. In optional specific embodiments, the HOP affinity fragments are as specified above but also comprising the DP1 and/or DP2 domains adjacent to the specified TPR domains.

In a particular embodiment, a HOP affinity molecule is a fusion protein, such as any of those described above, the fusion protein further comprising the DP1 domain of human HOP, which consists of amino acid residues 119 to 222 of human HOP (SEQ ID NO:9), and/or the DP2 domain of human HOP, which consists of amino acid residues 478 to 543 of human HOP (SEQ ID NO: 10).

In an embodiment, a HOP affinity molecule is a concatamer, which comprises two or more of one particular HOP affinity fragment or a variant thereof. For example, in an embodiment, a HOP affinity molecule is a concatamer that comprises two or more of HOP TPR1 (SEQ ID NO: 1) or a variant thereof. In another embodiment, a HOP affinity molecule is a concatamer that consists of two or more of HOP TPR2a (SEQ ID NO: 2) or a variant thereof. In another embodiment, a HOP affinity molecule is a concatamer that consists of two or more of HOP TPR1/2a (SEQ ID NO: 3) or a variant thereof.

In a particular embodiment, a HOP affinity molecule is a concatamer, such as any of those described in above, the concatamer further comprising the DP1 domain of human HOP (SEQ ID NO: 9), and/or the DP2 domain of human HOP (SEQ ID NO: 10).

In yet another embodiment, a HOP affinity molecule is a concatamer-fusion protein hybrid, which comprises two or more of one particular HOP affinity fragment and a protein sequence of a different HOP affinity fragment. For example, in one embodiment, a HOP affinity molecule is a concatamer-fusion protein hybrid that consists of two or more of HOP TPR1 (SEQ ID NO: 1) or a variant thereof and one or more of HOP TPR2a (SEQ ID NO: 2) or a variant thereof. In another embodiment, a HOP affinity molecule is a concatamer-fusion protein hybrid that consists of two or more of HOP TPR1 (SEQ ID NO: 1) and one or more of HOP TPR1/2a (SEQ ID NO: 3) or a variant thereof. In another embodiment, a HOP affinity molecule is a concatamer-fusion protein hybrid that consists of two or more of HOP TPR2a (SEQ ID NO: 2) or a variant thereof and one or more of HOP TPR1/2a (SEQ ID NO: 3) or a variant thereof. In another embodiment, a HOP affinity molecule is a concatamer-fusion protein hybrid that consists of two or more of HOP TPR2a (SEQ ID NO: 2) and one or more of HOP TPR1/2a (SEQ ID NO: 3) or a variant thereof, and one or more of HOP TPR1 (SEQ ID NO: 1) or a variant thereof. In another option, a HOP affinity molecule is a concatamer-fusion protein hybrid that consists of two or more of HOP TPR1 (SEQ ID NO: 1) or a variant thereof and one or more of HOP TPR1/2a (SEQ ID NO: 3) or a variant thereof, and one or more of HOP TPR2a (SEQ ID NO: 2) or a variant thereof. In one option, a HOP affinity molecule is a concatamer-fusion protein hybrid that consists of two or more of HOP TPR1/2a (SEQ ID NO: 3) or a variant thereof and one or more of HOP TPR2a (SEQ ID NO: 2) or a variant thereof, and one or more of HOP TPR1 (SEQ ID NO: 1) of a variant thereof. In certain options, the HOP affinity fragments are as specified above but are also comprising the DP1 and/or DP2 domains adjacent to the specified TPR domains.

A HOP affinity molecule may be a concatamer-fusion protein hybrid, which comprises two or more of one particular HOP affinity fragment or a variant thereof and a protein sequence other than a HOP affinity fragment. For example, in one option, a HOP affinity molecule is a concatamer-fusion protein hybrid that consists of two or more of HOP TPR1 (SEQ ID NO: 1) or a variant thereof and an affinity label. In another option, a HOP affinity molecule is a concatamer-fusion protein hybrid that consists of two or more of HOP TPR2a (SEQ ID NO: 2) or a variant thereof and an affinity label. In another embodiment, a HOP affinity molecule is a concatamer-fusion protein hybrid that consists of two or more of HOP TPR1/2a (SEQ ID NO: 3) or a variant thereof and an affinity label.

A HOP affinity molecule may comprise a non-protein chemical structure. For example, in some options, a HOP affinity molecule is modified by acetylation (*e.g*., at the N-terminus), amidation (*e.g*., at the C-terminus), glycosylation, phosphorylation, derivatization by known protecting/blocking groups and/or comprises a cross linker that facilitates covalent attachment to a solid phase (for use in the affinity purification methods of the invention). Many chemical cross linkers are known to those skilled in the art. Several are based upon polyethylene glycol ethers of various chain lengths (*e.g.* 4 to 24 PEG repeats) that have functionalized groups at either ends of the polymer chain. One of the functional groups is reactive towards proteins such as HOP affinity molecules. Examples of such functional groups include, but are not limited to, N-hydroxysuccinimide (NHS), which reacts with amine groups of the HOP affinity molecules; maleimide, which reacts with sulfhydryls of the HOP affinity molecules; and aldehyde and expoxy functionalities, which also react with amine groups of the HOP affinity molecules. The second functional group is selected to react with the solid support. Examples of second functional groups include, but are not limited to amine groups, which react with aldehyde on a solid support and expoxy functionalized solid supports. Chemical cross linkers may have the same or different functional groups at either end of the polymer chain. Some have photo reactive groups on one end of the polymer and a NHS group on the other to reduce possible side reactions. Such reagents are reacted with the HOP affinity molecule via the NHS group. The HOP affinity molecule that is attached to the chemical linker is then contacted with the surface of the solid support and subjected to UV radiation to activate the second functional group and link the protein to the solid support. In this way, the chemical cross linker reacts with either the protein or the surface of the solid support in discrete reactions to limit undesirable side reactions, such as intra protein cross linking.

In one option, when HOP TPR1 (SEQ ID NO: 1) is present in the HOP affinity molecule used for purification, the multichaperone-antigen complexes comprise the following HSPs: HSP60, HSP70, HSP90, HSP110, HSP40, HIP, and Calreticulin. In one option, when HOP TPR1 (SEQ ID NO: 1) is present in the HOP affinity molecule used for purification, the multichaperone-antigen complexes comprise the following HSPs: HSP70, HSP90, and HSP110. In a specific option, when HOP TPR1 (SEQ ID NO: 1) is present in the HOP affinity molecule used for purification, the multichaperone-antigen complexes comprise the following HSPs: HSP70 and HSP90.

In a specific option, when HOP TPR2a (SEQ ID NO: 2) is present in a HOP affinity molecule used for purification, the multichaperone-antigen complexes comprise HSP90. In another option, when HOP TPR1/2a (SEQ ID NO: 3) is present in a HOP affinity molecule used for purification, the multichaperone-antigen complexes comprise the following HSPs: HSP60, HSP70, HSP90, HSP110, HSP40, HIP, BIP and Calreticulin.

In one option, when HOP TPR1/2a (SEQ ID NO: 3) is present in a HOP affinity molecule used for purification, the multichaperone-antigen complexes comprise the following HSPs: HSP70 and HSP90. In a specific option, when HOP TPR1/2a (SEQ ID NO: 3) is present in a HOP affinity molecule used for purification, the multichaperone-antigen complexes comprise the following HSPs: HSP70, HSP90, and HSP110.

In one option, when HOP TPR1 (SEQ ID NO: 1) and HOP TPR1/2a (SEQ ID NO: 3) are present in the HOP affinity molecules of a mixed resin bed used for purification, the multichaperone-antigen complexes comprise the following HSPs: HSP60, HSP70, HSP90, HSP110, HSP40, HIP, and Calreticulin. In another option, when HOP TPR1 (SEQ ID NO: 1) and HOP TPR1/2a (SEQ ID NO: 3) are present in the HOP affinity molecules of a mixed resin bed used for purification, the multichaperone-antigen complexes comprise the following HSPs: HSP70, HSP90, and HSP110.

In a specific option, when HOP TPR1 (SEQ ID NO: 1), HOP TPR2a (SEQ ID NO: 2), or HOP TPR1/2a (SEQ ID NO: 3), or any combination of the foregoing are present in the HOP affinity molecule(s) used for purification, the multichaperone- antigen complexes comprise HSP 60 and/or calreticulin in trace amounts (e.g., HSP60 and/or calreticulin comprise less than 5%, less than 4%, less than 3%, less than 2%, or less than 1% of the total protein present in the sample containing the multichaperone-antigen complexes).

HOP affinity molecules can be obtained by recombinant expression, as described in more detail in Section 5.1.1.1, or by chemical synthesis as described in more detail in Section 5.1.1.2.

### 5.1.1.1. Expression of HOP Affinity Molecules

HOP affinity molecules, or portions thereof (e.g., HOP affinity fragments), that are proteins or peptides may be obtained by recombinant expression. Once the nucleotide sequence of a HOP affinity molecule of choice has been identified, the nucleotide sequence can be obtained and cloned into an expression vector for recombinant expression. The expression vector can then be introduced into a host cell for propagation of the HOP affinity molecule. Methods for recombinant production of HOP affinity molecules are described in detail herein.

A nucleic acid construct comprising the nucleotide sequence of a HOP affinity molecule is used. In particular options, a nucleic acid construct can comprise the cDNA sequence of any one of HOP TPR1 (SEQ ID NO: 5), HOP TPR2a (SEQ ID NO: 6), or HOP TPR1/2a (SEQ ID NO: 7), or a variant of any one of the foregoing, or a combination of any one or more of the foregoing. A nucleic acid construct also can further comprise a nucleotide sequence that does not encode a HOP affinity fragment. For example, in one option, a nucleic acid construct comprises the nucleotide sequence of one or more HOP affinity fragments and further comprises the nucleotide sequence of an affinity tag.

The DNA may be obtained by DNA amplification or molecular cloning directly from a tissue, cell culture, or cloned DNA (e.g., a DNA "library") using standard molecular biology techniques (see e.g., Methods in Enzymology, 1987, volume 154, Academic Press; Sambrook et al. 1989, Molecular Cloning - A Laboratory Manual, 2nd Edition, Cold Spring Harbor Press, New York; and Current Protocols in Molecular Biology, Ausubel et al. (eds.), Greene Publishing Associates and Wiley Interscience, New York). Clones derived from genomic DNA may contain regulatory and intron DNA regions in addition to coding regions; clones derived from cDNA will contain only exon sequences. Whatever the source, the HOP affinity molecule gene should be cloned into a suitable vector for propagation of the gene.

In a preferred option, DNA can be amplified from genomic or cDNA by polymerase chain reaction (PCR) amplification using primers designed from the known sequence of a related or homologous HOP affinity fragment. PCR is used to amplify the desired sequence in DNA clone or a genomic or cDNA library, prior to selection. PCR can be carried out, e.g., by use of a thermal cycler and Taq polymerase (Gene Amp®). The polymerase chain reaction (PCR) is commonly used for obtaining genes or gene fragments of interest. For example, a nucleotide sequence encoding a HOP affinity molecule can be generated using PCR primers that flank the nucleotide sequence. Alternatively, a HOP affinity molecule can be cleaved at appropriate sites with restriction endonuclease(s) if such sites are available, releasing a fragment of DNA encoding the HOP affinity molecule. If convenient restriction sites are not available, they may be created in the appropriate positions by site-directed mutagenesis and/or DNA amplification methods known in the art (see, for example, Shankarappa et al, 1992, PCR Method Appl. 1: 277-278). The DNA HOP affinity molecule is then isolated, and ligated into an appropriate expression vector, care being taken to ensure that the proper translation reading frame is maintained.

Any technique for mutagenesis known in the art can be used to modify individual nucleotides in a DNA sequence to make the variants of HOP affinity fragments that are described in Section 5.1.1. above, for purpose of making amino acid substitution(s) in the expressed peptide sequence, or for creating/deleting restriction sites to facilitate further manipulations. Such techniques include but are not limited to, chemical mutagenesis, *in vitro* site-directed mutagenesis (Hutchinson et al, 1978, J. Biol. Chem. 253: 6551), oligonucleotide-directed mutagenesis (Smith, 1985, Ann. Rev. Genet. 19: 423-463; Hill et al, 1987, Methods Enzymol. 155: 558-568), PCR-based overlap extension (Ho et al, 1989, Gene 77: 51-59), PCR-based megaprimer mutagenesis (Sarkar et al, 1990, Biotechniques 8: 404-407), etc. Nucleotide sequences encoding a HOP affinity fragment can be modified by any numerous strategies know in the art (Maniatis, T., 1989, Molecular Cloning, A Laboratory Manual 2d ed., Cold Spring Harbor Laboratory, Cold Spring Harbor, New York). Nucleotide sequences encoding a HOP affinity fragment can be cleaved at appropriate sites with restriction endonuclease(s) followed by further enzymatic modification if desired, isolated, and ligated *in vitro.* Modifications can be confirmed by double stranded dideoxynucleotide DNA sequencing.

Nucleotide sequences encoding a HOP affinity molecule can be inserted into the expression vector for propagation and expression in recombinant cells. An expression construct, as used herein, refers to a nucleotide sequence encoding a HOP affinity molecule operably associated with one or more regulatory regions which allows expression of the HOP affinity molecule in an appropriate host cell. "Operably-associated" refers to an association in which the regulatory regions and the HOP affinity molecule polypeptide sequence to be expressed are joined and positioned in such a way as to permit transcription, and ultimately, translation of the HOP affinity molecule sequence. A variety of expression vectors may be used for the expression of a HOP affinity molecule, including, but not limited to, plasmids, cosmids, phage, phagemids, or modified viruses. Examples include bacteriophages such as lambda derivatives, or plasmids such as pET24a(+). Typically, such expression vectors comprise a functional origin of replication for propagation of the vector in an appropriate host cell, one or more restriction endonuclease sites for insertion of the HOP affinity molecule gene sequence, and one or more selection markers.

Also disclosed herein are host cells, preferably mammalian or bacterial host cells, for expression of HOP affinity molecules. A preferred bacterial host cell is *E*. *coli.* In one embodiment, the vector used includes a prokaryotic origin of replication or replicon, *i.e*., a DNA sequence having the ability to direct autonomous replication and maintenance of the recombinant DNA molecule extra-chromosomally in a prokaryotic host cell, such as a bacterial host cell, transformed therewith. Such origins of replication are well known in the art. Preferred origins of replication are those that are efficient in the host organism. See Sambrook et al., in "Molecular Cloning: a Laboratory Manual", 2nd edition, Cold Spring Harbor Laboratory Press, New York (1989).

For expression of HOP affinity molecules in mammalian host cells, a variety of regulatory regions can be used, for example, the SV40 early and late promoters, the cytomegalovirus (CMV) immediate early promoter, and the Rous sarcoma virus long terminal repeat (RSV-LTR) promoter. Inducible promoters that may be useful in mammalian cells include but are not limited to those associated with the metallothionein II gene, mouse mammary tumor virus glucocorticoid responsive long terminal repeats (MMTV-LTR), the β-interferon gene, and the HSP70 gene (Williams et al., 1989, Cancer Res. 49: 2735-42 ; Taylor et al., 1990, Mol. Cell. Biol. 10: 165-75). The efficiency of expression of the HOP affinity molecules in a host cell may be enhanced by the inclusion of appropriate transcription enhancer elements in the expression vector, such as those found in SV40 virus, Hepatitis B virus, cytomegalovirus, immunoglobulin genes, metallothionein, β-actin (see Bittner et al., 1987, Methods in Enzymol. 153: 516-544; Gorman, 1990, Curr. Op. in Biotechnol. 1: 36-47).

The expression vector may also contain sequences that permit maintenance and replication of the vector in more than one type of host cell, or integration of the vector into the host chromosome. Such sequences may include but are not limited to replication origins, autonomously replicating sequences (ARS), centromere DNA, and telomere DNA. It may also be advantageous to use shuttle vectors that can be replicated and maintained in at least two types of host cells.

In addition, the expression vector may contain selectable or screenable marker genes for initially isolating or identifying host cells that contain DNA encoding a HOP affinity fragment. For long term, high yield production of HOP affinity molecules, stable expression in mammalian cells is preferred. A number of selection systems may be used for mammalian cells, including, but not limited, to the Herpes simplex virus thymidine kinase (Wigler et al., 1977, Cell 11: 223), hypoxanthine-guanine phosphoribosyltransferase (Szybalski and Szybalski, 1962, Proc. Natl. Acad. Sci. U.S.A. 48: 2026), and adenine phosphoribosyltransferase (Lowy et al., 1980, Cell 22: 817) genes can be employed in *tk*⁻, *hgprt*⁻ or *aprt*⁻ cells, respectively. Also, antimetabolite resistance can be used as the basis of selection for dihydrofolate reductase (*dhfr*), which confers resistance to methotrexate (Wigler et al., 1980, Proc. Natl. Acad. Sci. U.S.A. 77: 3567; O'Hare et al., 1981, Proc. Natl. Acad. Sci. U.S.A. 78: 1527); gpt, which confers resistance to mycophenolic acid (Mulligan and Berg, 1981, Proc. Natl. Acad. Sci. U.S.A. 78: 2072); neomycin phosphotransferase (neo), which confers resistance to the aminoglycoside G-418 (Colberre-Garapin et al., 1981, J. Mol. Biol. 150: 1); and hygromycin phosphotransferase (hyg), which confers resistance to hygromycin (Santerre et al., 1984, Gene 30: 147). Other selectable markers, such as but not limited to histidinol and Zeocin™ can also be used.

Expression constructs containing cloned HOP affinity molecule cDNA sequences can be introduced into the mammalian or bacterial host cell by a variety of techniques known in the art, including but not limited to calcium phosphate mediated transfection (Wigler et al., 1911 , Cell 11 223-232), liposome-mediated transfection (Schaefer-Ridder et al., 1982, Science 215: 166-168), electroporation (Wolff et al. , 1987, Proc. Natl. Acad. Sci. 84: 3344), and microinjection (Cappechi, 1980, Cell 22: 479-488).

Any of the cloning and expression vectors described herein may be synthesized and assembled from known DNA sequences by techniques well known in the art. The regulatory regions and enhancer elements can be of a variety of origins, both natural and synthetic. Some vectors and host cells may be obtained commercially. Non-limiting examples of useful vectors are described in Appendix 5 of Current Protocols in Molecular Biology, 1988, ed. Ausubel et al, Greene Publish. Assoc. & Wiley Interscience; and the catalogs of commercial suppliers such as Clontech Laboratories, Stratagene Inc., and Invitrogen, Inc.

Alternatively, a number of viral-based expression systems may also be utilized with mammalian cells for recombinant expression of HOP affinity fragments. Vectors using DNA virus backbones have been derived from simian virus 40 (SV40) (Hamer et al, 1979, Cell 17: 725), adenovirus (Van Doren et al., 1984, Mol. Cell Biol. 4: 1653), adeno-associated virus (McLaughlin et al., 1988, J. Virol. 62: 1963), and bovine papillomas virus (Zinn et al., 1982, Proc. Natl. Acad. Sci. 79: 4897). Also, BPV vectors such as pBCMGSNeo and pBCMGHis may be used to express HOP affinity fragments (Karasuyama et al., Eur. J. Immunol. 18: 97-104; Ohe et al., Human Gene Therapy 6: 325-33) which may then be transfected into a diverse range of cell types for HOP affinity fragment expression. Alternatively, the vaccinia 7.5K promoter may be used (see, e.g., Mackett et al., 1982, Proc. Natl. Acad. Sci. U.S.A. 79: 7415-7419; Mackett et al., 1984, J. Virol. 49: 857-864; Panicali et al., 1982, Proc. Natl. Acad. Sci. U.S.A. 79: 4927-4931) In cases where a human host cell is used, vectors based on the Epstein-Barr virus (EBV) origin (OriP) and EBV nuclear antigen 1 (EBNA-1; a trans-acting replication factor) may be used. Such vectors can be used with a broad range of human host cells, e.g., EBO-pCD (Spickofsky et al, 1990, DNA Prot. Eng. Tech. 2: 14-18), pDR2 and λDR2 (available from Clontech Laboratories). Recombinant HOP affinity fragment expression can also be achieved by a retrovirus-based expression system. In retroviruses such as Moloney murine leukemia virus, most of the viral gene sequences can be removed and replaced with an HOP affinity fragment coding sequence, while the missing viral functions can be supplied in *trans.*

The recombinant cells may be cultured under standard conditions of temperature, incubation time, optical density, and media composition. Alternatively, cells may be cultured under conditions emulating the nutritional and physiological requirements of a cell in which the HOP is endogenously expressed. Modified culture conditions and media may be used to enhance production of HOP affinity molecules. For example, recombinant cells may be grown under conditions that promote inducible HOP affinity fragment expression.

Isolation of untagged HOP affinity molecules from cell broth can be accomplished using standard chromatography methods of ion exchange, hydrophobic interaction chromatography (HIC) and gel filtration. In a preferred option, a combination of these techniques is used to purify individual HOP affinity fragments. It may be advantageous to reduce the complexity of the cell broth using one or more ammonium sulfate precipitation steps. A typical work flow would be to break open the cells, clarify the homogenate by centrifugation, perform an ammonium sulfate precipitation, isolate the HOP affinity fragment by a first chromatography step such as hydrophobic interaction chromatography followed by polishing using a second chromatography step such as DEAE anion exchange chromatography. The isolated reagent would then be exchanged into a buffer suitable for conjugation to a solid phase.

HOP affinity molecules of the invention may also be expressed as fusion proteins, including concatamers, to facilitate recovery and purification from the cells in which they are expressed. For example, a HOP affinity molecule may contain a signal sequence leader peptide to direct its translocation across the ER membrane for secretion into culture medium. Furthermore, a HOP affinity molecule may contain an affinity label, such as a histidine tag, fused to any portion of the HOP affinity molecule not involved in binding multichaperone-antigen complexes, such as for example, the carboxyl terminus. The affinity label can be used to facilitate purification of the protein, by binding to an affinity partner molecule.

Various methods for production of such fusion proteins, including concatamers, are well known in the art. The manipulations which result in their production can occur at the gene or protein level, preferably at the gene level. For example, the cloned HOP affinity molecule may be modified by any of numerous recombinant DNA methods known in the art (Sambrook et al., 1990, Molecular Cloning, A Laboratory Manual, 2d ed., Cold Spring Harbor Laboratory, Cold Spring Harbor, New York; Ausubel et al, in Chapter 8 of Current Protocols in Molecular Biology, Greene Publishing Associates and Wiley Interscience, New York).

In certain options, fusion proteins comprising HOP affinity fragments may be made using recombinant DNA techniques. For example, a recombinant gene encoding a HOP affinity fragment polypeptide may be constructed by introducing a HOP affinity fragment gene fragment in the proper reading frame into a vector containing the sequence of an affinity label, such that the HOP affinity fragment polypeptide is expressed as a peptide-tagged fusion protein. Affinity labels, which may be recognized by specific binding partners, may be used for affinity purification of the HOP affinity molecule polypeptide. For example, HOP affinity molecule consisting of a histidine-tagged HOP affinity fragment fusion protein can be loaded on a nickel column, nickel being the specific binding partner for histidine to isolate the histidine-tagged HOP affinity fragment fusion protein. The histidine-tagged HOP affinity fragment fusion protein can be further purified by gel filtration.

In a preferred option, the affinity label is fused via a peptide bond to the amino terminus, or, preferably, the carboxy terminus of a HOP affinity fragment, resulting in a fusion protein.

A variety of affinity labels known in the art may be used, such as, but not limited to, the immunoglobulin constant regions, polyhistidine sequence (Petty, 1996, Metal- chelate affinity chromatography, in Current Protocols in Molecular Biology, Vol. 2, Ed. Ausubel et al, Greene Publish. Assoc. & Wiley Interscience), glutathione S-transferase (GST; Smith, 1993, Methods Mol. Cell Bio. 4:220-229), the E. coli maltose binding protein (Guan et al., 1987, Gene 67:21-30), and various cellulose binding domains (U.S. Patent Nos. 5,496,934; 5,202,247; 5,137,819; Tomme et al., 1994, Protein Eng. 7: 117-123), etc. Other affinity labels may impart fluorescent properties to an HOP affinity fragment polypeptide, e.g., portions of green fluorescent protein and the like. Other possible affinity labels are short amino acid sequences to which monoclonal antibodies are available, such as but not limited to the following well known examples, the FLAG epitope, the myc epitope at amino acids 408-439, the influenza virus hemagglutinin (HA) epitope. Other affinity labels are recognized by specific binding partners and thus facilitate isolation by affinity binding to the binding partner which can be immobilized onto a solid support. Some affinity labels may afford the HOP affinity fragment novel structural properties, such as the ability to form multimers. These affinity labels are usually derived from proteins that normally exist as homopolymers. Affinity labels such as the extracellular domains of CD8 (Shiue et al, 1988, J. Exp. Med. 168: 1993-2005), or CD28 (Lee et al, 1990, J. Immunol. 145:344-352), or portions of the immunoglobulin molecule containing sites for interchain disulfide bonds, could lead to the formation of multimers. As will be appreciated by those skilled in the art, many methods can be used to obtain the coding region of the above-mentioned affinity labels, including but not limited to, DNA cloning, DNA amplification, and synthetic methods. Some of the affinity labels and reagents for their detection and isolation are available commercially.

An affinity label can be a non-variable portion of an immunoglobulin molecule. Typically, such portions comprise at least a functionally operative CH2 and CH3 domain of the constant region of an immunoglobulin heavy chain. Fusions are also made using the carboxyl terminus of the Fc portion of a constant domain, or a region immediately amino-terminal to the CH1 of the heavy or light chain. Suitable immunoglobulin-based affinity label may be obtained from IgG-1, -2, -3, or-4 subtypes, IgA, IgE, IgD, or IgM, but preferably IgG1. Many DNA encoding immunoglobulin light or heavy chain constant regions is known or readily available from cDNA libraries. See, for example, Adams et al, Biochemistry, 1980, 19:2711-2719; Gough et al, 1980, Biochemistry, 19:2702-2710; Dolby et al, 1980, Proc. Natl. Acad. Sci. U.S.A., 77:6027-6031; Rice et al, 1982, Proc. Natl. Acad. Sci. U.S.A., 79:7862-7865; Falkner et al, 1982, Nature, 298:286-288; and Morrison et al, 1984, Ann. Rev. Immunol, 2:239-256. Similarly, if the affinity label is an epitope with readily available antibodies, such reagents can be used with the techniques mentioned above to detect, quantitate, and isolate the HOP affinity fragment polypeptide containing the affinity label. In many instances, there is no need to develop specific antibodies to the HOP affinity fragment polypeptide.

Various leader sequences known in the art can be used for the efficient secretion of HOP affinity fragment polypeptide from bacterial and mammalian cells (von Heijne, 1985, J. Mol. Biol. 184:99-105). Leader peptides are selected based on the intended host cell, and may include bacterial, yeast, viral, animal, and mammalian sequences. For example, the herpes virus glycoprotein D leader peptide is suitable for use in a variety of mammalian cells. A preferred leader peptide for use in mammalian cells can be obtained from the V-J2-C region of the mouse immunoglobulin kappa chain (Bernard et al., 1981, Proc. Natl. Acad. Sci. 78:5812-5816). Preferred leader sequences for targeting HOP affinity fragment polypeptide expression in bacterial cells include, but are not limited to, the leader sequences of the *E. coli* proteins OmpA (Hobom et al., 1995, Dev. Biol. Stand. 84:255-262), Pho A (Oka et al., 1985, Proc. Natl. Acad. Sci 82:7212-16), OmpT (Johnson et al., 1996, Protein Expression 7:104-113), LamB and OmpF (Hoffman & Wright, 1985, Proc. Natl. Acad. Sci. USA 82:5107-5111), β-lactamase (Kadonaga et al., 1984, J. Biol. Chem. 259:2149-54), enterotoxins (Morioka-Fujimoto et al., 1991, J. Biol. Chem. 266:1728-32), and the *Staphylococcus aureus* protein A (Abrahmsen et al., 1986, Nucleic Acids Res. 14:7487-7500), and the *B. subtilis* endoglucanase (Lo et al., Appl. Environ. Microbiol. 54:2287-2292), as well as artificial and synthetic signal sequences (MacIntyre et al., 1990, Mol. Gen. Genet. 221:466-74; Kaiser et al., 1987, Science, 235:312-317).

DNA sequences encoding a desired affinity label or leader peptide, which may be readily obtained from libraries, produced synthetically, or may be available from commercial suppliers, are suitable for the preparation of HOP affinity molecules. Such methods are well known in the art.

### 5.1.1.2. Chemical Synthesis of HOP Affinity Molecules

HOP affinity molecules alternatively can be obtained by chemical synthesis. The HOP affinity molecules or portions thereof that are proteins or peptides can be synthesized by standard chemical methods including the use of a peptide synthesizer. Conventional peptide synthesis or other synthetic protocols well known in the art can be used.

Peptides having the amino acid sequence of a HOP affinity molecule or HOP affinity fragment or a variant thereof can be synthesized, for example, by solid-phase peptide synthesis using procedures similar to those described by Merrifield, 1963, J. Am. Chem. Soc., 85:2149. During synthesis, N-α-protected amino acids having protected side chains are added stepwise to a growing polypeptide chain linked by its C-terminal and to an insoluble polymeric support *i.e*., polystyrene beads. The peptides are synthesized by linking an amino group of an N-α-deprotected amino acid to an α-carboxyl group of an N-α-protected amino acid that has been activated by reacting it with a reagent such as dicyclohexylcarbodiimide. The attachment of a free amino group to the activated carboxyl leads to peptide bond formation. The most commonly used N-α-protecting groups include Boc which is acid labile and Fmoc which is base labile. Details of appropriate chemistries, resins, protecting groups, protected amino acids and reagents are well known in the art and so are not discussed in detail herein (See, Atherton, et al, 1989, Solid Phase Peptide Synthesis: A Practical Approach, IRL Press, and Bodanszky, 1993, Peptide Chemistry, A Practical Textbook, 2nd Ed., Springer-Verlag).

In addition, HOP affinity molecules that comprise variants of HOP affinity fragments can be chemically synthesized as described *supra.* If desired, nonclassical amino acids or chemical amino acid analogs can be introduced as a substitution or addition into the peptide sequence. Non-classical amino acids include, but are not limited to, the D-isomers of the common amino acids, α-amino isobutyric acid, 4-aminobutyric acid, hydroxyproline, sarcosine, citrulline, cysteic acid, t-butylglycine, t-butylalanine, phenylglycine, cyclohexylalanine, β-alanine, designer amino acids such as β-methyl amino acids, Cα-methyl amino acids, and Nα-methyl amino acids.

Purification of the resulting peptide is accomplished using conventional procedures, such as preparative HPLC using gel permeation, partition and/or ion exchange chromatography. The choice of appropriate matrices and buffers are well known in the art and so are not described in detail herein.

### 5.1.1.3. Immobilizing HOP Affinity Molecules on a Solid Phase

There is herein disclosed one or more HOP affinity molecules covalently bound to a solid phase, for use in isolating multichaperone-antigen complexes. Attachment of one or more HOP affinity molecules to the solid phase can be accomplished in any of various ways known to those skilled in the art, including but not limited to chemical cross-linking. Chemical crosslinking methods are well known in the art (see also a description of chemical crosslinking in Section 5.1.1).

In one embodiment, the one or more HOP affinity molecules covalently bound to a solid phase comprise or consist of HOP TPR1 (SEQ ID NO: 1) or a variant thereof, HOP TPR2a (SEQ ID NO: 2) or a variant thereof and/or HOP TPR1/2A (SEQ ID NO: 3) or a variant thereof, or a combination of any one or more of the foregoing. In one option, the HOP affinity molecules that are covalently bound on a solid phase are fusion proteins of two or more different HOP affinity fragments or variants thereof, such as those described in Section 5.1.1.

In a specific option, the HOP affinity molecules that are covalently bound on a solid phase are fusion proteins that comprise a protein sequence other than a HOP affinity fragment or a variant thereof. For example, in one option, a HOP affinity molecule that is covalently bound to a solid phase is a fusion protein that comprises an affinity label. In another option, a HOP affinity molecule that is covalently bound on a solid phase does not contain an affinity label. A fusion protein of two or more of HOP TPR1 or a variant thereof, HOP TPR2a or a variant thereof and/or HOP TPR1/2A or a variant thereof may be covalently bound to a solid phase. In yet another option, HOP affinity molecules are covalently bound on a solid phase as a concatamer, wherein two or more of a particular HOP affinity fragments or a variant thereof is present, such as those described in Section 5.1.1. In one option, a concatamer of two or more of HOP TPR1 or a variant thereof is covalently bound to a solid phase. In another embodiment, a concatamer of two or more of HOP TPR2a or a variant thereof is covalently bound to a solid phase. In another option, a concatamer of two or more of HOP TPR1/2a or a variant thereof is covalently bound to a solid phase.

The HOP affinity molecule that is covalently bound to a solid phase may be a concatamer-fusion protein hybrid, which comprises two or more of one particular HOP affinity fragment or a variant thereof and a protein sequence of a different HOP affinity fragment or a variant thereof such as those described in Section 5.1.1. In another option, a HOP affinity molecule that is covalently bound to a solid phase is a concatamer-fusion protein hybrid, which comprises two or more of one particular HOP affinity fragment or a variant thereof and a protein sequence other than a HOP affinity fragment or a variant thereof such as those described in Section 5.1.1.

The solid phase to which HOP affinity molecules are covalently bound may be a mixed resin bed comprising a first bead/resin to which a particular HOP affinity molecule is covalently bound and a second bead/resin to which a different HOP affinity molecule is covalently bound. In another option, a mixed resin bed comprises a first bead/resin to which a HOP affinity molecule comprising a first HOP affinity fragment or variant there of is covalently bound and a second bead/resin to which a different HOP affinity molecule comprising a second different HOP affinity fragment or variant thereof is covalently bound. In a specific option, a mixed resin bed comprises a HOP affinity molecule comprising HOP TPR1 (SEQ ID NO: 1) and a different HOP affinity molecule comprising HOP TPR1/2a (SEQ ID NO: 3). In another option, a single bead/resin that is present in a mixed resin bead is covalently bound to two or more different HOP affinity molecules. Thus, the different HOP affinity molecules of a mixed resin bed can be covalently bound to the same and/or different beads/resins.

After the one or more HOP affinity molecules are immobilized on a solid phase, unbound HOP affinity molecules are removed after the binding reaction of the HOP affinity molecules to the solid phase by washing the solid phase or by otherwise separating the solid phase from unbound HOP affinity molecules. The solid phase may be any surface or matrix suitable in the art for affinity purification purposes, such as, but not limited to, polycarbonate, polystyrene, polypropylene, polyethylene, glass, nitrocellulose, dextran, nylon, polyacrylamide and agarose. The solid phase can comprise beads, such as magnetic beads, membranes, microparticles, the interior surface of a reaction vessel such as a microtiter plate, test tube or other reaction vessel. The solid phase may comprise beads, such as magnetic beads. The beads are preferably functionalized with a chemical reactive group (e.g., NHS, aldehyde, epoxy, azolactone) to attach the HOP affinity molecules to the solid phase.

In another option, the solid phase comprises beads that are not packed in a column, but are used in batch mode. Batch mode is a process where a solid phase is incubated with a solution to effect extraction of a component or components of the solution. Typically, such incubations can be carried out in a closed container that is rotated end over end. By way of example, but not limitation, extractions are performed at room temperature or in a cold room at 2 to 8 degrees °C. Following this initial incubation, the slurry is mechanically separated by centrifugation, by the use of magnets to attract magnetic beads, or poured into a device that contains a membrane or frit. Following separation of the beads from the solution, the beads are usually washed with a suitable solvent, such as a buffer, using a similar approach (e.g. adding the solvent to the beads and incubating with end over end rotation). Subsequently, the beads are isolated and eluted with a different solvent. Again, this may be by incubation with end over end rotation. The extract is recovered by mechanic separation of the beads and solvent. There are many variations of this technique.

Monolithic columns are alternative solid phases that can be used. Monolithic columns can be thought of as fused beads that form a continuous column of chromatographic media. They can be polymeric or silica based devices. Some advantages to the use of monolithic columns include higher separation efficiency, and the column can be used at higher flow rates, which leads to shorter process times without sacrificing separation efficiency.

HOP affinity molecules may be covalently bound to NHS- Sepharose. In another option, HOP affinity molecules are covalently bound to aldehyde activated membranes. In another option, HOP affinity molecules are covalently bound to aldehyde functionalized magnetic beads, wherein HOP affinity molecules are linked by condensation of amine residues of the HOP fragment with the aldehyde of the beads. Azolactone functionalized beads will also react with amines to immobilize proteins.

### 5.1.2. Preparation of Biological Samples

In the methods disclosed herein, biological samples containing cellular proteins (HSPs and antigens) may be contacted with HOP affinity molecules covalently bound to the solid phase, in order to effect isolation of multichaperone-antigen complexes that contain antigens displaying the antigenicity of the cell from which the multichaperone-antigen complexes are isolated. The multichaperone-antigen complexes can then be administered to a subject to produce *in vivo* in the subject an immune response against the antigen(s), which is therapeutically useful where the cell displays the antigenicity of an infectious agent or cancer cell or other pathologic substance. The biological samples are preferably derived from a cancer cell or an infected cell. For example, for the treatment of cancer, in a specific embodiment, the biological samples are prepared, postoperatively, from tumor cells obtained from a cancer patient.

The biological samples can be obtained from one or more cellular fraction(s) containing cellular proteins, for example, the cytosol of the antigenic cells or the total cell lysate of the antigenic cells. Any technique known in the art for cell lysis or fractionation of cellular contents can be used. See, for example, Current Protocols in Immunology, vol. 2, chapter 8, Coligan et al. (ed.), John Wiley & Sons, Inc.; Pathogenic and Clinical Microbiology: A Laboratory Manual by Rowland et al, Little Brown & Co., June 1994.

In one option, the biological sample is a total cell lysate or whole cell extract which is not fractionated and/or purified. In another option, the biological sample is a cellular protein fraction.

To make a biological sample from cells, the lysing of cells or disruption of cell walls, or cell membranes can be performed using standard protocols known in the art. In various options, the cells can be lysed, for example, by mechanical shearing, sonication, freezing and thawing, adjusting the osmolarity of the medium surrounding the cells, or a combination of techniques. In less preferred options, the antigenic cells can be lysed by chemicals, such as detergents.

The methods disclosed herein may use biological samples derived from cancer cells, preferably human cancers, As used herein, the term "cells or tissue of the same type of cancer" refers to cells or tissue of cancer of the same tissue type, or metastasized from cancer of the same tissue type. In a specific option, the biological sample is a tumor cell extract, for example, a mammalian tumor cell extract. In one option, the biological sample is a human tumor cell extract. In a specific embodiment, the biological sample is primary tumor tissue that was excised from a mammal (e.g., a human), such as a tumor biopsy.

The methods disclosed herein may use biological samples derived from cells infected by a pathogen or infectious agent that causes the infectious disease. In a specific option, the pathogen is a virus, bacterium, fungus, protozoan, or a parasite. Preferably, the pathogen is one that infects humans. The biological sample may be an infected cell extract. In another option, the biological sample is a mammalian infected cell extract. In a specific option, the biological sample is a human infected cell extract.

The methods disclosed herein may use biological samples derived from cells, preferably mammalian or human cells. In one option, any tissues, or cells isolated from a cancer, including a cancer that has metastasized to multiple sites, can be used as an antigenic cell from which a biological sample is derived. For example, a biological sample can be derived from leukemic cells circulating in blood, lymph or other body fluids. Biological samples can also be derived from solid tumor tissue (e.g., primary tissue from a biopsy).

In another option, the cell from which the biological sample is derived is a preneoplastic cell, which is a cell in transition from a normal to a neoplastic form. The transition from non-neoplastic cell growth to neoplasia commonly consists of hyperplasia, metaplasia, and dysplasia (for review of such abnormal growth conditions (See Robbins and Angell, 1976, Basic Pathology, 2d Ed., W.B. Saunders Co., Philadelphia, pp. 68-79). A non-limiting list of cancers, the cells of which can be used herein is provided in Section 5.4.1 below.

In another option, any cell that is infected with a pathogen or infectious agent, i.e., an infected cell, can be used as a cell from which a biological sample is derived. In particular, cells infected by an intracellular pathogen, such as a virus, bacterium, fungus, parasite, or protozoan, are preferred. An exemplary list of infectious agents that can infect cells which can be used as described herein is provided in Section 5.4.2. below.

In yet another option, any pathogen or infectious agent that can cause an infectious disease can be used as the source from which a biological sample is derived. Variants of a pathogen or infectious agent, such as but limited to replication-defective variants, non-pathogenic or attenuated variants, non-infectious variants, can also be used for this purpose. For example, many viruses, bacteria, fungi, parasites and protozoans that can be cultured in vitro or isolated from infected materials can serve as a source from which a biological sample is derived. Methods known in the art for propagating such pathogens including viral particles can be used. An exemplary list of pathogens or infectious agents provided in Section 5.4.2. below.

Cell lines derived from cancer tissues, cancer cells, or infected cells can also be used as cells from which a biological sample is derived. Cancer or infected tissues, cells, or cell lines of human origin are preferred. Cancer cells, infected cells, or other cells can be identified and isolated by any method known in the art. For example, cancer cells or infected cells can be identified by morphology, enzyme assays, proliferation assays, or the presence of pathogens or cancer-causing viruses. If the characteristics of antigens of interest are known, cells can also be identified or isolated by any biochemical or immunological methods known in the art. For example, cancer cells or infected cells can be isolated by surgery, endoscopy, other biopsy techniques, isolation from body fluids (such as blood), affinity chromatography, and fluorescence activated cell sorting (e.g., with fluorescently tagged antibody against an antigen expressed by the cells).

Also disclosed herein, one or more antigenic proteins or peptides of interest may be synthesized in cell lines modified by the introduction of recombinant nucleic acids that encode such antigens, and such cells are used to prepare the biological samples. For example, one or more antigens of an infectious agent can be synthesized in cell lines modified by the introduction of recombinant nucleic acids that encode such antigens of an infectious agent.

If the number of cells obtained from a subject is insufficient for obtaining a biological sample, the cells may be cultured in vitro by standard methods to expand the number of cells prior to use in the present methods. There is no requirement that a clonal or homogeneous or purified population of antigenic cells be used for obtaining a biological sample. A mixture of cells can be used provided that a substantial number of cells in the mixture contain the antigenic determinants or antigens of interest. In one option, the antigenic cells and/or immune cells are purified prior to deriving a biological sample therefrom.

In order to prepare pathogen-infected cells, uninfected cells of a cell type susceptible to infection by the pathogen or infectious agent that causes the disease can be infected *in vitro.* Depending on the mode of transmission and the biology of the pathogen or infectious agent, standard techniques can be used to facilitate infection by the pathogen or infectious agent, and propagation of the infected cells. For example, influenza viruses may be used to infect normal human fibroblasts; and mycobacteria may be used to infect normal human Schwann cells. In some options, variants of an infectious agent, such as replication-defective viruses, non-pathogenic or attenuated mutants, or temperature- sensitive mutants can also be used to infect or transform cells to generate antigenic cells for the preparation of antigenic peptides. If large numbers of a pathogen are needed to infect cells, or if pathogens are used directly as antigenic cells, any method known in the art can be used to propagate and grow the pathogens. Such methods will depend on the pathogen, and may not involve infecting a host. For example, many techniques are known in the art for growing pathogenic bacteria, fungi and other non-viral microorganisms in culture, including large scale fermentation.

In one option, a biological sample is an extract of an engineered cell. In a specific embodiment, the engineered cell is a recombinant cell. In particular, if the gene encoding a tumor antigen (e.g., tumor-specific antigen and tumor-associated antigen) or antigen of the pathogen is available, normal cells of the appropriate cell type from the intended recipient may be transformed or transfected in vitro with an expression construct comprising a nucleic acid molecule encoding such antigen, such that the antigen is expressed in the recipient's cells. In another option, a tumor-associated antigen is an antigen that is expressed at a higher level in a tumor cell relative to a normal cell; a tumor-specific antigen is an antigen that is expressed only in a tumor cell and not in a normal cell. Optionally, more than one such antigen may be expressed in the recipient's cell in this fashion, as will be appreciated by those skilled in the art, any techniques known, such as those described in Ausubel et al. (1989, Current Protocols in Molecular Biology, Wiley Interscience), may be used to perform the transformation or transfection and subsequent recombinant expression of the antigen gene in recipient's cells.

Suitable proteins and peptides that may be expressed in such cells include, but are not limited to those displaying the antigenicity of cancer cells. For example, such tumor specific or tumor-associated antigens include but are not limited to KS 1/4 pan-carcinoma antigen (Perez and Walker, 1990, J. Immunol. 142:3662-3667; Bumal, 1988, Hybridoma 7(4):407-415); ovarian carcinoma antigen (CA125) (Yu, et al, 1991, Cancer Res. 51(2):468-475); prostatic acid phosphate (Tailer, et al, 1990, Nucl. Acids Res. 18(16):4928); prostate specific antigen (Henttu and Vihko, 1989, Biochem. Biophys. Res. Comm. 160(2):903-910; Israeli, et al, 1993, Cancer Res. 53:227-230); melanoma-associated antigen p97 (Estin, et al, 1989, J. Natl. Cancer Inst. 81(6):445-446); melanoma antigen gp75 (Vijayasardahl, et al, 1990, J. Exp. Med. 171(4): 1375-1380); high molecular weight melanoma antigen (Natali, et al, 1987, Cancer 59:55-63), MAGE-A3 (Kocher, et al, 1995, Cancer Res.;55:2236-2239), NY-ESO-1 (Chen et al, 1997, Proc. Natl. Acad. Sci. 94: 1914-1918, prostate specific membrane antigen, tyrosinase, gp100, melan-A, and mucins. Other proteins and peptides that may be expressed in such cells include peptides or proteins that are mutated at a high frequency in cancer cells, such as oncogenes (e.g., ras, in particular mutants of ras with activating mutations, which only occur in four amino acid residues (12, 13, 59 or 61) (Gedde-Dahl et al, 1994, Eur. J. Immunol. 24(2):410-414)) and tumor suppressor genes (e.g., p53, for which a variety of mutant or polymorphic p53 peptide antigens capable of stimulating a cytotoxic T cell response have been identified (Gnjatic et al, 1995, Eur. J. Immunol. 25(6): 1638-1642). See also the Peptide Database of Cancer immunity at http://www.cancerimmunity.org/peptidedatabase/Tcellepitopes.htm for additional cancer antigens that may be recombinantly expressed in a host cell.

In another option, the biological sample is a cell extract of an engineered cell. In particular, where it is desired to treat or prevent viral diseases, suitable proteins and peptides comprising epitopes of known viruses can be expressed in the appropriate cells. For example, such viruses include, but are not limited to, hepatitis type A, hepatitis type B, hepatitis type C, influenza, varicella, adenovirus, herpes simplex type I (HSV-I), herpes simplex type II (HSV-II), rinderpest, rhinovirus, echovirus, rotavirus, respiratory syncytial virus, papilloma virus, papova virus, cytomegalovirus, echinovirus, arbovirus, huntavirus, coxsackie virus, mumps virus, measles virus, smallpox virus, rubella virus, polio virus, human immunodeficiency virus type I (HIV-I), and human immunodeficiency virus type II (HIV-II).

Preferably, where it is desired to treat or prevent bacterial infections, suitable proteins and peptides comprising epitopes of known bacteria can be expressed in the appropriate cells. For example, such bacterial epitopes may be derived from various bacteria including, but not limited to, Gram positive bacillus (e.g., *Listeria, Bacillus* such as *Bacillus anthracis, Erysipelothrix* species), Gram negative bacillus (e.g., *Bartonella, Brucella, Campylobacter, Enterobacter, Escherichia, Francisella, Hemophilus, Klebsiella, Morganella, Proteus, Providencia, Pseudomonas, Salmonella, Serratia, Shigella, Vibrio,* and *Yersinia* species), spirochete bacteria (e.g., *Borrelia* species including *Borrelia burgdorferi* that causes Lyme disease, and *Leptospira*), anaerobic bacteria (e.g., *Actinomyces* and *Clostridium* species including *C tetani, C botulinum, C perfringens*), Gram positive and negative coccal bacteria, *Streptococcus* species, *Pneumococcus* species, *Staphylococcus* species (e.g., *S. aureus* and *S. pneumonia*), *Neisseria* species (e.g., *N. meningitidis*).

Preferably, where it is desired to treat or prevent fungal infections, suitable proteins and peptides comprising epitopes of known fungi can be expressed in the appropriate cells. For example, such antigenic epitopes may be derived from various fungi including, *Aspergillus (e.g., Aspergillus fumigatus), Cryptococcus (e.g., Cryptococcus neoformans*), *Sporotrix, Coccidioides, Paracoccidioides, Histoplasma, Blastomyces, Candida (e.g., Candida albicans), Rhizopus, Rhizomucor, Absidia,* and *Basidiobolus* species.

Preferably, where it is desired to treat or prevent parasitic infections, suitable proteins and peptides comprising epitopes of known protozoa, nematodes, or helminths can be expressed in the appropriate cells. For example, such antigenic epitopes may be derived from various protozoa including, but not limited to, *Entoamoeba, Plasmodium, Leishmania, Eimeria, Cryptosporidium, Giardiasis, Toxoplasma,* and *Trypanosoma* species.

In a specific option, the biological sample from which the multichaperone- antigen complexes are isolated by HOP affinity molecule methods as described herein, is a biological sample that has been depleted of one or more HSP-antigen complexes. For example, such another biological sample can be flow-through resulting from contacting a biological sample containing cellular proteins with a solid phase to which is bound a binding partner for a heat shock protein. In a specific embodiment, the solid phase to which is bound said binding partner is an anti-gp96 immunoaffinity column (e.g., an anti-gp96 scFv column) and said heat shock protein is gp96.

In one option, the biological sample is flow-through resulting from contacting a tumor cell extract, a pathogen-infected cell extract or an extract of cells transfected with and expressing a nucleic acid encoding a tumor associated antigen or a tumor specific antigen or infectious disease antigen, containing cellular proteins, with a solid phase to which is bound a binding partner for a heat shock protein. In a preferred option, the solid phase to which is bound said binding partner is an anti-gp96 immunoaffinity column and said heat shock protein is gp96

### 5.1.3. Conditions for Purification of Multichaperone-antigen Complexes Using Immobilized HOP Affinity Fragments

Disclosed herein are methods for preparing multichaperone- antigen complexes comprising (a) contacting a biological sample with a solid phase to which HOP affinity molecules are covalently bound, under conditions such that multichaperone-antigen complexes in the biological sample bind said HOP affinity molecules; (b) removing unbound components in the biological sample away from the solid phase; (c) eluting multichaperone-antigen complexes from the solid phase; and (d) recovering the eluted multichaperone-antigen complexes.

The flow through from a purification method of the invention (i.e. the material from the biological sample that does not bind to the HOP affinity molecules on the solid phase) may also be recovered and used to isolate HSP-antigen complexes therefrom, which HSP-antigen complexes can then be combined with the multichaperone-antigen complexes for use in therapy. In a specific option, wherein the HSP in the complexes to be isolated from the flow through is a glycoprotein and the HSP-antigen complexes are isolated using ConA chromatography, as described in Section 5.2, 1 mM to 20 mM (preferably 2 mM) CaCl₂ and 1 mM to 20 mM MgCl₂ (preferably 2 mM) is added to the flow through from the purification method before the flow through is loaded on to the ConA column.

The biological sample that is contacted with the solid phase is preferably in the form of a fluid, most preferably a solution. In one option, a biological sample is contacted with a solid phase to which HOP affinity molecules are covalently bound in a buffered solution containing 1 mM NaCl to 100 mM NaCl. In another option, a biological sample is contacted with a solid phase, to which HOP affinity molecules are covalently bound, in a buffered solution containing 1 mM NaCl, 5 mM NaCl, 10 mM NaCl, 15 mM NaCl, 20 mM NaCl, 25 mM NaCl, 30 mM NaCl, 35 mM NaCl, 40 mM NaCl, 45 mM NaCl, 50 mM NaCl, 55 mM NaCl, 60 mM NaCl, 65 mM NaCl, 70 mM NaCl, 75 mM NaCl, 80 mM NaCl, 85 mM NaCl, 90 mM NaCl, 95 mM NaCl, or 100 mM NaCl such that multichaperone-antigen complexes in the biological sample bind said HOP affinity molecules. In another option, a biological sample is contacted with a solid phase, to which HOP affinity molecules are covalently bound, in a buffer that does not contain NaCl. In another embodiment, a biological sample is contacted with a solid phase, to which HOP affinity molecules are covalently bound, in a buffered solution that has a pH range of 6 to 8.5. In another option, a biological sample is contacted with a solid phase, to which HOP affinity molecules are covalently bound, in a buffered solution that has a pH of 6.0, 6.5, 7.0, 7.5, 8.0, or 8.5.

A biological sample may be contacted with a solid phase, to which HOP affinity molecules are covalently bound, in a buffered solution consisting of 30 mM sodium phosphate, 1.5 mM magnesium chloride, and 50 mM NaCl, at pH 7.2. In a specific embodiment, a biological sample is contacted with a solid phase, to which HOP affinity molecules are covalently bound, in a buffered solution consisting of 30 mM sodium phosphate, 1.5 mM magnesium chloride, at pH 7.2.

In a specific option, a biological sample is contacted with a solid phase to which affinity molecules comprising HOP TPR1 (SEQ ID NO: 1) are covalently bound, in a buffered solution consisting of 30 mM sodium phosphate, 1.5 mM magnesium chloride, and 50 mM NaCl, at pH 7.2. In one option, a biological sample is contacted with a solid phase to which affinity molecules comprising HOP TPR2a (SEQ ID NO: 2) are covalently bound, in a buffered solution consisting of 30 mM sodium phosphate, 1.5 mM magnesium chloride, and 50 mM NaCl, at pH 7.2. In a specific embodiment, a biological sample is contacted with a solid phase, to which affinity molecules comprising HOP TPR1/2a (SEQ ID NO: 3) are covalently bound, in a buffered solution consisting of 30 mM sodium phosphate, 1.5 mM magnesium chloride, at pH 7.2.

In a specific option, wherein the solid phase is packed in a column, a biological sample is loaded onto a solid phase, to which HOP affinity molecules are covalently bound, at a ratio of 0.1 mL resin/1 g to 2 mL resin/ 1 g of tissue from which the biological sample is obtained. In another option, wherein the solid phase is packed in a column, a biological sample is loaded onto a solid phase to which HOP affinity molecules are covalently bound, at a rate of 30 cm/hr to 100 cm/hr. In a preferred option, wherein the solid phase is packed in a column, a biological sample is loaded onto a solid phase to which HOP affinity molecules are covalently bound, at a rate of 70 cm/hr to 100 cm/hr.

After the contacting step, unbound components in the biological sample are removed from the solid phase. In one option, unbound components in a biological sample are removed from the solid phase by washing the solid phase using a buffered solution that is identical to the buffered solution used in the contacting step (e.g., the loading buffer, in the instance where the solid phase is packed in a column). In another option, unbound components in a biological sample are removed from the solid phase by washing the solid phase using a buffered solution containing 1 mM NaCl to 100 mM NaCl. In another option, unbound components in a biological sample are removed from the solid phase by washing the solid phase using a buffered solution containing 1 mM NaCl, 5 mM NaCl, 10 mM NaCl, 15 mM NaCl, 20 mM NaCl, 25 mM NaCl, 30 mM NaCl, 35 mM NaCl, 40 mM NaCl, 45 mM NaCl, 50 mM NaCl, 55 mM NaCl, 60 mM NaCl, 65 mM NaCl, 70 mM NaCl, 75 mM NaCl, 80 mM NaCl, 85 mM NaCl, 90 mM NaCl, 95 mM NaCl, or 100 mM NaCl. In another option, unbound components in a biological sample are removed from the solid phase by washing the solid phase using a buffered solution that does not contain NaCl. In another option, unbound components in a biological sample are removed from the solid phase by washing the solid phase using a buffered solution that has a pH range of 6 to 8.5. In another option unbound components in a biological sample are removed from the solid phase by washing the solid phase using a buffered solution that has a pH range of 6 to 8.5 that has a pH of 6.0, 6.5, 7.0, 7.5, 8.0, or 8.5. In one option, unbound components in a biological sample are removed from the solid phase by washing the solid phase using a buffered solution consisting of 30 mM sodium phosphate, 1.5, mM magnesium chloride, and 50 mM NaCl, at pH 7.2. In one option, unbound components in a biological sample are removed from the solid phase by washing the solid phase using a buffered solution consisting of 30 mM sodium phosphate, 1.5, mM magnesium chloride, at pH 7.2.

In another option, wherein the solid phase is packed in a column, washing buffer is added to the column at a rate of 30 cm/hr to 100 cm/hr. In a preferred option, wherein the solid phase is packed in a column, washing buffer is added to the column at a rate of 70 cm/hr.

After the contacting and removing steps, multichaperone-antigen complexes are eluted from the solid phase. In one option, multichaperone-antigen complexes are eluted from the solid phase to which HOP affinity molecules are covalently bound, with a buffered solution containing 150 mM to 1.5 M NaCl at a pH in the range of 3 to 11. In another option, multichaperone-antigen complexes are eluted from the solid phase with a buffered solution containing 150 mM to 1.5 M NaCl at a pH in the range of 3 to 5 or a pH in the range of 8 to 10. In one option, multichaperone-antigen complexes are eluted from a solid phase to which affinity molecules comprising HOP TPR1 (SEQ ID NO: 1) are covalently bound, with a buffered solution containing 300 mM to 600 mM NaCl at a pH in the range of 8 to 10. In one option, multichaperone-antigen complexes are eluted from a solid phase to which affinity molecules comprising HOP TPR1 (SEQ ID NO: 1) are covalently bound, with a buffered solution containing 500 mM NaCl at pH 9. In another option, multichaperone-antigen complexes are eluted from a solid phase to which affinity molecules comprising HOP TPR1/2a (SEQ ID NO: 3) are covalently bound, with a buffered solution containing 300 mM to 600 mM NaCl at a pH in the range of 8 to 10. In one option, multichaperone-antigen complexes are eluted from a solid phase to which affinity molecules comprising HOP TPR1/2a (SEQ ID NO: 3) are covalently bound, with a buffered solution containing 500 mM NaCl at pH 9. In another option, multichaperone-antigen complexes are eluted from a solid phase to which affinity molecules comprising HOP TPR1/2a (SEQ ID NO: 3) are covalently bound, with a buffered solution containing 500 mM NaCl at pH 7.2 In another option, multichaperone-antigen complexes are eluted from a solid phase to which affinity molecules comprising HOP TPR2a (SEQ ID NO: 2) are covalently bound, with a buffered solution containing 200 mM to 400 mM NaCl at a pH in the range of 6 to 8. In one option, multichaperone-antigen complexes are eluted from a solid phase to which affinity molecules comprising HOP TPR2a (SEQ ID NO: 2) are covalently bound, with a buffered solution containing 300 mM NaCl at pH 7.2. In another option, multichaperone-antigen complexes are eluted from a solid phase to which HOP affinity molecules are covalently bound with a buffered solution containing Tris, phosphate, or glycine.

Multichaperone-antigen complexes may be eluted from a solid phase to which affinity molecules comprising HOP TPR1 (SEQ ID NO: 1) are covalently bound, with a buffered solution consisting of 20 mM Tris and 500 mM NaCl, at pH 9. In one option, multichaperone-antigen complexes are eluted from a solid phase to which affinity molecules comprising HOP TPR2a (SEQ ID NO: 2) are covalently bound, with a buffered solution consisting of 10 mM sodium phosphate and 300 mM NaCl, at pH 7.2. In another specific embodiment, multichaperone-antigen complexes are eluted from a solid phase to which affinity molecules comprising HOP TPR1/2a (SEQ ID NO: 3) are covalently bound, with a buffered solution consisting of either 10 mM sodium phosphate and 500 mM NaCl, at pH 7.2 or 20 mM Tris and 500 mM NaCl, at pH 9.

In another option, where the solid phase is a mixed resin bed (as described in Section 5.1.1.3) comprising an affinity molecule containing HOP TPR1 (SEQ ID NO: 1) and an affinity molecule containing HOP TPR1/2a (SEQ ID NO: 3), multichaperone-antigen complexes are eluted with 20 mM Tris and 500 mM NaCl, at pH 9.

After the multichaperone-antigen complexes are eluted from the solid phase, an additional purification step may optionally be performed, using, for example, DEAE chromatography or hydrophobic interaction chromatography (HIC).

In a recovering step, fractions containing multichaperone-antigen complexes are obtained. After the multichaperone-antigen complexes are recovered, if desired (e.g., if the salt concentration is above physiological levels), the complexes can be subjected to a final buffer exchange step, using, for example, G25 sepharose columns, to formulate the multichaperone-antigen complexes in a pharmaceutically acceptable solution, such as PBS or 9% sucrose-potassium phosphate buffer (5 mM potassium phosphate, 9% sucrose, pH 7). Recovered multichaperone-antigen complexes can be detected in fractions, e.g., by the detection methods described in Section 5.1.4., and the fractions can be subsequently pooled.

### 5.1.4. Detection of Multichaperone-antigen Complexes

The multichaperone-antigen complexes that are recovered by the purification methods described herein can be detected by any method standard in the art, including, but not limited to, western blot analysis, sodium dodecyl sulfate polyacrylamide gel electrophoresis (SDS-PAGE), and/or ELISA. Such assays are routine and well known in the art (*see, e.g.,* Ausubel et al, eds, 1994, Current Protocols in Molecular Biology, Vol. 1, John Wiley & Sons, Inc., New York). Multichaperone-antigen complexes may be considered purified when the HSPs that are present in the preparation containing the multichaperone-antigen complexes account for the majority of protein band intensity on an SDS-PAGE gel.

Particular proteins can be identified in a sample containing multichaperone- antigen complexes by liquid chromatography/tandem mass spectrometry (LC/MS/MS). A procedure that can be used for LC/MS/MS, presented by way of example and not limitation, is as follows: Following separation by SDS-PAGE, bands are excised from the gel and the proteins therein are digested with a protease, e.g., trypsin. Identification of peptide fragments by this treatment is by analysis of digested protein bands with a tandem mass spectrometer (such as an LCQ-Deca Mass Spectrometer (ThermoFisher) and an ADVANCE electrospray ionization (ESI) source (Michrom Bioresources Inc.)) in positive ion mode. To increase the number of peptide fragments that are identified in digested protein bands, samples are preferably separated by reversed phase chromatography (using for example a Surveyor HPLC (ThermoFisher) to deliver solvent to a Luna C18 reversed phase column, 75 µm ID X 10 mm, of 3 µm particles (Phenomenex Inc.). Mobile phases could be modified with 10 mM ammonium hydroxide or low concentrations (e.g. 0.1 to 1% by volume) of an organic acid such as formic or acetic acid). Peptide sequence identities can be determined by searching raw tandem MS (MS/MS) spectra against a library of protein sequences (using for example Mascot software (Matrix Sciences) to search MS/MS spectra against a protein sequence library such as the SwissProt_54.5 database.

### 5.1.5. In Vitro Production of Multichaperone-antigen Complexes

The multichaperone-antigen complexes disclosed herein can be mixed *in vitro* with an excess of antigen(s) of interest in order to complex the antigen(s) of interest to the HSPs in the multichaperone complex. Antigens of cancers and infectious agents known in the art or that can be identified by routine methods may be used and thus synthesized for this purpose. In this option, it is expected that a proportion of the antigens endogenously bound in a non-covalent manner to the multichaperones will be displaced by the synthetic antigens which will in turn form non-covalent complexes with the multichaperone. It is expected that the multichaperones so mixed with the synthetic antigen(s) will be useful in inducing immune response to the synthetic antigen(s) and preventing and treating cancer and infectious diseases, etc.

By way of example and not limitation, the antigens (1 µg) and the multichaperones (9 µg) (optionally pretreated by exposure to ATP or low pH (e.g., pH is 1, 2, 3, 4, 5, or 6 or less than 6, less than 5, or less than 4, or in the range of 4 to 6) or high concentrations of sodium chloride e.g., greater that IM) to release noncovalently bound peptides and proteins) are admixed to give an approximately 5 antigen: 1 HSP molar ratio. Then, the mixture is incubated for 15 minutes to 3 hours at 4° to 45°C in a suitable binding buffer such as one containing 20 mM sodium phosphate, pH 7.2, 350 mM NaCl, 3 mM MgCl₂ and lmM phenyl methyl sulfonyl fluoride (PMSF). The preparations are centrifuged through a Centricon 10 assembly (Millipore) to remove any unbound antigen. The association of the antigens with the HSPs can be assayed by SDS-PAGE. This is the preferred method for *in vitro* complexing of antigens isolated from MHC-antigen complexes or antigens disassociated from endogenous multichaperone-antigen complexes.

Following complexing, the immunogenic multichaperone -antigen complexes can optionally be assayed *in vitro* using for example the mixed lymphocyte target cell assay (MLTC) described below. Once immunogenic complexes have been isolated they can be optionally characterized further in animal models using any of the preferred administration protocols and excipients discussed below.

### 5.1.6. Covalent Multichaaerone-Antiaen Complexes

As an alternative to using non-covalent complexes of multichaperones and antigens, antigens may be covalently attached to multichaperones prior to administration according to the methods disclosed herein. Multichaperone-antigen complexes are preferably crosslinked after their purification from cells or tissues as described in Section 5.1.1. In one embodiment, multichaperones are covalently coupled to antigens by chemical crosslinking. Chemical crosslinking methods are well known in the art. For example, in a preferred option, glutaraldehyde crosslinking may be used. Glutaraldehyde crosslinking has been used for formation of covalent complexes of antigens and HSPs(see Barrios et al, 1992, Eur. J. Immunol. 22: 1365-1372). Preferably, 1-2 mg of HSP-antigen complex is crosslinked in the presence of 0.002% glutaraldehyde for 2 hours and then glutaraldehyde is removed by dialysis against phosphate buffered saline (PBS) overnight (Lussow et al, 1991, Eur. J. Immunol. 21: 2297-2302).

In another option, the multichaperones and specific antigen(s) are crosslinked with a reagent that contains two functional groups that when activated by ultraviolet (UV) light form covalent bonds with specific amino acid residues of the protein.

### 5.2. Combining Multichaperone-antigen Complexes With HSP-antigen Complexes

The multichaperone-antigen complexes that are recovered by the purification methods described herein optionally can be combined with HSP-antigen complexes for administration for therapeutic or prophylactic purposes. Preferably the HSP-antigen complexes, comprise peptides or proteins that display the antigenicity of an antigen in the multichaperone-antigen complexes. The HSP-antigen complexes can be endogenous (made intracellularly) or made in vitro; the HSPs and/or antigens can be recombinant and/or native. In one option, the multichaperone-antigen complexes of the invention are combined with gp96-antigen complexes, HSP70-antigen complexes, HSP90-antigen complexes, HSP110-antigen complexes, BIP-antigen complexes, grp170-antigen complexes or calreticulin-antigen complexes, or with any combination of the foregoing. In a specific option, the multichaperone-antigen complexes are combined with HSP-antigen complexes that comprise mammalian HSPs, preferably human HSPs, isolated from mammalian or human cells. In another option, the multichaperone-antigen complexes comprise mammalian antigens, preferably human antigens. In one option, the multichaperone-antigen complexes are combined with HSP-antigen complexes that comprise non-human mammalian HSPs and human antigens. In another option, the multichaperone-antigen complexes are combined with HSP- antigen complexes that comprise human mammalian HSPs and non-human mammalian antigens. In a preferred option, the multichaperone-antigen complexes are combined with HSP-antigen complexes that comprise human HSPs and human antigens, most preferably endogenously (non-recombinantly) expressed in human cells from which the HSP-antigen complexes are isolated. In another preferred option, the multichaperone-antigen complexes are combined with HSP-antigen complexes that comprise mammalian HSPs and mammalian antigens, most preferably endogenously (non-recombinantly) expressed in mammalian cells from which the complexes are isolated.

The HSP-antigen complexes can be isolated or purified by any method known in the art, including, but not limited to those methods described in the subsections below.

### 5.2.1. Preparation and purification of gp96-antigen complexes

The purification of gp96-antigen complexes has been described previously, see, for example, Zabrecky et al., 2004, Methods, 32: 3-6. A procedure that may be used, presented by way of example but not limitation, is described below. Another procedures that may be used is presented by way of example but not limitation, in Example 3, Section 6.3.

### 5.2.1.1. Materials

1. Suitable tissue source or packed cell pellet, either fresh or stored frozen at -80° C.
2. One liter Waring blender or equivalent.
3. Con A Sepharose 4B (Amersham) or equivalent; 5 mL HiTrap ConA Sepharose column (Amersham).
4. DEAE Sepharose Fast Flow (Amersham) or equivalent; 1 mL HiTrap DEAE FF column (Amersham).
5. Appropriately sized chromatography columns and solvent delivery systems such as peristaltic or syringe pumps.
6. PD-10 desalting columns (Amersham). Suitable large scale diafiltration tangential flow systems such as Pall/Filtron Ultracette or Mini-Ultracette.
7. Serine proteinase inhibitor, AEBSF (PEFA Block, Pentapharm) or equivalent. Note: AEBSF was found to be much more effective than PMSF.
8. Beckman J-20 Centrifuge, Rotor: JLA-16-250 for 250 mL bottles, JLA-10-500 for 500 mL bottles, and JLA-8-1000 for 1 L bottles. Appropriate bottles for each Rotor from Beckman.
9. Sartorius MiniSart 5 and 0.8 µm, 37 mm diameter cellulose acetate syringe filters or equivalent. Sartorius SartoClean ½ to 1 ft² of 3.0-0.8 µm cellulose acetate filtration capsule or equivalent.
10. Salts, buffers, and other reagents: NaCl, MgCl₂, CaCl₂, Hepes, ammonium sulfate, NaOH, acetic acid, isopropyl alcohol, and a-methyl mannopyranoside.

### 5.2.1.2. Chromatography column preparation

Five milliliter Con A and 1 mL DEAE HiTrap columns are prepared on the day of the preparation. The end caps of the Con A cartridge re broken open and equilibrated with 10 column volumes of Con A Buffer (10 mM sodium phosphate, pH 7.2 ± 0.1, and 150 mM NaCl containing 1-2 mM MgCl₂ + 1-2 mM CaCL₂). The DEAE column is washed with 3-5 CV of 30 mM Hepes, pH 7.2 ± 0.1, containing 1000 mM NaCl, then with 10 CV of PBS (10 mM sodium phosphate, pH 7.2 ± 0.1, and 150 mM NaCl).

### 5.2.1.3. Homogenization and clarification:

The volume of Homogenization Buffer (30 mM sodium phosphate, 1.5 mM magnesium chloride, pH 7.2) is determined based on a ratio of 4 mL for each gram of tissue. An additional ∼20% of the buffer is prepared to be used for re-suspension of the 18,000g pellet. Just before use, a protease inhibitor cocktail such as Cocktail III (Calbiochem, CA) is added to the Homogenization Buffer.

Fresh or frozen tissue is placed into a 1L blender along with 4 mL/g of Homogenization Buffer. The tissue was homogenized for three 15-20 s pulses at the highest speed. It is ensured that no large pieces of tissue remain. The mixture is clarified by centrifugation at 18,000g for 20 min. The supernatant is decanted into an appropriate sized container with a stir bar. The supernatant is maintained on ice with stirring. The pellets are resuspended with the remaining 20% volume of Homogenization Buffer and centrifuged again for 10 min. The supernatants are combined.

Next, for the ammonium sulfate precipitation step, an amount of ammonium sulfate equal to 0.3 g/mL of the total volume of clarified homogenate (supernatant resulting from above) is weighed and obtained. The ammonium sulfate is divided into three aliquots. With constant stirring, the first aliquot of ammonium sulfate is added over the course of 30-60 s to the clarified homogenate. This is repeated with the remaining aliquots at 10 min intervals. After the last addition, stirring is continued for 15 min. The resulting mixture is centrifuged at 18,000g for 20 min. The supernatant is decanted into a suitably sized flask and dilute with 0.3 volumes of Con A Buffer. Filtration is then carried out using 5 µm syringe filters.

### 5.2.1.4. Con A Affinity Chromatography:

The filtrate is applied to an appropriately equilibrated 5 mL Con A HiTrap cartridge or a larger prepacked Con A column. Once the entire volume is loaded, the column is washed with 5-10 column volumes (CVs) of Con A Buffer. The column is eluted with 3 CVs of Con A Elution Buffer (Con A Buffer plus 10% a-methyl mannopyranoside) at 1 mL/min for HiTrap cartridges. The eluate is collected immediately. After 1-1.5 CVs is collected, the flow is stopped and incubated for 3-10 min. The flow is resumed and the final Con A elution pool is about 3-3.5 times the column volume.

The Con A eluate is buffer exchanged using PD-10 columns or a diafiltration system. The PD-10 columns are equilibrated with 30 mL of PBS. 2.5 mL of Con A eluate is applied, the flow through is discarded, and the eluate is collected with 3.5 mL of PBS. Alternatively, 2.0 mL of the Con A elute is applied to the PD-10 columns and the flow through is discarded. A 0.5 mL chase with PBS is then applied and the flow through is discarded. The eluate is then collected by application of 2.8 mL PBS.

### 5.2.1.5. DEAE chromatography:

Buffer exchanged Con A eluate is applied to an equilibrated 1 mL DEAE HiTrap cartridge or an appropriately sized pre-packed DEAE column. The flow is 1 mL/min for the cartridge. The column is washed with 5-10 CVs of DEAE Wash Buffer (10 mM sodium phosphate, pH 7.2 ± 0.1, and 260 mM NaCl). The DEAE column is eluted with about 5-6 CVs of DEAE Elution Buffer (10 mM sodium phosphate, pH 7.2 ± 0.1, and 700 mM NaCl). Fractions are collected of about 1/4 CVs. The fractions are pooled based on Abs₂₈₀ₙₘ, colorimetric protein assay, SDS-PAGE or another suitable method.

Elution is performed according to the following scheme: flow of elution buffer was begun, a 0.5 mL fraction (F1) is collected, a 2 mL fraction (F2) is collected, and then a 1 mL fraction (F3) is collected. The product is contained in F2

### 5.2.1.6. Recovery Step

The final pooled product is buffer exchanged into 5 mM potassium phosphate, 9% sucrose, pH 7.2. The product is filter sterilized by passage through a 0.2 µm filter and stored frozen at -80° C.

### 5.2.2. Preparation and Purification of HSP70-antigen Complexes

The purification of HSP70-antigen complexes has been described previously, see, for example, Udono et al., 1993, J. Exp. Med. 178:1391-1396. A procedure that may be used, presented by way of example but not limitation, is described below.

Initially, tumor cells or tumor tissue are suspended in 4 volumes or 4X the tissue weight of homogenization buffer consisting of 30 mM sodium phosphate, 1.5 mM magnesium chloride, protease inhibitor cocktail such as Cocktail III (Calbiochem, CA), pH 7.2. Tumor cells or tissue are homogenized in a blender.

The resulting homogenate is recentrifuged at 100,000g for 90 minutes, the supernatant harvested and then mixed with Con A Sepharose equilibrated with phosphate buffered saline (PBS) containing 2 mM Ca2+ and 2 mM Mg2+. The supernatant is then allowed to bind to the Con A Sepharose for 2-3 hours at 4°C. The material that fails to bind is harvested and dialyzed for 36 hours (three times, 100 volumes each time) against 10 mM Tris-Acetate pH 7.5, 0.1 mM EDTA, 10 mM NaCl, 1mM PMSF. Then the dialyzate is centrifuged at 17,000 rpm (Sorvall SS34 rotor) for 20 minutes. Then the resulting supernatant is harvested and applied to a Mono Q FPLC column equilibrated in 20 mM Tris-Acetate pH 7.5, 20 mM NaCl, 0.1 mM EDTA and 15 mM 2-mercaptoethanol. The column is then developed with a 20 mM to 500 mM NaCl gradient and then eluted fractions fractionated by sodium dodecyl sulfate-polyacrylamide gel electrophoresis (SDS-PAGE) and characterized by immunoblotting using an appropriate anti-HSP70 antibody (such as from clone N27F3-4, from StressGen).

Fractions strongly immunoreactive with the anti-HSP70 antibody are pooled and the HSP70-antigen complexes precipitated with ammonium sulfate; specifically with a 50%-70% ammonium sulfate cut. The resulting precipitate is then harvested by centrifugation at 17,000 rpm (SS34 Sorvall rotor) and washed with 70% ammonium sulfate. The washed precipitate is then solubilized and any residual ammonium sulfate removed by gel filtration on a SephadexR G25 column (Pharmacia). If necessary the HSP70 preparation thus obtained can be repurified through the Mono Q FPLC Column as described above.

The HSP70-antigen complex can be purified to apparent homogeneity using this method. Typically 1 mg of HSP70-antigen complex can be purified from 1g of cells/tissue.

Alternatively, chromatography with nonhydrolyzable analogs of ADP, instead of ATP, can be used for purification of HSP70-antigen complexes. See Peng et al, Journal of Immunological Methods 204: 13-21 (1997). By way of example but not limitation, purification of HSP70-antigen complexes by ADP-agarose chromatography can be carried out as follows: Meth A sarcoma cells (500 million cells) are homogenized in hypotonic buffer and the lysate is centrifuged at 100,000 g for 90 minutes at 4°C. The supernatant is applied to an ADP-agarose column. The column is washed in buffer and is eluted with 5 column volumes of 3 mM ADP. The HSP70 elutes in fractions 2 through 10 of the total 15 fractions which elute. The eluted fractions are analyzed by SDS-PAGE. The HSP70 can be purified to apparent homogeneity using this procedure.

Alternatively, Hsp70 or HSP70-antigen can be purified by using immunoaffinity purification methods known in the art. For example, a Hsp70-specific scFv column can be used. (See Arnold-Schild et al., Cancer Research, 2000, 60(15):4175-4178. Although Arnold-Schild describes a gp96-specific scFv column, a Hsp70-specific scFv column can be produced by the equivalent method). By way of example but not limitation, the purification using Hsp70-specific scFv column can be carried out as follows: scFv anti-Hsp70 are coupled to CNBr-activated Sepharose. The samples containing Hsp70 or HSP70-antigen complex are applied to the scFv anti-Hsp70 column. After extensive washing with PBS, Hsp70 or HSP70-antigen can be eluted with PBS, 1.3 M NaCl, or 10 mM sodium phosphate (pH 7.2).

Separation of the protein from an HSP70-antigen complex can be performed in the presence of ATP or low pH. These two methods may be used to elute the protein from an HSP70-antigen complex. The first approach involves incubating an HSP70-antigen complex preparation in the presence of ATP. The other approach involves incubating an HSP70-antigen complex preparation in a low pH buffer (e.g., pH is 1, 2, 3, 4, 5, or 6 or less than 6, less than 5, or less than 4, or 4 to 6) or high concentrations of sodium chloride (e.g., greater that 1 M). These methods and any others known in the art may be applied to separate the HSP and protein from an HSP-protein complex.

### 5.2.3. Preparation and Purification of HSP90-antigen Complexes

A procedure that can be used, presented by way of example and not limitation, is as follows:

Initially, tumor cells or tumor tissue are suspended in 4 volumes or 4X the tissue weight of homogenization buffer consisting of 30 mM sodium phosphate, 1.5 mM magnesium chloride, protease inhibitor cocktail such as Cocktail III (Calbiochem, CA), pH 7.2. Tumor cells or tissue are homogenized in a blender.

The resulting homogenate is recentrifuged at 100,000g for 90 minutes, the supernatant harvested and then mixed with Con A Sepharose™ equilibrated with PBS containing 2 mM Ca²⁺ and 2 mM Mg²⁺. When the cells are lysed by mechanical shearing the supernatant is diluted with an equal volume of 2X Lysis buffer prior to mixing with Con A Sepharose™. The supernatant is then allowed to bind to the Con A Sepharose™ for 2-3 hours at 4°C. The material that fails to bind is harvested and dialyzed for 36 hours (three times, 100 volumes each time) against 20 mM Sodium Phosphate (pH 7.4), 1mM EDTA, 250 mM NaCl, 1mM PMSF. Then the dialyzate is centrifuged at 17,000 rpm (Sorvall SS34 rotor) for 20 minutes. Then the resulting supernatant is harvested and applied to a Mono Q FPLC™ ion exchange chromatographic column (Pharmacia) equilibrated with dialysis buffer. The proteins are then eluted with a salt gradient of 200 mM to 600 mM NaCl.

The eluted fractions are fractionated by SDS-PAGE and fractions containing the HSP90-antigen complexes identified by immunoblotting using an anti-HSP90 antibody such as 3G3 (Affinity Bioreagents). HSP90-antigen complexes can be purified to apparent homogeneity using this procedure. Typically, 150-200 µg of HSP90-antigen complex can be purified from 1g of cells/tissue.

Alternatively, HSP90 or HSP90-antigen can be purified by using any immunoaffinity purification methods known in the art. For example, HSP90-specific scFv column can be used. (See Arnold-Schild, incorporates herein by its entirety. Although Arnold-Schild describes a gp96-specific scFv column, a HSP90-specific scFv column can be produced by the equivalent method). By way of example but not limitation, the purification using HSP90-specific scFv column can be carried out as follows: scFv anti-HSP90 are coupled to CNBr-activated Sepharose. The samples containing HSP90 or HSP90-antigen complex are applied to the scFv anti-Hsp70 column. After extensive washing with PBS, HSP90 or HSP90-antigen can be eluted with PBS, 1.3 M NaCl, or 10 mM sodium phosphate (pH 7.2).

Separation of the protein from an HSP90-antigen complex can be performed in the presence of ATP or low pH. These two methods may be used to elute the protein from an HSP90-antigen complex. The first approach involves incubating an HSP90-antigen complex preparation in the presence of ATP. The other approach involves incubating an HSP90-antigen complex preparation in a low pH buffer (*e.g.,* pH is 1, 2, 3, 4, 5, or 6, or less than 6, less than 5, or less than 4, or 4 to 6) or high concentrations of sodium chloride e.g., greater that 1M). These methods and any others known in the art may be applied to separate the HSP and protein from an HSP-protein complex.

### 5.2.4. Preparation and Purification of HSP110-antigen Complexes

A procedure, described by Wang et al., 2001, J. Immunol. 166(1):490-7, with modifications, that can be used, presented by way of example and not limitation, is as follows:

Initially, tumor cells or tumor tissue are suspended in 4 volumes or 4X the tissue weight of homogenization buffer consisting of 30 mM sodium phosphate, 1.5 mM magnesium chloride, protease inhibitor cocktail such as Cocktail III (Calbiochem, CA), pH 7.2 Tumor cells or tissue are homogenized in a blender. The homogenate is centrifuged at 4,500 x g and then 100,000 x g for 2 hours. If the cells or tissues are of hepatic origin, the resulting supernatant is was first applied to a blue Sepharose column (Pharmacia) to remove albumin. Otherwise, the resulting supernatant is applied to a Con A-Sepharose column (Pharmacia Biotech, Piscataway, NJ) previously equilibrated with binding buffer (20 mM Tris-HCI, pH 7.5; 100 mM NaCl; 1mM MgCl₂; 1 mM CaCl₂; 1 mM MnCl₂; and 15 mM 2-ME). The bound proteins are eluted with binding buffer containing 15% methyl-α-d-mannopyranoside (Sigma, St. Louis, MO).

Con A-Sepharose unbound material is first dialyzed against a solution of 20 mM Tris-HCl, pH 7.5; 100 mM NaCl; and 15 mM 2-ME, and then applied to a DEAE-Sepharose column and eluted by salt gradient from 100 to 500 mM NaCl. Fractions containing HSP110 are collected, dialyzed, and loaded onto a Mono Q (Pharmacia) 10/10 column equilibrated with 20 mM Tris-HCl, pH 7.5; 200 mM NaCl; and 15 mM 2-ME. The bound proteins are eluted with a 200-500 mM NaCl gradient. Fractions are analyzed by SDS-PAGE followed by immunoblotting with an Ab for HSP110, as described by Wang et al, 1999, J. Immunol. 162:3378. Pooled fractions containing HSP110 are concentrated by Centriplus (Amicon, Beverly, MA) and applied to a Superose 12 column (Pharmacia). Proteins are eluted by 40 mM Tris-HC1, pH 8.0; 150 mM NaCl; and 15 mM 2-ME with a flow rate of 0.2 ml/min.

Alternatively, HSP110 or HSP110-antigen can be purified by using any immunoaffmity purification methods known in the art. For example, HSP110-specific scFv column can be used. (See Arnold-Schild et al, Cancer Research, 2000, 60(15):4175-4178. Although Arnold-Schild describes a gp96-specific scFv column, a HSP110-specific scFv column can be produced by the equivalent method). By way of example but not limitation, the purification using HSP110-specific scFv column can be carried out as follows: scFv anti-HSP110 are coupled to CNBr-activated Sepharose. The samples containing HSP110 or HSP110-antigen complex are applied to the scFv anti- HSP110 column. After extensive washing with PBS, HSP110 or HSP110-antigen can be eluted with PBS, 1.3 M NaCl, or 10 mM sodium phosphate (pH 7.2).

### 5.2.5. Preparation and Purification of grpl70-antiqen Complexes

A procedure, described by Wang et al, 2001, J. Immunol. 166(1):490-7, with modifications, that can be used, presented by way of example and not limitation, is as follows:

Initially, tumor cells or tumor tissue are suspended in 4 volumes or 4X the tissue weight of homogenization buffer consisting of 30 mM sodium phosphate, 1.5 mM magnesium chloride, protease inhibitor cocktail such as Cocktail III (Calbiochem, CA), pH 7.2 Tumor cells or tissue are homogenized in a blender. The homogenate is centrifuged at 4,500 x g and then 100,000 x g for 2 hours. If the cells or tissues are of hepatic origin, the resulting supernatant is was first applied to a blue Sepharose column (Pharmacia) to remove albumin. Otherwise, the resulting supernatant is applied to a Con A-Sepharose column (Pharmacia Biotech, Piscataway, NJ) previously equilibrated with binding buffer (20 mM Tris-HCl, pH 7.5; 100 mM NaCl; 1 mM MgCl₂; 1 mM CaCl₂; 1 mM MnCl₂; and 15 mM 2-ME).
The bound proteins are eluted with binding buffer containing 15% methyl-α-d-mannopyranoside (Sigma, St. Louis, MO).

Con A-Sepharose-bound material is first dialyzed against 20 mM Tris-HC1, pH 7.5, and 150 mM NaCl and then applied to a Mono Q column and eluted by a 150 to 400 mM NaCl gradient. Pooled fractions are concentrated and applied on the Superose 12 column (Pharmacia). Fractions containing homogeneous grp170 are collected.

Alternatively, GRP170 or GRP170-antigen can be purified by using any immunoaffmity purification methods known in the art. For example, GRP170-specific scFv column can be used. (See Arnold-Schild et al, Cancer Research, 2000, 60(15):4175-4178. Although Arnold-Schild describes a gp96-specific scFv column, a HSP170-specific scFv column can be produced by the equivalent method). By way of example but not limitation, the purification using HSP170-specific scFv column can be carried out as follows: scFv anti-HSP170 are coupled to CNBr-activated Sepharose. The samples containing HSP170 or HSP170-protein complex are applied to the scFv anti- HSP170 column. After extensive washing with PBS, HSP170 or HSP170-protein can be eluted with PBS, 1.3 M NaCl, or 10 mM sodium phosphate (pH 7.2).

### 5.2.6. Preparation and Purification of Calreticulin-antigen Complexes

A procedure, described by Basu et al, 1999, J. Exp. Med. 189(1):797-802, with modifications, that can be used, presented by way of example and not limitation, is as follows:

Initially, tumor cells or tumor tissue are suspended in 4 volumes or 4X the tissue weight of homogenization buffer consisting of 30 mM sodium phosphate, 1.5 mM magnesium chloride, protease inhibitor cocktail such as Cocktail III (Calbiochem, CA), pH 7.2 Tumor cells or tissue are homogenized in a blender. The homogenate is centrifuged at 4,500 x g and then 100,000 x g for 1 hour. If the cells or tissues are of hepatic origin, the resulting supernatant is was first applied to a blue Sepharose column (Pharmacia) to remove albumin. Otherwise, solid ammonium sulfate is added to bring the solution to 50% saturation. This is centrifuged at 14,000 rpm for 30 min. The precipitate is discarded and the supernatant subjected to subsequent fractionation at 80% ammonium sulphate. After centrifugation at 14,000 rpm for 30 min the precipitate is solubilized in PBS containing 2 mM CaCl₂ and 2 mM MgCl₂ This is applied to a Con A-Sepharose column (Pharmacia, NJ).

Con A-Sepharose unbound material is collected and changed to a 25 mM Na citrate buffer, pH 5.3, by PD-10 column (Sephadex G-25; Pharmaciea Biotech). It is then applied to the CM-Sephadex C-50 column. The buffer of unbound material of the CM-Sephadex is then changed to 19 mM Na-phosphate buffer, pH 6.1 by PD-10 columns. It is then applied to the DEAE-sephacel column and eluted by a 150 to 400 mM NaCl gradient. Fractions containing homogeneous calreticulin are collected.

Alternatively, calreticulin-antigen complexes can be purified by using any immunoaffmity purification methods known in the art. For example, a calreticulin specific scFv column can be used. (See Arnold-Schild et ah, Cancer Research, 2000, 60(15):4175-4178; although Arnold-Schild describes a gp96 -specific scFv column, a calreticulin - specific scFv column can be produced by an equivalent method). By way of example but not limitation, a purification using calreticulin-specific scFv column can be carried out as follows: scFv anti-calreticulin are coupled to CNBr- activated Sepharose. The samples containing calreticulin or calreticulin -antigen complex are applied to the scFv anti-calreticulin column. After extensive washing with PBS, calreticulin or calreticulin-antigen can be eluted with PBS, 1.3 M NaCl, or 10 mM sodium phosphate (pH 7.2).

### 5.2.7. Recombinant Expression of Heat Shock Proteins

An HSP can be recombinantly expressed in cells expressing an antigen of interest, from which cells HSP-antigen complexes can then be isolated for use. In one option, an HSP can be recombinantly expressed, and then the HSP can be purified from the cells and used in *in vitro* complexing methods make complexes of antigens or interest *in vitro,* as described in Section 5.2.8. In certain options, HSPs can be prepared from cells that express higher levels of HSPs through recombinant means. Amino acid sequences and nucleotide sequences of many HSPs are generally available in sequence databases, such as GenBank. Computer programs, such as Entrez, can be used to browse the database, and retrieve any amino acid sequence and nucleotide sequence data of interest by accession number. These databases can also be searched to identify sequences with various degrees of similarities to a query sequence using programs, such as FASTA and BLAST, which rank the similar sequences by alignment scores and statistics. Such nucleotide sequences of non-limiting examples of HSPs that can be used for the compositions, methods, and for preparation of the HSP-antigen complexes of the invention are as follows: human HSP70, Genbank Accession No. M24743, Hunt et al., 1995, Proc. Natl. Acad. Sci. U.S.A., 82: 6455-6489; human HSP90, Genbank Accession No. X15183, Yamazaki et al, Nucl. Acids Res. 17: 7108; human gp96: Genbank Accession No. X15187, Maki et al, 1990, Proc. Natl. Acad. Sci. U.S.A. 87: 5658-5562; human BiP: Genbank Accession No. M19645; Ting et al, 1988, DNA 7: 275-286; human HSP27, Genbank Accession No. M24743; Hickey et al, 1986, Nucleic Acids Res. 14: 4127-45; mouse HSP70: Genbank Accession No. M35021, Hunt et al, 1990, Gene 87: 199-204; mouse gp96: Genbank Accession No. M16370, Srivastava et al, 1987, Proc. Natl. Acad. Sci. U.S.A. 85: 3807-3811; and mouse BiP: Genbank Accession No. U16277, Haas et al, 1988, Proc. Natl. Acad. Sci. U.S.A. 85: 2250-2254. Degenerate sequences encoding HSPs can also be used.

Once the nucleotide sequence encoding the HSP of choice has been identified, the nucleotide sequence, or a fragment thereof, can be obtained and cloned into an expression vector for recombinant expression. The expression vector can then be introduced into a host cell for propagation of the HSP. Methods for recombinant production of HSPs are described in detail herein.

The DNA may be obtained by DNA amplification or molecular cloning directly from a tissue, cell culture, or cloned DNA (e.g., a DNA "library") using standard molecular biology techniques (see *e.g.,* Methods in Enzymology, 1987, volume 154, Academic Press; Sambrook et al 1989, Molecular Cloning - A Laboratory Manual, 2nd Edition, Cold Spring Harbor Press, New York; and Current Protocols in Molecular Biology, Ausubel et al. (eds.), Greene Publishing Associates and Wiley Interscience, New York). Clones derived from genomic DNA may contain regulatory and intron DNA regions in addition to coding regions; clones derived from cDNA will contain only exon sequences. Whatever the source, the HSP gene should be cloned into a suitable vector for propagation of the gene.

In a preferred option, DNA can be amplified from genomic or cDNA by polymerase chain reaction (PCR) amplification using primers designed from the known sequence of a related or homologous HSP. PCR is used to amplify the desired sequence in DNA clone or a genomic or cDNA library, prior to selection. PCR can be carried out, *e.g.,* by use of a thermal cycler and Taq polymerase (Gene Amp®). The polymerase chain reaction (PCR) is commonly used for obtaining genes or gene fragments of interest. For example, a nucleotide sequence encoding an HSP of any desired length can be generated using PCR primers that flank the nucleotide sequence encoding open reading frame. Alternatively, an HSP gene sequence can be cleaved at appropriate sites with restriction endonuclease(s) if such sites are available, releasing a fragment of DNA encoding the HSP gene. If convenient restriction sites are not available, they may be created in the appropriate positions by site-directed mutagenesis and/or DNA amplification methods known in the art (see, for example, Shankarappa et al, 1992, PCR Method Appl. 1: 277-278). The DNA fragment that encodes the HSP is then isolated, and ligated into an appropriate expression vector, care being taken to ensure that the proper translation reading frame is maintained.

In an alternative option, for the molecular cloning of an HSP from genomic DNA, DNA fragments are generated to form a genomic library. Since some of the sequences encoding related HSPs are available and can be purified and labeled, the cloned DNA fragments in the genomic DNA library may be screened by nucleic acid hybridization to a labeled probe (Benton and Davis, 1977, Science 196: 180; Grunstein and Hogness, 1975, Proc. Natl. Acad. Sci. U.S.A. 72: 3961). Those DNA fragments with substantial homology to the probe will hybridize. It is also possible to identify an appropriate fragment by restriction enzyme digestion(s) and comparison of fragment sizes with those expected according to a known restriction map.

Alternatives to isolating the HSP genomic DNA include, but are not limited to, chemically synthesizing the gene sequence itself from a known sequence or synthesizing a cDNA to the mRNA which encodes the HSP. For example, RNA for cDNA cloning of the HSP gene can be isolated from cells which express the HSP. A cDNA library may be generated by methods known in the art and screened by methods, such as those disclosed for screening a genomic DNA library. If an antibody to the HSP is available, the HSP may be identified by binding of a labeled antibody to the HSP-synthesizing clones.

Other options for the cloning of a nucleotide sequence encoding an HSP, are presented as examples but not by way of limitation, as follows: In a specific option, nucleotide sequences encoding an HSP can be identified and obtained by hybridization with a probe comprising a nucleotide sequence encoding HSP under various conditions of stringency which are well known in the art (including those employed for cross-species hybridizations).

In certain options, a nucleic acid encoding a secretory form of a non- secreted HSP is used to practice the methods of the present invention. Such a nucleic acid can be constructed by deleting the coding sequence for the ER retention signal, KDEL. Optionally, the KDEL coding sequence is replaced with a molecular tag to facilitate the recognition and purification of the HSP, such as the Fc portion of murine IgG1. In another option, a molecular tag can be added to naturally secreted HSPs. PCT publication no. WO 99/42121 demonstrates that deletion of the ER retention signal of gp96 resulted in the secretion of gp96-Ig peptide-complexes from transfected tumor cells, and the fusion of the KDEL-deleted gp96 with murine IgG1 facilitated its detection by ELISA and FACS analysis and its purification by affinity chromatography with the aid of Protein A.

### 5.3. Compositions

Also disclosed herein are compositions comprising the multichaperone- antigen complexes obtained by the methods disclosed herein.

In a specific option, a composition as disclosed herein comprises (a) human multichaperone-antigen complexes and (b) mammalian HOP affinity molecules, with the proviso that the HOP affinity molecules comprise a HOP affinity fragment or variant thereof that is not present as a fusion protein fused to a protein sequence that is not a HOP affinity fragment or a variant thereof, and wherein the HOP affinity molecules do not comprise a wild-type HOP protein. In a specific embodiment, the HOP affinity molecules are present in trace amounts (*e.g.,* the HOP affinity molecules comprise less than 5%, less than 4%, less than 3%, less than 2%, or less than 1% of the total protein present in the sample containing the multichaperone-antigen complexes).

In one option, the composition comprises multichaperone-antigen complexes that comprise a combination of at least two different heat shock proteins selected from the group consisting of HSP40, HSP70, HSP90, HSP110, HIP, BIP, and calreticulin. In a specific option the HOP affinity molecules in the composition comprise a HOP affinity fragment or variant thereof selected from the group consisting of HOP TPR1 (SEQ ID NO: 1) or a variant thereof, HOP TPR2a (SEQ ID NO: 2) or a variant thereof, HOP TPR1/2a (SEQ ID NO: 3) or a variant thereof, and a combination of any one or more of the foregoing. In another option, the HOP affinity molecules in the composition comprise a human HOP affinity fragment or variant thereof. In another option, the HOP affinity molecules in the composition comprise are present as concatamers of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof. In another option, the HOP affinity molecules in the composition comprise are present as fusion proteins of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof.

A composition as disclosed herein may comprise isolated human multichaperone-antigen complexes, wherein the human multichaperone-antigen complexes comprise the following heat shock proteins: HSP70, HSP90, and HSP110, with the proviso that gp96 is not present.

In one option, a composition as disclosed herein may comprise isolated human multichaperone-antigen complexes, wherein the human multichaperone-antigen complexes comprise the following heat shock proteins: HSP70, HSP90, gp96 and HSP110, with the proviso that HSP60 is not present.

The compositions as disclosed herein may further comprise HSP-antigen complexes that are not part of the multichaperone-antigen complexes of the invention. In a specific option, a composition comprises the multichaperone-antigen complexes mixed with HSP-antigen complexes. Preferably, the HSP-antigen complexes are not present in a noncovalent or covalent complex with the multichaperone-antigen complexes.

The compositions disclosed herein may be purified, such that the HSPs that are present in the preparation containing the multichaperone-antigen complexes account for the majority of protein band intensity on an SDS-PAGE gel.

Also disclosed herein is a composition comprising mammalian HOP affinity molecules covalently bound to a solid phase. In one option, the HOP affinity molecules in the composition comprise a HOP affinity fragment or variant thereof selected from the group consisting of HOP TPR1 or a variant thereof, HOP TPR2a or a variant thereof, HOP TPR1/2a or a variant thereof, and a combination of any one or more of the foregoing. In a specific option, the HOP affinity molecules comprise a HOP affinity fragment or variant thereof that is present as a concatamer of two or more of HOP TPR1 or a variant thereof, HOP TPR2a or a variant thereof, and/or HOP TPRI/2a or a variant thereof. In another option, the HOP affinity molecules comprise a HOP affinity fragment or variant thereof that is present as a fusion protein of two or more of HOP TPR1 or a variant thereof, HOP TPR2a or a variant thereof, and/or HOP TPR1/2a or a variant thereof. In a preferred option, the HOP affinity molecules comprise a human HOP affinity fragment or variant thereof. In a specific option, the solid phase in the composition comprises beads. The beads can be packed in a column or not packed in a column. The beads can also be magnetic. In another option, the solid phase is a membrane. In a specific option, the solid phase has a surface comprising polycarbonate, polystyrene, polypropylene, polyethylene, glass, nitrocellulose, dextran, nylon, polyacrylamide or agarose. In another option, HOP affinity molecules are attached via a bifunctional crosslinker to the solid phase.

The HOP affinity molecules in the composition may be noncovalently bound to mammalian multichaperone-antigen complexes. The multichaperone-antigen complexes can be a combination of at least two different heat shock proteins selected from the group consisting of HSP40, HSP70, HSP90, HSP110, HIP, BIP, and calreticulin. In a preferred option, the heat shock proteins are human heat shock proteins.

The solid phase in the composition may be in contact with a cell extract. The cell extract can be a mammalian cell extract, and is preferably a human cell extract. The cell extract can also be a tumor cell extract and/or an infected cell extract, and can further be an extract of an engineered cell that recombinantly expresses an antigen of interest (e.g., a tumor-associated antigen or a tumor-specific antigen or of an infectious agent).

### 5.3.1. Pharmaceutical Compositions and Formulations

Disclosed herein are pharmaceutical compositions comprising the multichaperone-antigen complexes obtained by the methods disclosed herein. In a preferred option, the pharmaceutical compositions comprise a pharmaceutically acceptable carrier or excipient.

A pharmaceutical composition as disclosed herein may comprise (a) human multichaperone-antigen complexes and (b) mammalian HOP affinity molecules, with the proviso that the HOP affinity molecules comprise a HOP affinity fragment or variant thereof that is not present as a fusion protein fused to a protein sequence that is not a HOP affinity fragment or a variant thereof, and wherein the HOP affinity molecules do not comprise a wild-type HOP protein. In a specific option, the HOP affinity molecules are present in trace amounts (e.g., the HOP affinity molecules comprise less than 5%, less than 4%, less than 3%, less than 2%, or less than 1% of the total protein present in the sample containing the multichaperone-antigen complexes).

The pharmaceutical composition may comprise multichaperone-antigen complexes that comprise a combination of at least two different heat shock proteins selected from the group consisting of HSP40, HSP70, HSP90, HSP110, HIP, BIP, and calreticulin. In one option the HOP affinity molecules in the pharmaceutical composition comprise a HOP affinity fragment or variant thereof selected from the group consisting of HOP TPR1 (SEQ ID NO: 1) or a variant thereof, HOP TPR2a (SEQ ID NO: 2) or a variant thereof, HOP TPR1/2a (SEQ ID NO: 3) or a variant thereof, and a combination of any one or more of the foregoing. In another option, the HOP affinity molecules in the pharmaceutical composition comprise a human HOP affinity fragment or variant thereof. In another option, the HOP affinity molecules in the pharmaceutical composition comprise are present as concatamers of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof. In another specific option, the HOP affinity molecules in the pharmaceutical composition comprise are present as fusion proteins of two or more of HOP TPR1 (SEQ ID NO: 1) or a variant thereof, HOP TPR2a (SEQ ID NO: 2) or a variant thereof, and/or HOP TPR1/2a (SEQ ID NO: 3) or a variant thereof.

A pharmaceutical composition as disclosed herein may comprise isolated human multichaperone-antigen complexes, wherein the human multichaperone-antigen complexes comprise the following heat shock proteins: HSP70, HSP90, and HSP110, with the proviso that gp96 is not present.

In a specific option, a pharmaceutical composition of the invention comprises isolated human multichaperone-antigen complexes, wherein the human multichaperone-antigen complexes comprise the following heat shock proteins: HSP70, HSP90, gp96 and HSP110, with the proviso that HSP60 is not present.

In one option, the pharmaceutical compositions of the invention further comprise HSP-antigen complexes that are not part of the multichaperone-antigen complexes disclosed herein. In a specific option, a pharmaceutical composition comprises the multichaperone-antigen complexes mixed with HSP-antigen complexes. Preferably, the HSP-antigen complexes are not present in a noncovalent or covalent complex with the multichaperone-antigen complexes.

In a specific option, the pharmaceutical compositions of the invention are purified, such that the HSPs that are present in the preparation containing the multichaperone-antigen complexes account for the majority of protein band intensity on an SDS-PAGE gel.

In one option, the pharmaceutical compositions of the invention further comprise a pharmaceutically acceptable carrier.

The pharmaceutical compositions disclosed herein can be administered to a patient at therapeutically effective doses to treat or ameliorate a cell proliferative disorder or infectious disease. A therapeutically effective dose refers to that amount of the pharmaceutical composition sufficient to result in amelioration of symptoms of such a disorder. The effective dose of the complexes may be different when another treatment modality is being used in combination. The appropriate and recommended dosages, formulation and routes of administration for treatment modalities such as chemotherapeutic agents, radiation therapy and biological/immunotherapeutic agents such as cytokines are known in the art and described in such literature as the Physician 's Desk Reference (56th ed., 2002).

In a specific option, the pharmaceutical compositions disclosed herein may comprise a therapeutically effective amount of multichaperone-antigen complexes to treat cancer, wherein said multichaperone-antigen complexes comprise an epitope of a tumor- specific antigen or a tumor-associated antigen.

In another option, the pharmaceutical compositions disclosed herein may comprise a therapeutically effective amount of multichaperone-antigen complexes to treat an infectious disease, wherein said multichaperone-antigen complexes comprise an epitope that displays the antigenicity of an agent that causes said infectious disease.

Pharmaceutical compositions for use as disclosed herein may be formulated in conventional manner using one or more physiologically acceptable carriers or excipients.

Thus, the complexes and their physiologically acceptable salts and solvates may be formulated for administration by inhalation or insufflation (either through the mouth or the nose) oral, buccal, parenteral, rectal, or transdermal administration. Non-invasive methods of administration are also contemplated.

For oral administration, the pharmaceutical compositions may take the form of, for example, tablets or capsules prepared by conventional means with pharmaceutically acceptable excipients such as binding agents (*e.g.,* pregelatinised maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.,* sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p-hydroxybenzoates or sorbic acid). The preparations may also contain buffer salts, flavoring, coloring and sweetening agents as appropriate.

Preparations for oral administration may be suitably formulated to give controlled release of the active complexes.

For buccal administration the compositions may take the form of tablets or lozenges formulated in conventional manner.

For administration by inhalation, the complexes for use according to the present invention are conveniently delivered in the form of an aerosol spray presentation from pressurized packs or a nebuliser, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.,* gelatin for use in an inhaler or insufflator may be formulated containing a powder mix of the complexes and a suitable powder base such as lactose or starch.

The complexes may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The complexes may also be formulated in rectal compositions such as suppositories or retention enemas, e.g., containing conventional suppository bases such as cocoa butter or other glycerides.

In addition to the formulations described previously, the complexes may also be formulated as a depot preparation. Such long acting formulations may be administered by implantation (for example subcutaneously or intramuscularly) or by intramuscular injection. Thus, for example, the complexes may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

Also encompassed is the use of adjuvants in combination with or in admixture with the complexes of the invention. Adjuvants contemplated include but are not limited to mineral salt adjuvants or mineral salt gel adjuvants, particulate adjuvants, microparticulate adjuvants, mucosal adjuvants, and immunostimulatory adjuvants. Adjuvants can be administered to a subject as a mixture with complexes of the invention, or used in combination with the multichaperone-antigen complexes of the invention.

In a specific option, the multichaperone-antigen complexes are not used in combination with an adjuvant.

Also contemplated is the use of adenosine diphosphate (ADP) in combination with or in admixture with the pharmaceutical compositions of the invention.

### 5.3.2. Effective Dose

In another option, an amount of pharmaceutical composition is administered that is in the range of about 0.1 microgram to about 600 micrograms, and preferably about 1 micrograms to about 60 micrograms for a human patient. The amount of pharmaceutical composition administered is 0.1, 0.2, 0.5, 1, 2, 5, 10, 20, 30, 40, 50, 60, 70, 80, 90, 100, 200, 250, 300, 400, 500 or 600 micrograms. Preferably, the amount is less than 100 micrograms. Most preferably, the amount of pharmaceutical composition administered is 5 micrograms, 25 micrograms, or 50 micrograms. The dosage of pharmaceutical composition in a human patient provided by the present invention is in the range of about 5 to 5,000 micrograms. These doses are preferably administered intradermally or subcutaneously. These doses can be given once or repeatedly, such as daily, every other day, weekly, biweekly, or monthly. Preferably, the pharmaceutical compositions are given once weekly for a period of about 4-6 weeks, and the mode or site of administration is preferably varied with each administration. Thus, by way of example and not limitation, the first injection may be given subcutaneously on the left arm, the second on the right arm, the third on the left belly, the fourth on the right belly, the fifth on the left thigh, the sixth on the right thigh, etc. The same site may be repeated after a gap of one or more injections. Also, split injections may be given. Thus, for example, half the dose may be given in one site and the other half on an other site on the same day. Alternatively, the mode of administration is sequentially varied, e.g., weekly injections are given in sequence intradermally, intramuscularly, subcutaneously, intravenously or intraperitoneally. Preferably, the once weekly dose is given for a period of 4 weeks. After 4-6 weeks, further injections are preferably given at two-week or monthly intervals over a period of time of one or more months, or until supply of complexes is exhausted. Later injections may be given monthly. The pace of later injections may be modified, depending upon the patient's clinical progress and responsiveness to the immunotherapy. In a preferred example, intradermal administrations are given, with each site of administration varied sequentially.

Accordingly, there are herein disclosed methods of preventing and treating cancer or an infectious disease in a subject comprising administering a pharmaceutical composition which stimulates the immunocompetence of the host individual and elicits specific immunity against the preneoplastic and/or neoplastic cells or infected cells.

Combination therapy refers to the use of pharmaceutical compositions of the invention with another therapeutic modality to prevent or treat cancer and infectious diseases. The administration of the pharmaceutical compositions as disclosed herein can augment the effect of anti-cancer agents or anti-infectives, and vice versa. This approach is commonly termed combination therapy, adjunctive therapy or conjunctive therapy (the terms are used interchangeably herein). In a specific option, the additional form of therapeutic modality is a non-HSP modality, i.e., the modality does not comprise HSP as a component. In another option, this additional form of therapeutic modality is a HSP modality, i.e., this modality comprises HSP-antigen complexes as a component. With combination therapy, additive potency or additive therapeutic effect can be observed. Synergistic outcomes where the therapeutic efficacy is greater than additive can also be observed. The use of combination therapy can also provide better therapeutic profiles than the administration of the treatment modality, or the pharmaceutical compositions of the invention alone. The additive or synergistic effect may allow the dosage and/or dosing frequency of either or both modalities be adjusted to reduce or avoid unwanted or adverse effects.

In one option, during combination therapy, the pharmaceutical composition is administered in a sub-optimal amount, e.g., an amount that does not manifest detectable therapeutic benefits when administered in the absence of the therapeutic modality, as determined by methods known in the art. In such methods, the administration of such a sub-optimal amount of multichaperone-antigen complexes to a subject receiving a therapeutic modality results in an overall improvement in effectiveness of treatment.

In a preferred option, a pharmaceutical composition is administered in an amount that does not result in tumor regression or cancer remission or an amount wherein the cancer cells have not been significantly reduced or have increased, when said multichaperone-antigen complexes are administered in the absence of the therapeutic modality. In a preferred option, the sub-optimal amount of a pharmaceutical composition is administered to a subject receiving a treatment modality whereby the overall effectiveness of treatment is improved. Among these subjects being treated with a pharmaceutical composition are those receiving chemotherapy or radiation therapy. A sub-optimal amount can be determined by appropriate animal studies. Such a sub-optimal amount in humans can be determined by extrapolation from experiments in animals.

### 5.3.3. Therapeutic Regimens

For any of the combination therapies described above for treatment or prevention of cancer and infectious diseases, in specific options, the pharmaceutical compositions disclosed herein can be administered prior to, concurrently with, or subsequent to the administration of a non-HSP based therapeutic modality. The non-HSP therapeutic modality can be any one of the modalities described above for treatment or prevention of cancer or infectious disease.

In one option, the pharmaceutical compositions disclosed herein may be administered to a subject at reasonably the same time as the other modality. This method provides that the two administrations are performed within a time frame of less than one minute to about five minutes, or up to about sixty minutes from each other, for example, at the same doctor's visit.

In another option, the pharmaceutical compositions disclosed herein and other therapeutic modality are administered at exactly the same time. In yet another option the pharmaceutical compositions of the invention and the other therapeutic modality are administered in a sequence and within a time interval such that the complexes of the invention and the modality can act together to provide an increased benefit than if they were administered alone. In another option, the pharmaceutical compositions disclosed herein and other therapeutic modality are administered sufficiently close in time so as to provide the desired therapeutic or prophylactic outcome. Each can be administered simultaneously or separately, in any appropriate form and by any suitable route. The pharmaceutical compositions disclosed herein and the other therapeutic modality may be administered by different routes of administration. In an alternate option, each is administered by the same route of administration. The pharmaceutical compositions disclosed herein can be administered at the same or different sites, e.g. arm and leg. When administered simultaneously, the pharmaceutical compositions disclosed herein and the other therapeutic modality may or may not be administered in admixture or at the same site of administration by the same route of administration.

In various options, the pharmaceutical compositions as disclosed herein and the other therapeutic modality are administered less than 1 hour apart, at about 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In other options, the complexes disclosed herein and other therapeutic modality are administered 2 to 4 days apart, 4 to 6 days apart, 1 week a part, 1 to 2 weeks apart, 2 to 4 weeks apart, one moth apart, 1 to 2 months apart, or 2 or more months apart. In preferred options, the pharmaceutical compositions disclosed herein and the modality are administered in a time frame where both are still active. One skilled in the art would be able to determine such a time frame by determining the half life of each administered component.

The pharmaceutical compositions disclosed herein and the other therapeutic modality may be administered within the same patient visit. In a specific preferred option, the pharmaceutical compositions disclosed herein are administered prior to the administration of the modality. In an alternate option, the pharmaceutical compositions disclosed herein are administered subsequent to the administration of the other therapeutic modality.

In certain options, the pharmaceutical compositions disclosed herein and the other therapeutic modality are cyclically administered to a subject. Cycling therapy involves the administration of the complexes disclosed herein for a period of time, followed by the administration of a modality for a period of time and repeating this sequential administration. Cycling therapy can reduce the development of resistance to one or more of the therapies, avoid or reduce the side effects of one of the therapies, and/or improve the efficacy of the treatment. In such options, the alternating administration of complexes disclosed herein followed by the administration of a modality 4 to 6 days later, preferable 2 to 4 days, later, more preferably 1 to 2 days later, wherein such a cycle may be repeated as many times as desired is contemplated. In certain options, the complexes of the invention and the other therapeutic modality are alternately administered in a cycle of less than 3 weeks, once every two weeks, once every 10 days or once every week. In a specific option, complexes disclosed herein may be administered to a subject within a time frame of one hour to twenty four hours after the administration of another therapeutic modality. The time frame can be extended further to a few days or more if a slow- or continuous-release type of modality delivery system is used.

### 5.3.4. Prevention and Treatment of Cancer and Infectious Disease

In accordance with the disclosure, a pharmaceutical composition as disclosed herein, which comprises multichaperone-antigen complexes, may be administered to treat a subject with cancer or an infectious disease. In one option, "treatment" or "treating" refers to an amelioration of cancer or an infectious disease, or at least one discernible symptom thereof. In another option, "treatment" or "treating" refers to an amelioration of at least one measurable physical parameter associated with cancer or an infectious disease, not necessarily discernible by the subject. In yet another option, "treatment" or "treating" refers to inhibiting the progression of a cancer or an infectious disease, either physically, e.g., stabilization of a discernible symptom, physiologically, e.g., stabilization of a physical parameter, or both.

In certain options, the pharmaceutical compositions disclosed herein are administered to a subject as a preventative measure against such cancer or an infectious disease. As used herein, "prevention" or "preventing" refers to a reduction of the risk of acquiring a given cancer or infectious disease. In one mode of the option, the pharmaceutical compositions disclosed herein are administered as a preventative measure to a subject having a genetic predisposition to a cancer. In another mode of the option, the pharmaceutical compositions disclosed herein are administered as a preventive measure to a subject facing exposure to carcinogens including but not limited to chemicals and/or radiation, or to a subject facing exposure to an agent of an infectious disease.

For example, in certain options, administration of a pharmaceutical composition as disclosed herein leads to an inhibition or reduction of the growth of cancerous cells or infectious agents by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth in absence of said pharmaceutical composition.

The pharmaceutical compositions prepared by methods disclosed herein comprise multichaperone-antigen complexes. The pharmaceutical compositions may ultimately cause a regression of the tumor burden in the cancer patients treated. The pharmaceutical compositions prepared by the methods disclosed herein can enhance the immunocompetence of the subject and elicit specific immunity against infectious agents or specific immunity against preneoplastic and neoplastic cells. These pharmaceutical compositions have the capacity to prevent the onset and progression of infectious diseases, and to inhibit the growth and progression of tumor cells.

In various options, the combination therapy comprises the administration of pharmaceutical compositions as disclosed herein to a subject treated with a treatment modality wherein the treatment modality administered alone is not clinically adequate to treat the subject such that the subject needs additional effective therapy, e.g., a subject is unresponsive to a treatment modality without administering the pharmaceutical compositions disclosed herein. Included in such options are methods comprising administering pharmaceutical compositions as disclosed herein to a subject receiving a treatment modality wherein said subject has responded to therapy yet suffers from side effects, relapse, develops resistance, etc. Such a subject might be non-responsive or refractory to treatment with the treatment modality alone, i.e., at least some significant portion of cancer cells or pathogens are not killed or their cell division is not arrested. The options provide that the methods disclosed herein comprising administration of pharmaceutical compositions disclosed herein to a subject refractory to a treatment modality alone can improve the therapeutic effectiveness of the treatment modality when administered as contemplated by the methods disclosed herein. The methods disclosed herein comprising administration of pharmaceutical compositions disclosed herein to a subject refractory to a treatment modality alone can also improve the therapeutic effectiveness of the treatment modality when administered as contemplated by the methods disclosed herein. The determination of the effectiveness of a treatment modality can be assayed *in vivo* or *in vitro* using methods known in the art. Art-accepted meanings of refractory are well known in the context of cancer. In one option, a cancer or infectious disease is refractory or non-responsive where respectively, the number of cancer cells or pathogens has not been significantly reduced, or has increased. Among these subjects being treated are those receiving chemotherapy or radiation therapy.

According to the disclosure, pharmaceutical compositions as disclosed herein can be used in combination with many different types of treatment modalities. Some of such modalities are particularly useful for a specific type of cancer or infectious disease and are discussed in Section 5.4.1 and 5.4.2. Many other modalities have an effect on the functioning of the immune system and are applicable generally to both neoplastic and infectious diseases.

Pharmaceutical compositions as disclosed herein may be used in combination with one or more biological response modifiers to treat cancer or infectious disease. One group of biological response modifiers is the cytokines. In one such option, a cytokine is administered to a subject receiving pharmaceutical compositions as disclosed herein. In another such option, pharmaceutical compositions as disclosed herein are administered to a subject receiving a chemotherapeutic agent in combination with a cytokine. In various options, one or more cytokine(s) can be used and are selected from the group consisting of IL-1α, IL-1β, IL-2, IL-3, IL-4, IL-5, IL-6, IL-7, IL-8, IL-9, IL-10, IL-11, IL-12, IFNα, IFNβ, IFNγ, TNFα, TNFβ, G-CSF, GM-CSF, TGF-β, IL-15, IL-18, GM-CSF, INF-γ, INF-α, SLC, endothelial monocyte activating protein-2 (EMAP2), MIP-3α, MIP-3β, or an MHC gene, such as HLA-B7. Additionally, other exemplary cytokines include other members of the TNF family, including but not limited to TNF-α-related apoptosis-inducing ligand (TRAIL), TNF-α-related activation-induced cytokine (TRANCE), TNF-α-related weak inducer of apoptosis (TWEAK), CD40 ligand (CD40L), lymphotoxin alpha (LT-α), lymphotoxin beta (LT-β), OX40 ligand (OX40L), Fas ligand (FasL), CD27 ligand (CD27L), CD30 ligand (CD30L), 41 BB ligand (41 BBL), APRIL, LIGHT, TL1, TNFSF16, TNFSF17, and AITR-L, or a functional portion thereof. See, e.g., Kwon et al., 1999, Curr. Opin. Immunol. 11:340-345 for a general review of the TNF family. Preferably, the pharmaceutical compositions disclosed herein are administered prior to the treatment modalities.

In another option, pharmaceutical compositions as disclosed herein are used in combination with one or more biological response modifiers which are agonists or antagonists of various ligands, receptors and signal transduction molecules of the immune system. For examples, the biological response modifiers include but are not limited to agonists of Toll-like receptors (TLR-2, TLR-7, TLR-8 and TLR-9); LPS; agonists of 4-1 BB, OX40 ligand, ICOS, and CD40; and antagonists of Fas ligand, PD1, PD-L1, and CTLA-4. These agonists and antagonists can be antibodies, antibody fragments, peptides, peptidomimetic compounds, and polysaccharides.

In yet another option, pharmaceutical compositions as disclosed herein are used in combination with one or more biological response modifiers which are immunostimulatory nucleic acids. Such nucleic acids, many of which are oligonucleotides comprising an unmethylated CpG motif, are mitogenic to vertebrate lymphocytes, and are known to enhance the immune response. See Woolridge, et al., 1997, Blood 89:2994-2998. Such oligonucleotides are described in International Patent Publication Nos. WO 01/22972, WO 01/51083, WO 98/40100 and WO 99/61056, as well as United States Patent Nos. 6,207,646, 6,194,388, 6,218,371, 6,239,116, 6,429,199, and 6,406,705.

In yet another option, pharmaceutical compositions as disclosed herein are used in combination with one or more adjuvants. The adjuvant(s) can be administered separately or present in a pharmaceutical composition in admixture with pharmaceutical compositions as disclosed herein. A systemic adjuvant is an adjuvant that can be delivered parenterally. Systemic adjuvants include adjuvants that creates a depot effect, adjuvants that stimulate the immune system and adjuvants that do both. An adjuvant that creates a depot effect as used herein is an adjuvant that causes the antigen to be slowly released in the body, thus prolonging the exposure of immune cells to the antigen. This class of adjuvants includes but is not limited to alum (e.g., aluminum hydroxide, aluminum phosphate); or emulsion-based formulations including mineral oil, non-mineral oil, water-in-oil or oil-in-water-in oil emulsion, oil-in-water emulsions such as Seppic ISA series of Montanide adjuvants (e.g., Montanide ISA 720, AirLiquide, Paris, France); MF-59 (a squalene-in-water emulsion stabilized with Span 85 and Tween 80; Chiron Corporation, Emeryville, Calif.; and PROVAX (an oil-in-water emulsion containing a stabilizing detergent and a micelle-forming agent; IDEC, Pharmaceuticals Corporation, San Diego, Calif).

Other adjuvants stimulate the immune system, for instance, cause an immune cell to produce and secrete cytokines or IgG. This class of adjuvants includes but is not limited to immunostimulatory nucleic acids, such as CpG oligonucleotides; saponins purified from the bark of the Q. saponaria tree, such as QS21 (Antigenics, MA); derivatives of lipopolysaccharides (LPS) such as monophosphoryl lipid A (MPL; Ribi ImmunoChem Research, Inc., Hamilton, Mont.), muramyl dipeptide (MDP; Ribi) and threonyl-muramyl dipeptide (t-MDP; Ribi).

Other systemic adjuvants are adjuvants that create a depot effect and stimulate the immune system. These compounds are those compounds which have both of the above-identified functions of systemic adjuvants. This class of adjuvants includes but is not limited to ISCOMs (Immunostimulating complexes which contain mixed saponins, lipids and form virus-sized particles with pores that can hold antigen; CSL, Melbourne, Australia); AS01 B (GlaxoSmithKline adjuvant system) which is a liposome based formulation containing MPL and QS21; AS02A (GlaxoSmithKline adjuvant system) which is an oil-in- water-based formulation containing MPL and QS21, and AS15 (GlaxoSmithKline adjuvant system) which is a formulation containing QS21, CpG oligonucleotides and MPL.

The mucosal adjuvants useful according to the disclosure are adjuvants that are capable of inducing a mucosal immune response in a subject when administered to a mucosal surface in conjunction with a pharmaceutical composition as disclosed herein. Mucosal adjuvants include but are not limited to CpG nucleic acids (e.g. International Publication No.WO 99/61056) and Bacterial toxins: e.g., Cholera toxin (CT).

### 5.3.5. Target Cancers

In one option, combination therapy encompasses, in addition to the administration of the pharmaceutical compositions of the invention, the adjunctive uses of one or more modalities that aid in the prevention or treatment of cancer, which modalities include, but is not limited to chemotherapeutic agents, immunotherapeutics, anti-angiogenic agents, cytokines, hormones, antibodies, polynucleotides, radiation and photodynamic therapeutic agents. In other options, combination therapy can be used to prevent the recurrence of cancer, inhibit metastasis, or inhibit the growth and/or spread of cancer or metastasis.

Types of cancers that can be treated or prevented by the methods disclosed herein include, but are not limited to human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms'tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease.

In another option, the patient having a cancer is immunosuppressed by reason of having undergone anti-cancer therapy (e.g., chemotherapy radiation) prior to administration of the pharmaceutical compositions disclosed herein.

There are many reasons why immunotherapy as provided by the present invention is desired for use in cancer patients. First, surgery with anesthesia may lead to immunosuppression. With appropriate immunotherapy in the preoperative period, this immunosuppression may be prevented or reversed. This could lead to fewer infectious complications and to accelerated wound healing. Second, tumor bulk is minimal following surgery and immunotherapy is most likely to be effective in this situation. A third reason is the possibility that tumor cells are shed into the circulation at surgery and effective immunotherapy applied at this time can eliminate these cells.

The preventive and therapeutic methods disclosed herein are directed at enhancing the immunocompetence of the cancer patient either before surgery, at or after surgery, and to induce tumor-specific immunity to cancer cells, with the objective being inhibition of cancer, and with the ultimate clinical objective being total cancer regression and eradication. The methods disclosed herein can also be used in individuals at enhanced risk of a particular type of cancer, e.g., due to familial history or environmental risk factors.

In various options, one or more anti-cancer agents, in addition to the pharmaceutical composition of the invention, is administered to treat a cancer patient. An anti-cancer agent refers to any molecule or compound that assists in the treatment of malignant tumors or cancer.

An anti-cancer agent can be a chemotherapeutic agents which include but are not limited to, the following groups of compounds: cytotoxic antibiotics, antimetabolities, antimitotic agents, alkylating agents, platinum compounds, arsenic compounds, DNA topoisomerase inhibitors, taxanes, nucleoside analogues, plant alkaloids, and toxins; and synthetic derivatives thereof. Table 1 lists exemplary compounds of the groups:

**TABLE 1;:**

| | |
|---|---|
| Alkylating agents | |
| Nitrogen mustards: | Cyclophosphamide |
| | Ifosfamide |
| | Trofosfamide |
| | Chlorambucil |
| Nitrosoureas: | Carmustine (BCNU) |
| | Lomustine (CCNU) |
| Alkylsulphonates: | Busulfan |
| | Treosulfan |
| Triazenes: | Dacarbazine |
| Platinum containing compounds: | Cisplatin |
| | Carboplatin |
| | Aroplatin |
| | Oxaliplatin |
| Plant Alkaloids | |
| Vinca alkaloids: | Vincristine |
| | Vinblastine |
| | Vindesine |
| | Vinorelbine |
| Taxoids: | Paclitaxel |
| | Docetaxol |
| | |
| DNA Topoisomerase Inhibitors | |
| Epipodophyllins: | Etoposide |
| | Teniposide |
| | Topotecan |
| | 9-aminocamptothecin |
| | Camptothecin |
| | Crisnatol |
| mitomycins: | Mitomycin C |
| | |
| Anti-folates: | |
| DHFR inhibitors: | Methotrexate |
| | Trimetrexate |
| IMP dehydrogenase Inhibitors: | Mycophenolic acid |
| | Tiazofurin |
| | Ribavirin |
| | EICAR |
| Ribonuclotide reductase | Hydroxyurea |
| Inhibitors: | |
| | Deferoxamine |
| Pvrimidine analogs: | |
| Uracil analogs: | 5-Fluorouracil |
| | Floxuridine |
| | Doxifluridine |
| | Ratitrexed |
| Cytosine analogs: | Cytarabine (ara C) |
| | Cytosine arabinoside |
| | Fludarabine |
| Purine analogs: | Mercaptopurine |
| | Thioguanine |
| DNA Antimetabolites: | 3-HP |
| | 2'-deoxy-5-fluorouridine |
| | 5-HP |
| | alpha-TGDR |
| | aphidicolin glycinate |
| | ara-C |
| | 5-aza-2'-deoxycytidine |
| | beta-TGDR |
| | cyclocytidine |
| | guanazole |
| | inosine glycodialdehyde |
| | macebecin II |
| | pyrazoloimidazole |
| Antimitotic agents: | allocolchicine |
| | Halichondrin B |
| | colchicine |
| | colchicine derivative |
| | dolstatin 10 |
| | maytansine |
| | rhizoxin |
| | thiocolchicine |
| | trityl cysteine |
| Others: | |
| Isoprenylation inhibitors: | |
| Dopaminergic neurotoxins: | 1-methyl-4-phenylpyridinium ion |
| Cell cycle inhibitors: | Staurosporine |
| Actinomycins: | Actinomycin D |
| | Dactinomycin |
| Bleomycins: | Bleomycin A2 |
| | Bleomycin B2 |
| | Peplomycin |
| Anthracyclines: | Daunorubicin |
| | Doxorubicin (adriamycin) |
| | Idarubicin |
| | Epirubicin |
| | Pirarubicin |
| | Zorubicin |
| | Mitoxantrone |
| MDR inhibitors: | Verapamil |
| Ca²⁺ ATPase inhibitors: | Thapsigargin |

Other targeted anti-cancer therapies include, but are not limited to, sorafenib (Bayer, PA), sunitinib (Pfizer, CT), temsirolimus (Wyeth, PA), imatinib mesylate (Novartis, NJ), and erlotinib (Genentech, CA). Additional anti-cancer therapies are disclosed in the 2009 PhRMA report entitled *Medicines in Development for Cancer,* which can be found at http//www.phrma.org.

Pharmaceutical compositions comprising one or more chemotherapeutic agents (e.g., FLAG, CHOP) are also contemplated by the present disclosure. FLAG comprises fludarabine, cytosine arabinoside (Ara-C) and G-CSF. CHOP comprises cyclophosphamide, vincristine, doxorubicin, and prednisone. Each of the foregoing lists is illustrative, and is not intended to be limiting.

The pharmaceutical compositions disclosed herein can be administered prior to, subsequently, or concurrently with anti-cancer agent(s), for the prevention or treatment of cancer. Depending on the type of cancer, the subject's history and condition, and the anti-cancer agent(s) of choice, the use of the complexes of the invention can be coordinated with the dosage and timing of chemotherapy.

In a preferred option, the invention further encompasses the use of low doses of chemotherapeutic agents when administered as part of the combination therapy regimen. For example, initial treatment with the pharmaceutical compositions disclosed herein increases the sensitivity of a tumor to subsequent challenge with a dose of chemotherapeutic agent, which dose is near or below the lower range of dosages when the chemotherapeutic agent is administered without the pharmaceutical compositions disclosed herein.

In another option, the pharmaceutical compositions disclosed herein are administered in combination with one or more immunotherapeutic agents, such as antibodies and vaccines. In a preferred option, the antibodies have *in vivo* therapeutic and/or prophylactic uses against cancer. In some options, the antibodies can be used for treatment and/or prevention of infectious disease. Examples of therapeutic and prophylactic antibodies include, but are not limited to MDX-1106 (Medarex, NJ) which is an anti-PD1 antibody; MDX-1105 (Medarex, NJ) which is an anti-PD-L1 antibody; BMS-663513 (BMS, NJ) which is an anti-4-1BB antibody; MDX-010 (Medarex, NJ) which is an anti-CTLA-4 antibody; CP-675,206 (Pfizer, CT) which is another anti-CTLA-4 antibody; SYNAGIS® (MedImmune, MD) which is a humanized anti-respiratory syncytial virus (RSV) monoclonal antibody for the treatment of patients with RSV infection; HERCEPTIN® (Trastuzumab) (Genentech, CA) which is a humanized anti-HER2 monoclonal antibody for the treatment of patients with metastatic breast cancer. Other examples are a humanized anti-CD18 F(ab')₂ (Genentech); CDP860 which is a humanized anti-CD18 F(ab')₂ (Celltech, UK); PRO542 which is an anti-HIV gp120 antibody fused with CD4 (Progenics/Genzyme Transgenics); Ostavir which is a human anti Hepatitis B virus antibody (Protein Design Lab/Novartis); PROTOVIR™ which is a humanized anti-CMV IgG1 antibody (Protein Design Lab/Novartis); MAK-195 (SEGARD) which is a murine anti-TNF-α F(ab')₂ (Knoll Pharma/BASF); IC14 which is an anti-CD14 antibody (ICOS Pharm); a humanized anti-VEGF IgG1 antibody (Genentech); OVAREX™ which is a murine anti-CA 125 antibody (Altarex); PANOREX™ which is a murine anti-17-1A cell surface antigen IgG2a antibody (Glaxo Wellcome/Centocor); BEC2 which is a murine antiidiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); Smart ID10 which is a humanized anti-HLA antibody (Protein Design Lab); ONCOLYM™ (Lym-1) is a radiolabeled murine anti-HLA DIAGNOSTIC REAGENT antibody (Techniclone); ABX-IL8 is a human anti-IL8 antibody (Abgenix); anti-CD11a is a humanized IgG1 antibody (Genentech/Xoma); ICM3 is a humanized anti-ICAM3 antibody (ICOS Pharm); IDEC-114 is a primatized anti-CD80 antibody (IDEC Pharm/Mitsubishi); ZEVALIN™ is a radiolabeled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (C5) antibody (Alexion Pharm); D2E7 is a humanized anti-TNF-α antibody (CAT/BASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-α IgG4 antibody (Celltech); LDP-02 is a humanized anti-α4β7 antibody (LeukoSite/Genentech); OrthoClone 0KT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); MDX-33 is a human anti-CD64 (FcγR) antibody (Medarex/Centeon); SCH55700 is a humanized anti-IL-5 IgG4 antibody (Celltech/Schering); SB-240563 and SB-240683 are humanized anti-IL-5 and IL-4 antibodies, respectively, (SmithKline Beecham); rhuMab-E25 is a humanized anti-IgE IgG1 antibody (Genentech/Norvartis/Tanox Biosystems); ABX-CBL is a murine anti CD-147 IgM antibody (Abgenix); BTI-322 is a rat anti-CD2 IgG antibody (Medimmune/Bio Transplant); Orthoclone/OKT3 is a murine anti-CD3 IgG2a antibody (ortho Biotech); SIMULECT™ is a chimeric anti-CD25 IgG1 antibody (Novartis Pharm); LDP-01 is a humanized anti-β₂-integrin IgG antibody (LeukoSite); Anti-LFA-1 is a murine anti CD18 F(ab')₂ (Pasteur-Merieux/Immunotech); CAT-152 is a human anti-TGF-β₂ antibody (Cambridge Ab Tech); and Corsevin M is a chimeric anti-Factor VII antibody (Centocor). In another option, the immunoreactive reagent is a cytotoxic protein such as denileukin diftitox (Eisai, NJ). The above-listed immunoreactive reagents, as well as any other immunoreactive reagents, may be administered according to any regimen known to those of skill in the art, including the regimens recommended by the suppliers of the immunoreactive reagents.

In another option, the pharmaceutical compositions as disclosed herein are administered in combination with one or more anti-angiogenic agents, which includes, but is not limited to, angiostatin, thalidomide and endostatin,

Other peptides that inhibit angiogenesis and correspond to fragments of laminin, fibronectin, procollagen, and EGF have also been described (see, e.g., Cao, 1998, Prog Mol Subcell Biol. 20: 161-176).

In yet another option, the pharmaceutical compositions as disclosed herein are used in association with a hormonal treatment. Hormonal therapeutic treatments comprise hormonal agonists, hormonal antagonists (e.g., flutamide, bicalutamide, tamoxifen, raloxifene, leuprolide acetate (LUPRON), LH-RH antagonists), inhibitors of hormone biosynthesis and processing, and steroids (e.g., dexamethasone, retinoids, deltoids, betamethasone, Cortisol, cortisone, prednisone, dehydrotestosterone, glucocorticoids, mineralocorticoids, estrogen, testosterone, progestins), vitamin A derivatives (e.g., all-trans retinoic acid (ATRA)); vitamin D3 analogs; antigestagens (e.g., mifepristone, onapristone), and antiandrogens (e.g., cyproterone acetate).

In another option, the pharmaceutical compositions as disclosed herein are administered in conjunction with a regimen of radiation therapy. For radiation treatment, the radiation can be gamma rays or X-rays. The methods encompass treatment of cancer comprising radiation therapy, such as external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et ah, eds., J.B. Lippencott Company, Philadelphia. In preferred options, the radiation treatment is administered as external beam radiation or teletherapy wherein the radiation is directed from a remote source. In various preferred options, the radiation treatment is administered as internal therapy or brachytherapy wherein a radioactive source is placed inside the body close to cancer cells or a tumor mass. Also encompassed is the combined use of the pharmaceutical compositions of the invention with photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

### 5.3.6. Target Infectious Diseases

Infectious diseases that can be treated or prevented by the methods disclosed herein are caused by infectious agents including, but not limited to, viruses, bacteria, fungi, protozoa, helminths, and parasites. The disclosure is not limited to treating or preventing infectious diseases caused by intracellular pathogens. Many medically relevant microorganisms have been described extensively in the literature, e.g., see C.G.A Thomas, Medical Microbiology, Bailliere Tindall, Great Britain 1983.

Infectious diseases that can be treated or prevented by the methods of the present invention are caused by infectious agents including, but not limited to, viruses, bacteria, fungi protozoa, helminths, and parasites. The disclosure is not limited to treating or preventing infectious diseases caused by intracellular pathogens. Many medically relevant microorganisms have been described extensively in the literature, e.g., see C.G.A Thomas, Medical Microbiology, Bailliere Tindall, Great Britain 1983.

Combination therapy encompasses in addition to the administration of the pharmaceutical compositions disclosed herein, the uses of one or more modalities that aid in the prevention or treatment of infectious diseases, which modalities include, but is not limited to antibiotics, antivirals, antiprotozoal compounds, antifungal compounds, and antihelminthics. Other treatment modalities that can be used to treat or prevent infectious diseases include immunotherapeutics, polynucleotides, antibodies, cytokines, and hormones as described above.

Infectious virus of both human and non-human vertebrates, include retroviruses, RNA viruses and DNA viruses. Examples of virus that have been found in humans include but are not limited to: Retroviridae (e.g. human immunodeficiency viruses, such as HIV-1 (also referred to as HTLV-III, LAV or HTLV-III/LAV, or HIV-III; and other isolates, such as HIV-LP; Picornaviridae (e.g. polio viruses, hepatitis A virus; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronaviridae (e.g. coronaviruses); Rhabdoviridae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Retroviruses that are contemplated include both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-1, HTLV-II, simian T-cell leukemia virus (STLV), and bovine leukemia virus (BLV). The foamy viruses include human foamy virus (HFV), simian foamy virus (SFV) and bovine foamy virus (BFV).

Examples of RNA viruses that are antigens in vertebrate animals include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picornaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that are antigens in vertebrate animals include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV -40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents.
Many examples of antiviral compounds that can be used in combination with the pharmaceutical compositions disclosed herein are known in the art and include but are not limited to: rifampicin, nucleoside reverse transcriptase inhibitors (e.g., AZT, ddl, ddC, 3TC, d4T), non-nucleoside reverse transcriptase inhibitors (e.g., Efavirenz, Nevirapine, Etravirine), protease inhibitors (e.g., aprenavir, indinavir, ritonavir, and saquinavir), idoxuridine, cidofovir, acyclovir, ganciclovir, zanamivir, amantadine, and Palivizumab. Other examples of anti-viral agents include but are not limited to Amantadine, Delavirdine; Ribavirin; Rimantadine; Valacyclovir; Vidarabine;
Bacterial infections or diseases that can be treated or prevented by the methods disclosed herein are caused by bacteria including, but not limited to, bacteria that have an intracellular stage in its life cycle, such as mycobacteria (e.g., *Mycobacteria tuberculosis, M. bovis, M. avium, M. leprae,* or *M. africanum*), rickettsia, mycoplasma, chlamydia, and legionella. Other examples of bacterial infections contemplated include but are not limited to infections caused by Gram positive bacillus (e.g., *Listeria, Bacillus* such as *Bacillus anthracis, Erysipelothrix* species), Gram negative bacillus (e.g., *Bartonella, Brucella, Campylobacter, Enterobacter, Escherichia, Francisella, Hemophilus, Klebsiella, Morganella, Proteus, Providencia, Pseudomonas, Salmonella, Serratia, Shigella, Vibrio,* and *Yersinia* species), spirochete bacteria (e.g., *Borrelia* species including *Borrelia burgdorferi* that causes Lyme disease), anaerobic bacteria (e.g., *Actinomyces* and *Clostridium* species), Gram positive and negative coccal bacteria, *Enterococcus* species, *Streptococcus* species, *Pneumococcus* species, *Staphylococcus* species, *Neisseria* species. Specific examples of infectious bacteria include but are not limited to: *Helicobacter pyloris, Borelia burgdorferi, Legionella pneumophilia, Mycobacteria tuberculosis, M. avium, M.*
*intracellulare, M. kansaii, M. gordonae, Staphylococcus aureus, Neisseria gonorrhoeae, Neisseria meningitides, Listeria monocytogenes, Streptococcus pyrogenes* (Group A Streptococcus), *Streptococcus agalactiae* (Group B Streptococcus), *Streptococcus viridans, Streptococcus faecalis, Streptococcus bovis, Streptococcus pneumoniae, Haemophilus influenza, Bacillus antracis, corynebacterium diphtheriae, Erysipelothrix rhusiopathiae, Clostridium perfringers, Clostridium tetani, Enterobacter aerogenes, Klebsiella penumoniae, Pasturella multocida, Fusobacterium nucleatum, Streptobacillus moniliformis, Treponema pallidium, Treponema pertenue, Leptospira, Rickettsia,* and *Actinomyces israelli.*
Antibacterial agents or antibiotics that can be used in combination with the pharmaceutical compositions disclosed herein include but are not limited to: aminoglycoside antibiotics (*e.g.,* apramycin, arbekacin, bambermycins, butirosin, dibekacin, neomycin, neomycin, undecylenate, netilmicin, paromomycin, ribostamycin, sisomicin, and spectinomycin), amphenicol antibiotics (*e.g.,* azidamfenicol, chloramphenicol, florfenicol, and thiamphenicol), ansamycin antibiotics (*e.g.,* rifamide and rifampin), carbacephems (*e.g.,* loracarbef), carbapenems (*e.g.,* biapenem and imipenem), cephalosporins (*e.g.,* cefaclor, cefadroxil, cefamadole, cefatrizine, cefazedone, cefozopran, cefpimizole, cefpiramide, and cefpirome), cephamycins (*e.g.,* cefbuperazone, cefmetazole, and cefminox), monobactams (*e.g.,* aztreonam, carumonam, and tigemonam), oxacephems (*e.g.,* flomoxef, and moxalactam), penicillins (*e.g.,* amdinocillin, amdinocillin pivoxil, amoxicillin, bacampicillin, benzylpenicillinic acid, benzylpenicillin sodium, epicillin, fenbenicillin, floxacillin, penamccillin, penethamate hydriodide, penicillin o-benethamine, penicillin 0, penicillin V, penicillin V benzathine, penicillian V hydrabamine, penimepicycline, and phencihicillin potassium), lincosamides (*e.g.,* clindamycin, and lincomycin), macrolides (*e.g.,* azithromycin, carbomycin, clarithromycin, dirithromycin, erythromycin, and erythromycin acistrate), amphomycin, bacitracin, capreomycin, colistin, endurcidin, enviomycin, tetracyclines (*e.g.,* apicycline, chloretetracycline, clomocycline, and demeclocycline), 2,4-diaminopyrimidines (*e.g.,* brodimoprim), nitrofurans (*e.g.,* furaltadone, and furazolium chloride), quinolones and analogs thereof (e.g., cinoxacin, ciprofloxacin, clinafloxacin, flumequine, and grepagloxacin), sulphonamides (*e.g.,* acetyl sulfamethoxypyrazine, benzylsulfamide, noprylsulfamide, phthalylsulfacetamide, sulfachrysoidine, and sulfacytine), sulfones (*e.g.,* diathymosulfone, glucosulfone sodium, and solasulfone), cycloserine, mupirocin and tuberin.
Fungal diseases that can be treated or prevented by the methods disclosed herein include but not limited to aspergilliosis, crytococcsis, sporotrichosis, coccidioidomycosis, paracoccidioidomycosis, histoplasmosis, blastomycosis, zygomycosis, and candidiasis. Antifungal compounds that can be used in combination with the complexes as disclosed herein include but are not limited to: polyenes (*e.g.,* amphotericin b, candicidin, mepartricin, natamycin, and nystatin), allylamines (*e.g.,* butenafine, and naftifine), imidazoles (*e.g.,* bifonazole, butaconazole, chlordantoin, flutrimazole, isoconazole, ketoconazole, and lanoconazole), thiocarbamates (*e.g.,* tolciclate, tolindate, and tolnaftate), triazoles (*e.g.,* fluconazole, itraconazole, saperconazole, and terconazole), bromosalicylchloranilide, buclosamide, calcium propionate, chlorphenesin, ciclopirox, azaserine, Griseofulvin, oligomycins, neomycin undecylenate, pyrrolnitrin, siccanin, tubercidin, and viridin
Parasitic diseases that can be treated or prevented by the methods disclosed herein including, but not limited to, amebiasis, malaria, leishmanial, coccidia, giardiasis, cryptosporidiosis, toxoplasmosis, and typanosomiasis. Also encompassed are infections by various worms, such as but not limited to ascariasis, ancylostomiasis, trichuriasis, stronglyoidiasis, toxocariasis, trichinosis, onchocerciasis, filaria, and dirofilariaisis. Also encompassed are infections by various flukes, such as but not limited to schistosomiasis, paragonimiasis, and clonorchiasis. Parasites that cause these diseases can be classified based on whether they are intracellular or extracellular. An "intracellular parasite" as used herein is a parasite whose entire life cycle is intracellular. Examples of human intracellular parasites include *Leishmania* spp., *Plasmodium* spp., *Trypanosoma cruzi, Toxoplasma gondil, Babesia* spp., and *Trichinella spiralis.* An "extracellular parasite" as used herein is a parasite whose entire life cycle is extracellular. Extracellular parasites capable of infecting humans include *Entamoeba histolytica, Giardia lamblia, Enterocytozoon bieneusi, Naegleria* and *Acanthamoeba* as well as most helminths. Yet another class of parasites is defined as being mainly extracellular but with an obligate intracellular existence at a critical stage in their life cycles. Such parasites are referred to herein as "obligate intracellular parasites". These parasites may exist most of their lives or only a small portion of their lives in an extracellular environment, but they all have at least one obligate intracellular stage in their life cycles. This latter category of parasites includes *Trypanosoma rhodesiense* and
*Trypanosoma gamiense, Isopora* spp., *Cryptosporidium* spp., *Eimeria* spp., *Neospora* spp., *Sarcocystis* spp., and *Schistomsoma* spp.

Many examples of antiprotozoal compounds that can be used in combination with the pharmaceutical compositions of the invention to treat parasitic diseases are known in the art and include but are not limited to: quinines, chloroquine, mefloquine, proguanil, pyrimethamine, metronidazole, diloxanide furoate, tinidazole, amphotericin, sodium stibogluconate, trimoxazole, and pentamidine isetionate. Many examples of antiparasite drugs that can be used in combination with the pharmaceutical compositions of the invention of the invention to treat parasitic diseases are known in the art and include but are not limited to: mebendazole, levamisole, niclosamide, praziquantel, albendazole, ivermectin, diethylcarbamazine, and thiabendazole. Further examples of anti-parasitic compounds include but are not limited to Acedapsone; Amodiaquine Hydrochloride; Amquinate; Arteflene; Chloroquine; Chloroquine Hydrochloride; Chloroquine Phosphate; Cycloguanil Pamoate; Enpiroline Phosphate; Halofantrine Hydrochloride; Hydroxychloroquine Sulfate; Mefloquine Hydrochloride; Menoctone; Mirincamycin Hydrochloride; Primaquine Phosphate; Pyrimethamine; Quinine Sulfate; and Tebuquine.

In a less preferred option, the pharmaceutical compositions of the invention can be used in combination with a non-HSP-based vaccine composition. Examples of such vaccines for humans are described in The Jordan Report 2000, Accelerated Development of Vaccines, National Institute of Health. Many vaccines for the treatment of non-human vertebrates are disclosed in Bennett, K. Compendium of Veterinary Products, 3rd ed. North American Compendiums, Inc., 1995.

### 5.3.7. Autologous Embodiment

The specific immunogenicity of multichaperone-antigen complexes derives not from HSPs that are present in the multichaperone-antigen complexes per se, but from the antigenic proteins and/or peptides bound to them. In a preferred option, the pharmaceutical compositions disclosed herein for use as cancer vaccines are autologous complexes, thereby circumventing two of the most intractable hurdles to cancer immunotherapy. First is the possibility that human cancers, like cancers of experimental animals, are antigenically distinct. To circumvent this hurdle, in a preferred option , the multichaperones are complexed to antigenic proteins and peptides, and the complexes are used to treat the cancers in the same subject from which the proteins or peptides are derived. Second, most current approaches to cancer immunotherapy focus on determining the CTL-recognized epitopes of cancer cell lines. This approach requires the availability of cell lines and CTLs against cancers. These reagents are unavailable for an overwhelming proportion of human cancers. In an option of the present disclosure directed to the use of autologous antigenic proteins and/or peptides, cancer immunotherapy does not depend on the availability of cell lines or CTLs nor does it require definition of the antigenic epitopes of cancer cells. These advantages make complexes of multichaperones bound to autologous antigenic proteins and/or peptides attractive immunogens against cancer. Thus, in a specific, autologous optoin, the multichaperone-antigen complexes are isolated from cancerous tissue of the cancer patient to which the complexes are to be administered for treatment of the cancer.

In other options, therapeutic or prophylactic multichaperone-antigen complexes can be prepared from cancerous tissue of the same type of cancer from a subject allogeneic to the subject to whom the complexes are administered.

### 5.4. Determination of Immunogenicity of Multichaperone-antigen Complexes

Optionally, the mutltichaperone-antigen complexes obtained by the methods disclosed herein can be assayed for immunogenicity using any method known in the art. Such methods can also be used to assay the immunogenicity of HSP-antigen complexes in combination therapy with the multichaperone-antigen complexes. By way of example but not limitation, one of the following procedures can be used.

### 5.4.1. The MLTC Assay

Briefly, mice are injected with an amount of the multichaperone-antigen complexes of the invention, using any convenient route of administration. Cells known to contain specific antigens, e.g. tumor cells or cells infected with an agent of an infectious disease, may act as a positive control for the assay. The mice are injected twice, 7-10 days apart. Ten days after the last immunization, the spleens are removed and the lymphocytes released. The released lymphocytes may be re-stimulated subsequently *in vitro* by the addition of dead cells that expressed the antigen of interest.

For example, 8x10⁶ immune spleen cells may be stimulated with 4x10⁴ mitomycin C treated or γ-irradiated (5-10,000 rads) cells containing the antigen of interest (or cells transfected with an appropriate gene, as the case may be) in 3 ml RPMI medium containing 10% fetal calf serum. In certain cases 33% secondary mixed lymphocyte culture supernatant may be included in the culture medium as a source of T cell growth factors (*See,* Glasebrook, et al., 1980, J. Exp. Med. 151:876). To test the primary cytotoxic T cell response after immunization, spleen cells may be cultured without stimulation. In some experiments spleen cells of the immunized mice may also be re-stimulated with antigenically distinct cells, to determine the specificity of the cytotoxic T cell response.

Six days later the cultures are tested for cytotoxicity in a 4 hour ⁵¹Cr-release assay (*See,* Palladino, et al., 1987, Cancer Res. 47:5074-5079 and Blachere, at al., 1993, J. Immunotherapy 14:352-356). In this assay, the mixed lymphocyte culture is added to a target cell suspension to give different effector:target (E:T) ratios (usually 1:1 to 40:1). The target cells are prelabelled by incubating 1x10⁶ target cells in culture medium containing 20 mCi ⁵¹Cr/ml for one hour at 37°C. The cells are washed three times following labeling. Each assay point (E:T ratio) is performed in triplicate and the appropriate controls incorporated to measure spontaneous ⁵¹Cr release (no lymphocytes added to assay) and 100% release (cells lysed with detergent). After incubating the cell mixtures for 4 hours, the cells are pelletted by centrifugation at 200g for 5 minutes. The amount of ⁵¹Cr released into the supernatant is measured by a gamma counter. The percent cytotoxicity is measured as cpm in the test sample minus spontaneously released cpm divided by the total detergent released cpm minus spontaneously released cpm.

In order to block the MHC class I cascade a concentrated hybridoma supernatant derived from K-44 hybridoma cells (an anti-MHC class I hybridoma) is added to the test samples to a final concentration of 12.5%.

### 5.4.2. CD4⁺ T Cell Proliferation Assay

Primary T cells are obtained from spleen, fresh blood, or CSF and purified by centrifugation using FICOLL-PAQUE PLUS (Pharmacia, Upsalla, Sweden) essentially as described by Kruse and Sebald, 1992, EMBO J. 11: 3237-3244. The peripheral blood mononuclear cells are incubated for 7-10 days with a lysate of cells expressing an antigen. Antigen presenting cells may, optionally be added to the culture 24 to 48 hours prior to the assay, in order to process and present the antigen in the lysate. The cells are then harvested by centrifugation, and washed in RPMI 1640 media (GibcoBRL, Gaithersburg, Md.). 5x10⁴ activated T cells/well (PHA-blasts) are in RPMI 1640 media containing 10% fetal bovine serum, 10 mM HEPES, pH 7.5, 2 mM L-glutamine, 100 units/ml penicillin G, and 100 µg/ml streptomycin sulphate in 96 well plates for 72 hrs at 37°C, pulsed with 1 µCi ³H-thymidine (DuPont NEN, Boston, Mass.)/well for 6 hrs, harvested, and radioactivity measured in a TOPCOUNT scintillation counter (Packard Instrument Co., Meriden, Conn.).

### 5.4.3. Antibody Response Assay

In a certain option, the immunogenicity of a multichaperone-antigen complex of the invention is determined by measuring antibodies produced in response to the administration with the complex. In one mode of the option, microtitre plates (96-well Immuno Plate II, Nunc) are coated with 50 µl/well of a 0.75 µg/ml solution of a purified, non-complexed form of the antigenic peptide used in the multichaperone-antigen complex (e.g. Aβ42) in PBS at 4°C for 16 hours and at 20°C for 1 hour. The wells are emptied and blocked with 200 µl PBS-T-BSA (PBS containing 0.05% (v/v) TWEEN 20 and 1% (w/v) bovine serum albumin) per well at 20°C for 1 hour, then washed 3 times with PBS-T. Fifty µl/well of plasma or CSF from a animal (such as a model mouse or a human patient) that has received the multichaperone-antigen complex of the invention is applied at 20°C for 1 hour, and the plates are washed 3 times with PBS-T. The anti-peptide antibody activity is then measured calorimetrically after incubating at 20°C for 1 hour with 50 µl/well of sheep anti-mouse or anti-human immunoglobulin, as appropriate, conjugated with horseradish peroxidase (Amersham) diluted 1:1,500 in PBS-T-BSA and (after 3 further PBS-T washes as above) with 50 µl of an o-phenylene diamine (OPD)-H2θ2 substrate solution. The reaction is stopped with 150 µl of 2M H₂SO₄ after 5 minutes and absorbance is determined in a Kontron SLT-210 photometer (SLT Lab-instr., Zurich, Switzerland) at 492 nm (ref. 620 nm).

### 5.4.4. Cytokine Detection Assay

The CD4+ T cell proliferative response to multichaperone-antigen complexes disclosed herein may be measured by detection and quantitation of the levels of specific cytokines. In one option, for example, intracellular cytokines may be measured using an IFN-γ detection assay to test for immunogenicity of a complex as disclosed herein. In an example of this method, peripheral blood mononuclear cells from a subject treated with a lectin-HSP-antigen complex are stimulated with peptide antigens of a given tumor or with peptide antigens of an agent of infectious disease. Cells are then stained with T cell-specific labeled antibodies detectable by flow cytometry, for example FITC-conjugated anti-CD8 and PerCP-labeled anti-CD4 antibodies. After washing, cells are fixed, permeabilized, and reacted with dye-labeled antibodies reactive with human IFN-γ (PE-anti-IFN-γ). Samples are analyzed by flow cytometry using standard techniques.

Alternatively, a filter immunoassay, the enzyme-linked immunospot assay (ELISPOT) assay, may be used to detect specific cytokines surrounding a T cell. In one option, for example, a nitrocellulose-backed microtiter plate is coated with a purified cytokine-specific primary antibody, i.e., anti-IFN-γ, and the plate is blocked to avoid background due to nonspecific binding of other proteins. A sample of mononuclear blood cells, containing cytokine-secreting cells, obtained from a subject treated with a lectin-HSP- antigen complex, which sample is diluted onto the wells of the microtitre plate. A labeled, e.g., biotin-labeled, secondary anti-cytokine antibody is added. The antibody cytokine complex can then be detected, i.e. by enzyme-conjugated streptavidin - cytokine-secreting cells will appear as "spots" by visual, microscopic, or electronic detection methods.

### 5.4.5. Tetramer Assay

In another option, the "tetramer staining" assay (Altman et al, 1996, Science 274: 94-96) may be used to identify antigen-specific T-cells. For example, in one option, an MHC molecule containing a specific peptide antigen, such as a tumor- specific antigen, is multimerized to make soluble peptide tetramers and labeled, for example, by complexing to streptavidin. The MHC-antigen complex is then mixed with a population of T cells obtained from a subject treated with a lectin-HSP-complex. Biotin is then used to stain T cells which express the antigen of interest, i.e., the tumor-specific antigen.

### 5.5. Monitoring of Effects During Cancer Prevention and Immunotherapy

The effect of immunotherapy with pharmaceutical compositions comprising multichaperone-antigen complexes on the development and progression of neoplastic diseases can be monitored by any method known to one skilled in the art, including but not limited to measuring: a) delayed hypersensitivity as an assessment of cellular immunity; b) activity of cytolytic T-lymphocytes *in vitro;* c) levels of tumor specific antigens, *e*.*g*., carcinoembryonic (CEA) antigens; d) changes in the morphology of tumors using techniques such as a computed tomographic (CT) scan; and e) changes in levels of putative biomarkers of risk for a particular cancer in individuals at high risk, and f) changes in the morphology of tumors using a sonogram.

The following subsections describe optional, exemplary procedures.

### 5.5.1. Delayed Hypersensitivity Skin Test

Delayed hypersensitivity skin tests are of great value in the overall immunocompetence and cellular immunity to an antigen. Inability to react to a battery of common skin antigens is termed anergy (Sato, T., et al., 1995, Clin. Immunol. Pathol. 74:35-43).

Proper technique of skin testing requires that the antigens be stored sterile at 4°C, protected from light and reconstituted shortly before use. A 25- or 27-gauge need ensures intradermal, rather than subcutaneous, administration of antigen. Twenty-four and 48 hours after intradermal administration of the antigen, the largest dimensions of both erythema and induration are measured with a ruler. Hypoactivity to any given antigen or group of antigens is confirmed by testing with higher concentrations of antigen or, in ambiguous circumstances, by a repeat test with an intermediate test.

### 5.5.2. Activity of Cytolytic T-Lymphocytes In Vitro

8x10⁶ Peripheral blood derived T lymphocytes isolated by the Ficoll-Hypaque centrifugation gradient technique, are restimulated with 4x10⁴ mitomycin C treated tumor cells in 3 ml RPMI medium containing 10% fetal calf serum. In some experiments, 33% secondary mixed lymphocyte culture supernatant or IL-2, is included in the culture medium as a source of T cell growth factors.

In order to measure the primary response of cytolytic T-lymphocytes after immunization, T cells are cultured without the stimulator tumor cells. In other experiments, T cells are restimulated with antigenically distinct cells. After six days, the cultures are tested for cytotoxicity in a 4 hour ⁵¹Cr-release assay. The spontaneous ⁵¹Cr-release of the targets should reach a level less than 20%. For the anti-MHC class I blocking activity, a tenfold concentrated supernatant of W6/32 hybridoma is added to the test at a final concentration of 12.5% (Heike M., et al., J. Immunotherapy 15:165-174).

### 5.5.3. Levels of Tumor Specific Antigens

Although it may not be possible to detect unique tumor antigens on all tumors, many tumors display antigens that distinguish them from normal cells. The monoclonal antibody reagents have permitted the isolation and biochemical characterization of the antigens and have been invaluable diagnostically for distinction of transformed from nontransformed cells and for definition of the cell lineage of transformed cells. The best-characterized human tumor-associated antigens are the oncofetal antigens. These antigens are expressed during embryogenesis, but are absent or very difficult to detect in normal adult tissue. The prototype antigen is carcinoembryonic antigen (CEA), a glycoprotein found on fetal gut an human colon cancer cells, but not on normal adult colon cells. Since CEA is shed from colon carcinoma cells and found in the serum, it was originally thought that the presence of this antigen in the serum could be used to screen patients for colon cancer. However, patients with other tumors, such as pancreatic and breast cancer, also have elevated serum levels of CEA. Therefore, monitoring the fall and rise of CEA levels in cancer patients undergoing therapy has proven useful for predicting tumor progression and responses to treatment.

Several other oncofetal antigens have been useful for diagnosing and monitoring human tumors, *e.g.,* alpha-fetoprotein, an alpha-globulin normally secreted by fetal liver and yolk sac cells, is found in the serum of patients with liver and germinal cell tumors and can be used as a matter of disease status.

### 5.5.4. Computed Tomographic (CT) Scan

CT remains the choice of techniques for the accurate staging of cancers. CT has proved more sensitive and specific than any other imaging techniques for the detection of metastases.

### 5.5.5. Measurement of Putative Biomarkers

The levels of a putative biomarker for risk of a specific cancer are measured to monitor the effect of compositions comprising cytosolic and membrane-derived proteins. For example, in individuals at enhanced risk for prostate cancer, serum prostate-specific antigen (PSA) is measured by the procedure described by Brawer, M.K., et al., 1992, J. Urol. 147:841-845, and Catalona, W.J., et al., 1993, JAMA 270:948-958; or in individuals at risk for colorectal cancer CEA is measured as described above in Section 4.5.3; and in individuals at enhanced risk for breast cancer, 16-α-hydroxylation of estradiol is measured by the procedure described by Schneider, J. et al, 1982, Proc. Natl. Acad. Sci. ISA 79:3047-3051.

### 5.5.6. Sonogram

A Sonogram remains an alternative choice of technique for the accurate staging of cancers.

### 5.6. Kits

Also disclosed herein are kits for carrying out the therapeutic regimens of the invention. Such kits comprise in one or more containers therapeutically or prophylactically effective amounts of the multichaperone-antigen complexes disclosed herein in pharmaceutically acceptable form. The multichaperone-antigen complexes in a vial of a kit as disclosed herein may be in the form of a pharmaceutically acceptable solution, e.g., in combination with sterile saline, dextrose solution, or buffered solution, or other pharmaceutically acceptable sterile fluid. Alternatively, the multichaperone-antigen complex may be lyophilized or desiccated; in this instance, the kit optionally further comprises in a container a pharmaceutically acceptable solution (e.g., saline, dextrose solution, etc.), preferably sterile, to reconstitute the complex to form a solution for injection purposes.

In one option, such kits comprise in one or more containers the multichaperone-antigen complexes disclosed herein in pharmaceutically acceptable form, for combining or combination therapy with HSP-antigen complexes that are provided in a second container. Preferably, the HSP-antigen complexes in the second container are gp96- antigen complexes.

In another option, a kit as disclosed herein further comprises a needle or syringe, preferably packaged in sterile form, for injecting the complex, and/or a packaged alcohol pad. Instructions are optionally included for administration of multichaperone- antigen complexes of the invention by a clinician or by the patient. The disclosure also provides a specific embodiment of a syringe containing a pharmaceutical composition of the invention.

In some options, the present disclosureprovides kits comprising a plurality of containers each comprising a pharmaceutical formulation or composition comprising a dose of multichaperone-antigen complexes as disclosed herein sufficient for a single therapeutic or prophylactic administration. Also disclosed herein are kits comprising a container comprising an immunologically and/or biologically active glycoprotein or a complex thereof, and a container comprising lectin. Optionally, instructions for formulating the oligomerized complexes according to the methods of the invention can be included in the kits.

In a specific option, a kit comprises a first container containing purified multichaperone-antigen complexes; and a second container containing a different treatment modality in an amount that, when administered before, concurrently with, or after the administration of the multichaperone-antigen complexes in the first container, is effective to improve overall treatment effectiveness over the effectiveness of the administration of each component alone, or is effective to decrease side effects of the treatment (e.g., as compared to side effects observed) when each component is used alone. In a preferred specific option, there is herein disclosed a kit comprising in a first container, a purified multichaperone-antigen complex as disclosed herein comprising a population of noncovalent antigen complexes obtained from cancerous tissue of a mammal; in a second container, a composition comprising a purified cancer chemotherapeutic agent; and in a third container, a composition comprising a purified cytokine.

In one option, a kit comprises in one or more containers a composition comprising mammalian HOP affinity molecules covalently bound to a solid phase. In a specific option, the HOP affinity molecules comprise a HOP affinity fragment or variant thereof selected from the group consisting of HOP TPR1 or a variant thereof, HOP TPR2a or a variant thereof, HOP TPR1/2a or a variant thereof, and a combination of any one or more of the foregoing. In a specific option, the HOP affinity molecules comprise a HOP affinity fragment or variant thereof that is present as a concatamer of two or more of HOP TPR1 or a variant thereof, HOP TPR2a or a variant thereof, and/or HOP TPR1/2a or a variant thereof. In a specific option, the HOP affinity molecules comprise a HOP affinity fragment or variant thereof that is present as a fusion protein of two or more of HOP TPR1 or a variant thereof, HOP TPR2a or a variant thereof, and/or HOP TPR1/2a or a variant thereof. In a specific option, the HOP affinity molecules comprise a human HOP affinity fragment or variant thereof. In a specific option, the solid phase comprises beads. The beads can be packed in a column or not packed in a column. In another option the solid phase comprises magnetic beads. In another specific option, the solid phase is a membrane. In another option, the solid phase has a surface comprising polycarbonate, polystyrene, polypropylene, polyethylene, glass, nitrocellulose, dextran, nylon, polyacrylamide or agarose.

### 6. EXAMPLES

### 6.1. Example 1: Expression of three Hop affinity fragments

The following three fragments of the human HOP protein sequence were cloned with C-terminal histidine tags into the pET24a(+) vector: HOP TPR1 (amino acid residues 1 to 118 of human HOP), HOP TPR2a (amino acid residues 223 to 352 of human HOP), and HOP TPR1/2A (amino acid residues 1 to 352 of human HOP).

*E. coli* strain BL21 (DE3) was transformed separately with the three TPR pET24a(+) constructs. These reagents were used to inoculate 10 mL of sterile LB-media containing 100 µg/mL kanamycin. A sample of inoculates was taken to assess protein expression prior to induction. Induction was achieved by using Overnight Express Instant TB media containing 100 µg/mL kanamycin, at 30°C with shaking at 300 rpm. Cell pellets were harvested and induced protein expression was detected by SDS-PAGE and by Western blot. SDS-PAGE was performed using a 4-20% SDS Tris-glycine gel and protein bands were visualized with GelCode Blue dye. Western blot analysis was performed with an anti-histidine antibody (Tetra-His antibody from Qiagen).

### 6.1.1. Results

SDS-PAGE and Western blot analysis for each of the TPR constructs of HOP were consistent with expression of each construct (FIGS. 1 - 3). Apparent molecular weights were consistent with those expected for each engineered protein (∼14 kDa for HOP TPR1, -16 kDa for HOP TPR2a, and -41.5 kDa for TPR1/2a). A positive Western blot was observed for each when probed with an anti-tetra-histidine antibody (Qiagen).
Detection of the HOP TPR proteins prior to induction was suggestive of somewhat leaky protein expression in this system.

### 6.2. Example 2: Purification of HOP TPR1 or HOP TPR1/2a from E. coli Pellets and Immobilization to NHS-Sepharose

### 6.2.1. Reagents

*Sonication Buffer:* 10 mM sodium phosphate, 150 mM sodium chloride (pH 7.2) with 1X Bugbuster (Novagen Cat# 70921), 1 EDTA free protease inhibitor pellet per 50 mL of buffer (Roche Cat# 11 873 580 001), 10 µg/mL Dnase I (Roche Cat # 10 104 159 001) and 200 µg/ml lysozyme (Sigma Cat # L-6876). *Nickel Column Conditioning Buffer:* 10 mM sodium phosphate, 150 mM sodium chloride, 5 mM imidazole (pH 7.2). *Nickel Column Elution Buffer:* 10 mM sodium phosphate, 150 mM sodium chloride, 500 mM imidazole (pH 7.2). *Sephacryl Conditioning*/*Running Buffer:* 10 mM sodium phosphate, 300 mM sodium chloride (pH 7.2). Nickel Resin (Qiagen Cat# 30450). Sephacryl S-200 Resin (GE Cat# 17-0584-01)

### 6.2.2. Preparation of bacterial pellet:

A 5 - 8 gram pellet of *E. coli* cells was added to a container and resuspended with ∼25 mL of sonication buffer. The resuspended pellet was transferred to a second container filled with sonication buffer to achieve a 10x buffer to pellet ratio (*e.g.* 100 mL buffer to 10 g cell pellet). This container was placed in an ice bucket and sonicated for 30 seconds, followed by a 30 second rest. The process was repeated 3 times after which the sample was allowed to settle on ice for 30 minutes. The sonication process was repeated and the suspension was transferred to centrifuge tubes. Cell debris was removed by centrifugation (14,000x g for 10 minutes at 4°C). The supernatant was collected and filtered through 0.45 µm filters (Sartorius Cat# 17829). Nickel elution buffer was added to the recovered supernatant to achieve a final concentration of 5 mM imidazole (1 mL/100 mL sample).

### 6.2.3. Metal Affinity and Gel Filtration Chromatography:

Approximately 10 - 20 mL of nickel resin was used to isolate the histidine-tagged HOP affinity fragment (HOP TPR1 or HOP TPR1/2a) from a 5 - 8 gram pellet. The filtered supernatant was loaded onto the nickel column which was subsequently washed with 20 column volumes of the nickel column-conditioning buffer. The HOP affinity fragment was recovered using a step elution to nickel column elution buffer. Purity and identity of the HOP affinity fragment was confirmed by SDS-PAGE and Western blot analysis using an anti-histidine-tag antibody. The HOP affinity fragment pool was further isolated by a gel filtration column (Sephacryl S-200). The HOP affinity fragment was collected and analyzed by SDS-PAGE and quantified by the Bradford assay. The HOP affinity fragment was concentrated by ultrafiltration using a 3,000 Da. (HOP TPR1) or 10,000 Da. (HOP TPR1/2a) molecular weight cutoff filter to a target concentration of 10 mg/mL. The HOP affinity fragment was immobilized on resin or stored at -80°C.

### 6.2.4. Immobilization of HOP TPR1 or HOP TPR1/2a to NHS Sepharose

HOP TPR1 or HOP TPR1/2a was immobilized at a ratio of 10 mg per mL of NHS Sepharose resin The HOP affinity fragment (HOP TPR1 or HOP TPR1/2a) was exchanged in to a HEPES buffer (50 mM HEPES, 500 mM sodium sulfate, pH 8.6) following its isolation by gel filtration. This solution was used to immobilize the HOP affinity fragment to NHS-Sepharose 4 fast flow resin. The resin was washed with 1 mM HCl. Approximately ¾ of the reagent solution was reacted with the washed NHS resin at room temperature with end over end rotation for at least 2 hours. After washing with 1 M Tris pH 9.0, the resin was incubated with the same buffer overnight to block NHS groups that had not reacted with reagent molecules. The resin was washed with 20% ethanol and a resin to 20% ethanol slurry of 1:2 was prepared for storage at 4°C.

### 6.2.5. Results

A UV chromatogram collected at a wavelength of 280 nm and an SDS-PAGE analysis of the HOP affinity fragments isolated by metal affinity chromatography and by gel filtration demonstrated effective purification of both HOP TPR1 (FIGS. 4 A-D) and HOP TPR1/2a (FIGS. 5 A-D) from *E. coli* pellets by nickel affinity chromatography followed by gel filtration. This process also reduced endotoxin levels in these reagents. Endotoxin levels were typically reduced from ∼2,000 EU/mL to <100 EU/mL as measured by the *Limulus* amoebocyte lysate (LAL) assay.

### 6.3. Example 3: Purification of gp96-antigen complexes

### 6.3.1. Anti-gp96 scFv Isolation Method

Anti-gp96 scFv was immobilized to NHS-activated Sepharose using a method similar to the method for immobilizing the HOP affinity molecules (see Section 6.2.4). In particular, the immunoaffinity resin was prepared with a ratio of 10 mg of scFv per mL of resin at a concentration of 10 mg of scFv per mL of buffer. NHS-activated Sepharose 4 Fast Flow resin in isopropanol was washed with cold 1 mM HCl, and resuspended with a solution of the scFv in 50 mM Borate 500 mM sodium chloride (pH 9.0). This mixture was incubated with rotation for 2 hours at room temperature. Subsequently, the resin was washed with 1 M Tris (pH 9) to remove unbound protein, and blocked by overnight incubation with rotation in the same Tris buffer. The resin was washed with 1.3 M sodium chloride in 10 mM sodium phosphate (pH 7.2), then 1.3 M sodium chloride in 10 mM sodium phosphate (pH 7.2) containing 20% ethanol and stored at 4°C.

Before use, the resin was packed into a column of appropriate size (1 mL of resin per 10 g of tissue) and washed with 5 column volumes of 1.3 M sodium chloride in 10 mM sodium phosphate (pH 7.2). Subsequently, the column was equilibrated with 10 column volumes of 30 mM sodium phosphate and 1.5 mM magnesium chloride (pH 7.2).

A tissue homogenate derived from mouse methylcholanthrene-induced fibrosarcoma tissue (Meth A) was prepared in the 30 mM sodium phosphate and 1.5 mM magnesium chloride (pH 7.2) buffer containing cocktail III protease inhibitors, clarified by sedimentation (36,000x g for 1 hour at 4°C) and filtered through a 5 µm filter. The clarified homogenate was applied to the column of immobilized scFv at a flow rate of 70 cm/hour and chased with 5 column volumes of 30 mM sodium phosphate and 1.5 mM magnesium chloride (pH 7.2). The clarified homogenate that was applied to the column of immobilized scFv at a flow rate of 70 cm/hour could have optionally been chased with the same solution containing up to 25 mM NaCl. The column was washed with 10 column volumes of 10 mM sodium phosphate containing 240 mM sodium chloride (pH 7.2). gp96 was eluted from the column using 1.3 M sodium chloride in 10 mM sodium phosphate (pH 7.2) in 20 quarter column volume fractions. gp96 could have optionally been eluted from the column using 900 mM sodium chloride in 10 mM sodium phosphate (pH 7.2) in 20 quarter column volume fractions. A rough Bradford assay was performed for each fraction and those with highest protein concentration (as noted by deepness of blue color) were combined. This material was buffer exchanged into 5 mM potassium phosphate buffer containing 9% (wt/volume) Sucrose (pH 7.3) using PD-10 cartridges (G-25 size exclusion media).

The flow through of the scFv column was subsequently used to isolate multichaperone-antigen complex fractions (HOP TPR2a, HOP TPR1/2a and a mixed bed of HOP TPR1 and HOP TPR1/2a) or modified by the addition of sodium chloride to a concentration of 50 mM before it is applied to a column of resin conjugated HOP TPR1.

### 6.4. Example 4: Purification of Multichaperone-antigen Complexes using HOP TPR1

Optimization of a method for purification of multichaperone-antigen complexes from tissues by immobilized HOP TPR1 included investigation of HOP TPR1 immobilization to resin, tissue load conditions, and multichaperone- antigen elution conditions.

### 6.4.1. Reagents:

*Homogenization Buffer:* 30 mM sodium phosphate, 1.5 mM magnesium chloride (pH 7.2). *TPR1-Sepharose Conditioning Buffer:* 30 mM sodium phosphate, 1.5 mM magnesium chloride, 50 mM sodium chloride (pH 7.2). *TPR1-Sepharose Pre-Conditioning*/*Elution Buffer* 20 mM Tris, 500 mM sodium chloride (pH 9.0). 5 M sodium chloride solution in water.

### 6.4.2. Tissue Preparation:

Frozen tissue was removed from freezer and thawed slightly in homogenization buffer at a volume equal to 4x the tissue weight. Once semi-thawed, the tissue was homogenized in a blender and clarified by centrifugation (36,000x g for 1 hour at 4°C). The clarified homogenate was recovered and filtered through 0.45 µm filters. The sodium chloride concentration of the clarified homogenate was increased to 50 mM by addition of a 5 M aqueous solution of sodium chloride (1 mL/100 mL supernatant).

### 6.4.3. Chromatography:

TPR1-Sepharose was used at a ratio of 1 mL of resin to 1 g of tissue. For a 20 g tissue sample, a 20 mL column was packed in a column of 2.5 cm internal diameter with a slurry of the TPR1 Sepharose resin in TPR1-Sepharose conditioning buffer. For smaller tissue samples the method was linearly scaled. The column was washed with 5 column volumes of the TPR1 Sepharose pre-conditioning buffer at a flow rate of 70 cm/hr, then conditioned with 5 column volumes of the TPR1 Sepharose conditioning buffer at 70 cm/hr. The clarified homogenate was loaded at a flow rate of 70 cm/hr and washed with 5 column volumes of the TPR1 Sepharose conditioning buffer. A multichaperone fraction was isolated by step elution to TPR1 Sepharose elution buffer for 5 column volumes and 0.5 column volume fractions were collected. Fractions were pooled according to UV absorbance at 280 nm, rough Bradford quantification or results of SDS-PAGE analysis. In process samples (flow through and wash) were collected for analysis.

### 6.4.4. Analysis:

SDS-PAGE analyses were performed using 4-20% SDS Tris-glycine gels to evaluate the consistency and purity of isolated multichaperone preparations. Western blot analyses were performed to elucidate the presence of HSPs in isolated fractions.

### 6.4.5. Results and Discussion

During initial experiments, sodium chloride was added to a phosphate buffer (pH 7.2) to elute captured HSPs. Chromatographically, these conditions led to broad elution profiles as monitored by SDS-PAGE (FIG. 6A). Increasing sodium chloride concentration did not improve HSP elution (FIG. 6B). Therefore, an experiment was performed to examine HSP elution at increased pH using a Tris (20 mM) buffer with and without added sodium chloride at pH 8.0 (FIGS. 6C and 6D) or pH 9.0 (FIGS. 6E and F). In this experiment a pH of 9.0 with or without addition of 1.5 M sodium chloride provided best HSP yield from the resin (FIG. 6F). Increasing pH of the elution buffer beyond pH 9.0 had no benefit, and may have caused breakdown of isolated HSPs (FIG. 6G). Subsequently, sodium chloride (500 mM) was added to the pH 9.0 Tris buffer to reproducibly isolate multichaperone preparations from resin immobilized HOP TPR1 (FIG. 7).

Multichaperone preparations isolated using a 5 mL column of resin immobilized HOP TPR1 from 5 g of mouse organ tissue harvested from tumor bearing mice were characterized by two intense bands that migrated at approximate molecular weights of 70 kDa. and 110 kDa. (FIG. 7A). The 70 kDa. band comprised HSP70 and the 110 kDa. band comprised HSP110 as detected by Western blots with appropriate antibodies (FIG. 7B). The combined density of these protein bands in the SDS-PAGE gel typically constituted 70 - 80 % (∼50% to 60% HSP70 and ∼20% HSP110) of detected proteins as assessed by laser densitometry. Western blots using antibodies raised against other HSPs were performed, and demonstrated that elution pools comprised various heat shock proteins including HSP40, HSP70, HSC70, HSP110, HIP, and Calreticulin (see Example 6 at Section 6.6).

It was of interest to determine if a similar pattern of protein bands would be observed by SDS-PAGE for preparations made from other tissues, e.g., mouse methylcholanthrene-induced fibrosarcoma (Meth A, FIG. 8). For these studies, Meth A tissue homogenate was prepared from two separate pools (20 g each) of frozen tissue as described in Section 6.4.2 except that the sodium chloride concentration of the clarified homogenate was not increased to 50 mM. The homogenate was then depleted of gp96 using an anti-gp96 scFv immunoaffinity column. The flow through from the scFv column was then modified by addition of sodium chloride (to 50 mM) before processing by a 20 mL column of resin immobilized HOP TPR1 according to the chromatography procedure described in Section 6.4.3. Each Meth A preparation was consistent with the other and had a similar protein pattern to that of the mouse organ preparations, as detected by SDS-PAGE. HSP70 and HSP110 were abundant proteins in both preparations. HSP70 prepared by ADP agarose affinity chromatography was detected as a control for the expected migration of HSP70 isolated by HOP TPR1 (FIG. 8).

The purity of multichaperone preparations isolated by resin immobilized HOP TPR1 was improved by addition of sodium chloride to the clarified homogenate. Increasing sodium chloride concentration of the clarified homogenate had clear purity advantages, as demonstrated by SDS-PAGE (FIG. 9 A-C). The multichaperone preparation isolated from a homogenate that contained 50 mM sodium chloride (FIG. 9C) was empirically "cleaner" than that prepared from a homogenate containing 25 mM sodium chloride (FIG. 9A). The eluate elution profile was also improved by the addition of sodium chloride in the clarified homogenate.

As the degree of loading of a HOP affinity fragment on to resin can affect quality and yield of isolates, the effects of HOP TPR1 loading on NHS-sepharose were investigated. The quality and yield of multichaperone-antigen eluates were compared for HOP TPR1 loading conditions of 10, 15 and 20 mg of HOP TPR1 protein/mL of resin (FIGS. 10A-B). Homogenates prepared from organs harvested from tumor bearing mice were used as the resin immobilized HOP TPR1 feedstock. The quality of each preparation was assessed by the amount of HSP70 detected by laser densitometry of SDS-PAGE gels (FIG. 10B). From this parameter there were no differences observed between the preparations when eluted from the column with a buffer comprising 20 mM Tris and 150 mM sodium chloride at pH 9.0. However, the yield was much improved when 10 mg of HOP TPR1 was loaded per mL of NHS-sepharose.

In summary it was apparent that addition of sodium chloride to the clarified homogenate improved the purity of the multichaperone preparation, which was most efficiently eluted with a 500 mM sodium chloride solution at pH 9.0. These conditions were used to prepare material for investigations of tumor rejection activity in preclinical models (see Example 7 at Section 6.7).

### 6.5. Example 5: Analysis of HOP TPR1 Eluate by Liquid chromatography/tandem mass spectrometry (LC/MS/MS)

The composition of a HOP TPR1 preparation of Meth A tissue was examined by LC/MS/MS. To prepare the HOP TPR1 preparation, frozen Meth A tissue was thawed, homogenized and clarified as described in Section 6.4.2. The homogenate was then subjected to chromatography, fractions were collected and pooled and subjected to SDS-PAGE as described in Sections 6.4.3 and 6.4.4. Following separation by SDS-PAGE, bands were excised from the gel and the proteins therein were digested with trypsin. Peptides were isolated and analyzed by LC/MS/MS. Peptide sequences were identified from unprocessed tandem MS spectra by contemporary database searching algorithms.

### 6.5.1. Protein Separation and In-gel Digest Conditions

Samples of the multichaperone fraction prepared from Meth A tissue were loaded into and separated on a 4-20% Tris-Glycine gel. Coomassie stained bands of the same molecular weight were excised from multiple lanes, cut into small pieces and pooled for further processing to generate a tryptic digest.

### 6.5.2. Mass Spectrometric Analysis of Peptide Fractions

LC/MS/MS analysis of trypsin digested protein bands identified proteins that were co-purified using the resin immobilized HOP TPR1 reagent. All LC/MS experiments were performed using an LCQ-Deca Mass Spectrometer (ThermoFisher) and an ADVANCE electrospray ionization (ESI) source (Michrom Bioresources Inc.) in positive ion mode. Chromatography was performed using a Surveyor HPLC (ThermoFisher) to deliver solvent to a Luna C18 reversed phase column, 75 µm ID X 10 mm, of 3 µm particles (Phenomenex Inc.). Mobile phases were modified with 10 mM ammonium hydroxide. Mascot software (Version 2.2.0, Matrix Sciences) was used to identify peptides from LC/MS/MS spectra and correlate them to a protein sequence. The database used for this purpose was SwissProt_54.5.

### 6.5.3. Results and Discussion

SDS-PAGE results were consistent with analyses of other Meth A samples prepared by resin immobilized HOP TPR1 (FIG. 11). From this gel several protein bands that exceeded∼1-2 % of the sample composition (as evaluated by laser densitometry) were analyzed by LC/MS/MS (FIG. 11). The most abundant protein of each of the analyzed bands was identified. Interestingly, HSP 110 was detected in three high molecular weight bands. The reason for this result is unknown. It was possible that this observation was due to an artifact of the analysis. However, it is also possible that these signals represent partially dissociated HSP110 complexes even though samples were boiled in SDS before being loaded on the gel. In addition to HSP110, the most abundant protein band was identified as HSP70. Other protein constituents included HSP90, tubulin, elongation factor 1 (EF1), and actin. In this experiment, additional HSPs that had been previously detected by Western blots (see Example 6 at Section 6.6) were not identified in the excised bands. This suggested that other HSPs that comprise this mixture were likely present in catalytic amounts in these preparations.

This example demonstrates the feasibility of identifying protein components of multichaperone preparations by LC/MS/MS.

### 6.6. Example 6: Detection of Multichaperone Complexes

It was hypothesized that the HOP affinity fragments would isolate protein complexes that comprise several HSPs. To test this hypothesis, multichaperones were isolated from either normal organs of tumor bearing mice or from the human leukemia cell line K562 using the HOP TPR1 affinity reagent. The respective frozen mouse and human tissues were thawed, homogenized and clarified as described in Section 6.4.2. The homogenate was then subject to HOP TPR1 chromatography, fractions were collected and pooled and subjected to SDS-PAGE as described in Sections 6.4.3 and 6.4.4. The most abundant chaperone isolated by resin immobilized HOP TPR1 was HSP70 (FIG. 11). However, minimal HSP90 was detected in such multichaperone preparations (FIG. 11). Instead HSP110 was consistently the second most abundant HSP isolated by resin immobilized HOP TPR1. As HSP110 is known to bind to HSP70, we investigated whether HOP TPR1 had isolated complexes of HSP70 with HSP110. This experiment was performed using glutaraldehyde, a small molecule dialdehyde that reacts with primary amines that are in close proximity with each other. For protein chemistry, this reagent is used to effectively cross link protein complexes that are formed by non-covalent interactions. Due to increased molecular size, cross-linked proteins migrate slower in SDS-PAGE gels and are detected with higher apparent molecular weight. This approach was used in conjunction with Western blot analysis to analyze the composition of multichaperone- antigen preparations prepared by resin immobilized HOP TPR1.

### 6.6.1. Results

Glutaraldehyde cross-linked multichaperone preparations isolated from mouse tissues by resin immobilized HOP TPR1 were separated by SDS-PAGE. Typical gel shift patterns were observed for cross-linked proteins isolated from mouse (FIG. 12A) or the human cell line, K562 (FIG. 12B). In both examples, the most intense protein band was detected with an apparent molecular weight of ∼200 kDa. Western blots using antibodies specific to HSP70, HSP110, HSP40 and HIP indicated all were components of the -200 kDa. protein band that was observed in the mouse sample (FIG. 13). Calreticulin was also detected in this preparation. This HSP migrated with an apparent molecular weight consistent with its molecular size and not in the -200 kDa. band and internally controlled the experiment (FIG. 14). This result suggested that this chaperone was not a component of the large macromolecular complexes detected in FIGS. 12 and 13. This result was an internal control for glutaraldehyde cross-linking studies and indicated that the reagent was used at a concentration that effectively cross-linked only those proteins that were in close proximity.

As expected, resin immobilized HOP TPR1 isolated HSP complexes from mouse tissue. Contrary to initial predictions, this reagent isolated complexes of HSP70 with HSP110, and other smaller HSPs and not complexes of HSP70 with HSP90. Migration of calreticulin at a molecular weight that was consistent with the molecular weight of this chaperone internally controlled the experiment, and confirmed that the glutaraldehyde reaction conditions were sufficiently controlled to enable cross-linking of only those proteins that were in close proximity and components of multichaperone complexes.

### 6.7. Example 7: Investigation of Tumor Rejection Activity of the Multichaperone Preparation Isolated by Resin Immobilized HOP TPR1

The mouse Meth A model has been used extensively to investigate the tumor rejection activity of HSP preparations. This model was used in a prophylaxis setting to evaluate activity of the same two multichaperone-antigen preparations isolated by resin immobilized HOP TPR1 described in Section 6.4.5. The final product intended for immunization of mice was formulated in a phosphate buffer that contained sucrose (5 mM potassium phosphate, 9% sucrose, pH 7.2) by buffer exchange using a PD-10 gel filtration cartridge. In addition, gp96 eluted from the scFv immunoaffinty column from one of the two starting tissue samples was tested for tumor rejection activity. For the prophylaxis setting mice were vaccinated with the multichaperone preparation on the initial day of the experiment and seven days later. These animals were then inoculated with 10⁵ Meth A cells one week after the second vaccination. Various doses of the multichaperone preparation were used including 1.7 µg, 5 µg and 16.7 µg (as assessed by the Bradford total protein assay). Groups of mice treated with buffer or 2 x 10⁷ irradiated Meth A cells controlled this experiment. For comparison, a group of mice was vaccinated with gp96. In addition, a group of mice was vaccinated with a mixture of the multichaperone preparation (5 µg) with gp96 (3 µg). Tumor measurements were made every 3 to 4 days. In a follow on experiment mice were vaccinated with doses of a HOP TPR1 multichaperone preparation ranging from 0.1 µg to 3 µg. Other conditions of the experiment were as described above.

### 6.7.1. Results and Discussion

The quality of the multichaperone preparations used in these investigations was consistent with other Meth A and mouse organ preparations (FIG. 8). Tumor rejection was observed in all groups except for the buffer control. High activity of the multichaperone preparation was observed with 10 of 10 mice rejecting their tumor following vaccination with 1.7 µg doses of one of the multichaperone preparations and 9 of 10 mice rejecting their tumor follow vaccination with the same dose of the other multichaperone preparation (FIG. 15). Good activity was also observed at the other two dose levels of the multichaperone preparations, and following vaccination with gp96. In this experiment 8 of 10 mice rejected tumor when vaccinated with a combination of the multichaperone preparation and gp96. A titration of dose performance was observed in the follow on experiment (FIG. 16). Protection was highest when animals were vaccinated with 3 µg doses of the multichaperone preparation (8/10 animals rejected tumor) although it is also notable that 50% of mice reject their tumor at a dose as low as 0.5 µg.

These data confirmed that resin immobilized HOP TPR1 isolated a multichaperone preparation that had high anti-tumor activity. Two independent multichaperone preparations were observed to elicit robust tumor rejection activity across replicate experiments using doses between 0.5 and 3 µg. Compatibility of extraction of multiple chaperones using the immobilized HOP TPR1 method with a separate process for isolating gp96 was demonstrated. These encouraging results highlight a high commercial potential for isolating gp96 followed by additional HSPs in the form of multichaperone antigen complexes from the same tissue source to create multicomponent vaccines for treatment of cancers and infectious diseases.

### 6.8. Example 8: A Multichaperone Preparation Isolated from Human Tissue by Resin Immobilized HOP TPR1

While preclinical testing requires isolation of mouse chaperones, commercial opportunities rely on the ability of resin immobilized HOP TPR1 reagent to isolate HSPs from human tumors. The human leukemia cell line K562 was used for these investigations. An additional objective of this experiment was to combine the HOP TPR1 method with an approach for isolating gp96. K562 cells were homogenized as described in Section 6.4.2 except that the sodium chloride concentration of the clarified homogenate was not increased to 50 mM. The clarified homogenate was then depleted of gp96 using an anti-gp96 scFv affinity immunoaffinity column, as described in Section 6.3.1. Sodium chloride was added to material that flowed through the scFv column to achieve a concentration of 50 mM, and this material was passed through the immobilized HOP TPR1 resin according to the chromatography procedure described in Section 6.4.3. Sample analysis was by SDS-PAGE.

### 6.8.1. Results

As expected, gp96 was isolated from K562 cells in high purity (FIG. 17). The resin immobilized HOP TPR1 column efficiently isolated HSPs from the sodium chloride modified flow through of the column of immobilized gp96 immunoaffinity reagent. From this feedstock the HOP TPR1 resin isolated predominantly HSP70 and HSP110, which was consistent with observations made from extracts of mouse tissues (see FIGS. 7A and 8). Both HSP70 and Hsc70, as characterized by the doublet of proteins bands detected with an apparent 70 kDa. molecular weight, were isolated from this human tissue by resin immobilized HOP TPR1 (FIG. 17). Detection of these isoforms of HSP70 in the K562 extract was consistent with expression levels of these proteins and the composition of extracts prepared by literature reported HSP70 isolation methods.

In this example the efficacy of resin immobilized HOP TPR1 for extraction of human HSPs was demonstrated. The compatibility of this method with a process for extraction of gp96 was shown. These results were consistent with observations made for mouse tissues, and support potential commercial benefit of the described approaches for preparation of human vaccines.

### 6.9. Example 9: Purification of Multichaperone Complexes from HOP TPR1/2a

HOP TPR1/2a, which encompasses the TPR1-DP1-TPR2a domains was immobilized to resin and the efficacy of this reagent for preparing multiple chaperones from tissue homogenates was evaluated.

### 6.9.1. Reagents:

*Homogenization Buffer:* 30 mM sodium phosphate, 1.5 mM magnesium chloride (pH 7.2). *TPR1*/*2a Sepharose Elution Buffer:* 10 mM sodium phosphate, 500 mM sodium chloride (pH 7.2).

### 6.9.2. Tissue Preparation:

Frozen tissue was removed from freezer and thawed slightly in homogenization buffer at a volume equal to 4x the tissue weight. Once semi-thawed, the tissue was homogenized in a blender and clarified by centrifugation (36,000x g for 1 hour at 4°C). The clarified homogenate was recovered and filtered through 0.45 µm filters.

### 6.9.3. Chromatography:

TPR1/2a-Sepharose was used at a ratio of 1 mL of resin to 3 g of tissue. A 7 mL column was packed in a column of 1 cm diameter with a slurry of TPR1/2a-Sepharose resin in TPR1/2a-Sepharose conditioning buffer. The column was conditioned with 5 column volumes of the same buffer. The clarified homogenate was loaded at a flow rate of 70 cm/hr and washed with 5 column volumes of homogenization buffer. A multichaperone fraction was isolated by step elution to TPR1/2a-Sepharose elution buffer for 5 column volumes and 0.5 column volume fractions were collected. Fractions were pooled according to UV absorbance at 280 nm, rough Bradford quantification or results of SDS-PAGE analysis. In process samples (load, flow through and wash) were collected for analysis. Following elution of resin immobilized HOP TPR1/2a with TPR1/2a-Sepharose elution buffer, the resin was also eluted with TPR1-Sepharose Pre-Conditioning/Elution Buffer (20 mM Tris, 500 mM sodium chloride, pH 9.0) to assess whether HSP elution was complete. SDS-PAGE and Western blot analysis were performed on in process and the elution pool.

### 6.9.4. Results and Discussion

A 3 mL column of resin immobilized HOP TPR1/2a isolated a HSP90 rich preparation from a 10g sample of organ tissue harvested from tumor bearing mice (FIGS. 18A and B). HSP70 was the only other protein band of significance in this preparation. Both proteins accounted for ∼85% (∼70% HSP90 and ∼15% HSP70) of the protein band density detected by laser densitometry of the stained gel image. Resin elution with TPR1-Sepharose Pre-Conditioning/Elution Buffer isolated an additional HSP-rich fraction, comprising HSP70, and HSP90 in somewhat trace amounts (FIG. 18C). These results confirmed resin immobilized HOP TPR1/2a was able to isolate HSPs from mouse organ tissue.

### 6.10. Example 10: Analysis of HOP TPR1/2a Eluate by Liquid chromatography/Tandem Mass Spectrometry (LC/MS/MS)

The composition of a HOP TPR1/2a preparation of mouse organs was examined by LC/MS/MS. To prepare the HOP TPR1/2a preparation, frozen mouse organs were thawed, homogenized and clarified as described in Section 6.9.2. The homogenate was then subjected to chromatography, fractions were collected and pooled and subjected to SDS-PAGE as described in Section 6.9.3. Following separation by SDS-PAGE, protein bands that reflect the composition of the HSPs isolated by the resin immobilized HOP TPR1/2a were excised from the gel and digested with trypsin. Peptides were isolated and analyzed by LC/MS/MS. Peptide sequences were identified from unprocessed tandem MS spectra by contemporary database searching algorithms.

### 6.10.1. Protein Separation and In-gel Digest Conditions

Samples of the multichaperone fraction prepared from mouse organs were loaded into and separated on a 4-20% Tris-Glycine gel. Coomassie stained bands of the same molecular weight were excised from multiple lanes, cut into small pieces and pooled for further processing to generate a tryptic digest.

### 6.10.2. Mass Spectrometric Analysis of Peptide Fractions

LC/MS/MS analysis of trypsin digested protein bands identified proteins that were co-purified by using the immobilized HOP TPR1/2a resin. All LC/MS experiments were performed using an LCQ-Deca Mass Spectrometer (ThermoFisher) and an ADVANCE electrospray ionization (ESI) source (Michrom Bioresources Inc.) in positive ion mode. Chromatography was performed using a Surveyor HPLC (ThermoFisher) to deliver solvent to a Luna C18 reversed phase column, 75 µm ID X 10 mm, of 3 µm particles (Phenomenex Inc.). Mobile phases were modified with 10 mM ammonium hydroxide. Mascot software (Version 2.2.0, Matrix Sciences) was used to identify peptides from LC/MS/MS spectra and correlate them to a protein sequence. The database used for this purpose was SwissProt_54.5.

### 6.10.3. Results and Discussion

Mass spectrometry analysis of trypsin-digested protein bands identified proteins that were co-purified by resin immobilized TPR1/2a (using the HOP TPR1/2a Sepharose elution buffer to isolate HSPs from the resin). Interestingly, HSP90 was detected in three protein bands of highest molecular weight (FIG. 19). This was similar to the results described in Example 5 at Section 6.5, where for a HOP TPR1 eluate, HSP110 was detected in three protein bands of highest molecular weight. While these bands may have been an artifact of the analysis, they may also represent partially denatured protein complexes of HSP90. Other proteins identified included HSP70, tubulin, carbomyl phosphate synthase, and glutamate dehydrogenase (FIG. 19). The latter two proteins are liver enzymes derived from the predominant organ in the extracted tissue.

Feasibility of identifying protein components of multichaperone preparations by LC/MS/MS was demonstrated.

### 6.11. Example 11: Purification of Multichaperone Complexes from a Mixed Bed Resin Comprising HOP TPR1 and HOP TPR1/2a

In this example, a mixed bed comprising resin immobilized HOP TPR1, and resin immobilized HOP TPR1/2a was used to isolate HSPs from mouse organ tissue.

### 6.11.1. Reagents:

*Homogenization Buffer* 30 mM sodium phosphate, 1.5 mM magnesium chloride (pH 7.2). *TPRI-Sepharose Pre-ConditioninglElution Buffer:* 20 mM Tris, 500 mM sodium chloride (pH 9.0).

### 6.11.2. Tissue Preparation:

Frozen tissue was removed from freezer and thawed slightly in homogenization buffer at a volume equal to 4x the tissue weight. Once semi-thawed, the tissue was homogenized in a blender and clarified by centrifugation (36,000x g for 1 hour at 4°C). The clarified homogenate was recovered and filtered through 0.45 µm filters.

### 6.11.3. Chromatography:

A mixed bed comprising TPR1-Sepharose (2.5 mL) and TPR1/2a-Sepharose (1.6 mL) was used to isolate HSPs from 5 g of mouse organ tissue. The column was washed with 5 column volumes of the TPR1 Sepharose pre-conditioning/elution buffer at a flow rate of 70 cm/hr, then conditioned with 5 column volumes of homogenization buffer at 70 cm/hr. The clarified homogenate was loaded at a flow rate of 70 cm/hr and washed with 5 column volumes of homogenization buffer. A multichaperone fraction was isolated by step elution to TPR1/2a Sepharose pre-conditioning/elution buffer for 5 column volumes and 0.5 column volume fractions were collected. Fractions were pooled according to UV absorbance at 280 nm, rough Bradford quantification or results of SDS-PAGE analysis. In process samples (flow through and wash) were collected for analysis. SDS-PAGE analyses were performed using 4-20% SDS Tris-glycine gels and Western blots were performed to elucidate the presence of HSPs in tissue isolates.

### 6.11.4. Results

As shown by SDS-PAGE analysis, a mixed bed of HOP TPR1-Sepharose and HOP TPR1/2a-Sepharose successfully isolated HSPs from mouse organ tissue (FIG. 20). It was determined from the SDS-PAGE gel that the eluate comprised the three HSPs (HSP70, HSP90, and HSP110) that were isolated by the resins individually. The approximate contribution of these HSPs to the total protein content of the fraction (as measured by laser densitometry of this example) was ∼13% HSP70, ∼68% HSP90, and ∼7% HSP110. The presence of these and other HSPs in the eluate of the mixed bed resin were confirmed by Western blots using appropriate antibodies (FIG. 21). This analysis determined that HSP40, HIP and calreticulin were also components of the eluate, albeit at levels that were below detection limits of the SDS-PAGE gel.

### 6.12. Example 12: Purification of HOP TPR2a from E. coli Pellets and Immobilization to NHS-Sepharose

### 6.12.1. Reagents:

*Sonication Buffer:* made in 30 mM sodium phosphate, 1.5 mM magnesium chloride (pH 7.2) with 1 EDTA free protease inhibitor pellet per 50 ml of buffer (Roche Cat# 11 873 580 001), and 10 µg/mL nase I (Roche Cat # 10 104 159 001). Dilu*ent Buffer:* 30 mM sodium phosphate, 1.5 mM magnesium chloride, 500 mM imidazole (pH 7.2) *Nickel Column Conditioning Buffer:* 30 mM sodium phosphate, 1.5 mM magnesium chloride, 10 mM imidazole (pH 7.2). *Nickel Column Elution Buffer:* 30 mM sodium phosphate, 1.5 mM magnesium chloride, 250 mM imidazole (pH 7.2). *Superdex 75 Conditioning*/*Running Buffer:* 10 mM sodium phosphate, 150 mM sodium chloride (pH 7.2). Nickel Resin (Qiagen Cat# 30450)

### 6.12.2. Preparation of bacterial pellet:

A 5 - 7 g pellet of *E*. *coli* cells was added to a container and resuspended with -35 mL sonication buffer. This container was placed in an ice bucket and sonicated for 30 seconds, followed by a 15 second rest. The process was repeated 3 times, before the lysate was centrifuge at 14,000x g for 30 minutes at 4°C. The supernatant was decanted and saved for processing. The pellet was resuspended in 15 mL of sonication buffer and sonicated as described above. This mixture was centrifuged at 36,000x g for 30 minutes at 4°C. The supernatant was collected, combined with the first and filtered through 0.45 µm filters (Sartorius Cat# 17829). Diluent buffer was added to the recovered supernatant to a final concentration of 10 mM imidazole (1 mL/50 mL sample).

### 6.12.3. Metal Affinity and Gel Filtration Chromatography:

A column containing 5 mL of nickel resin was used to isolate the histidine-tagged HOP TPR2a reagent from bacterial supernatant. Subsequently, the column was washed with 15 column volumes of the nickel column-conditioning buffer and the reagent was recovered using a gradient of the nickel column elution buffer. Purity and identity of the reagent was assessed by SDS-PAGE and Western blot analysis using an anti-histidine-tag antibody. The reagent pool was further isolated by gel filtration (Superdex 75). The reagent was collected and analyzed by SDS-PAGE and quantified by the Bradford assay. The reagent was concentrated by ultrafiltration using a 3,000 or 5,000 molecular weight cutoff filter to a target concentration was 10 mg/mL. This reagent was immobilized on resin or stored frozen at -80°C.

### 6.12.4. Immobilization of HOP TPR2a to NHS Sepharose

HOP TPR2a was immobilized at a ratio of 10 mg per mL of NHS Sepharose resin. The HOP reagent was exchanged in to a HEPES buffer (50 mM HEPES, 500 mM sodium sulfate, pH 8.6) following its isolation by gel filtration. This solution was used to immobilize the HOP reagent to NHS-Sepharose 4 fast flow resin. The resin was washed with 1 mM HCl. The reagent was reacted with the washed NHS resin at room temperature with end over end rotation for at least 2 hours. After washing with 1M Tris pH 9.0, the resin was incubated with the same buffer overnight to block NHS groups that had not reacted with reagent molecules. The resin was washed with 3 column volumes of 20% ethanol, and a resin to 20% ethanol slurry of 1:2 was prepared for storage at 4°C.

### 6.12.5. Results

Examples chromatograms and SDS-PAGE gels (FIG. 22) demonstrate effective purification of both HOP TPR2a from *E. coli* pellets by nickel affinity chromatography followed by gel filtration.

### 6.13. Example 13: Purification of Multichaperone complexes from HOP TPR2a

### 6.13.1. Reagents:

*Homogenization Buffer:* 30 mM sodium phosphate, 1.5 mM magnesium chloride (pH 7.2). *TPR2a Sepharose Conditioning Buffer:* 10 mM sodium phosphate, 5 mM sodium chloride (pH 7.2). *TPR2a Sepharose Elution Buffer:* 10 mM sodium phosphate, 300 mM sodium chloride (pH 7.2). *DEAE Conditioning Buffer:* 10 mM potassium phosphate and 150 mM sodium chloride (pH 7.2). *DEAE Wash Buffer:* 10 mM potassium phosphate and 200 mM sodium chloride (pH 7.2). *DEAE Elution Buffer:* 10 mM potassium phosphate and 300 mM sodium chloride (pH 7.2). 5 M aqueous solution of sodium chloride.

### 6.13.2. Tissue Preparation:

Frozen tissue was removed from freezer and thawed in homogenization buffer at a volume equal to 4x the tissue weight. Once thawed, the tissue was homogenized in a blender and clarified by centrifugation (36,000x g for 1 hour at 4°C). The clarified homogenate was recovered and filtered through 0.45 µm filters. Sodium chloride was added to the filtered homogenate to a concentration of 5 mM to improve the purity of isolated HSPs.

### 6.13.3. Chromatography:

TPR2a-Sepharose was used at a ratio of 0.6 mL of resin to each gram of tissue. A 12 mL column was packed in to a column of 1.0 cm diameter with a slurry of the TPR2a Sepharose resin in TPR2a Sepharose conditioning buffer. The column was conditioned with 10 column volumes of the same buffer. The clarified homogenate was loaded at a flow rate of 1 mL/min and washed with 5 column volumes of the TPR2a Sepharose conditioning buffer. A multichaperone fraction was isolated with a linear gradient to HOP TPR2a Sepharose elution buffer developed over 20 column volumes, and 0.5 column volume fractions were collected. Fractions were pooled according to rough Bradford quantification. In process samples (flow through and wash) were collected for analysis. Isolated HSPs were further purified by DEAE chromatography. The pooled eluate (from the HOP TPR2a) column was diluted with an equal volume of 10 mM potassium phosphate (pH 7.2). This was loaded onto a 1 mL DEAE column that had been conditioned with 10 column volumes of DEAE conditioning buffer. The load was chased with 10 column volumes of the same buffer and washed with 10 column volumes of the DEAE wash buffer. The column was eluted with a linear gradient of DEAE elution buffer developed over 20 column volumes. The eluate was collected in 0.5 mL fractions, which were pooled according to results from a rough Bradford assay. SDS-PAGE and Western blot analysis were performed on in process and the elution pool.

### 6.13.4. Results

Resin immobilized HOP TPR2a effectively isolated a HSP90 rich preparation from mouse tissues (FIG. 23). A relatively low sodium chloride concentration was required to elute HSP90 from this reagent, and polishing by a second chromatography step was required to yield a preparation of reasonable purity (FIG. 23).

## Claims

1. A method for preparing multichaperone-antigen complexes which comprise a combination of at least two different heat shock proteins selected from the group consisting of HSP40, HSP70, HSP90, HSP110, HIP, BIP, and calreticulin, said method comprising the steps of:
(a) contacting a biological sample with a solid phase to which HOP affinity molecules are covalently bound, under conditions such that multichaperone-antigen complexes in the biological sample bind said HOP affinity molecules, wherein the HOP affinity molecules comprise HOP TPR1, having an amino acid sequence as set forth in SEQ ID NO: 1, HOP TPR2a, having an amino acid sequence as set forth in SEQ ID NO: 2. HOP TPR1/2a, having an amino acid sequence as set forth in SEQ ID NO: 3, or a combination or variant of any one or more of the foregoing, wherein a variant is a HOP affinity molecule defined above containing deletions, insertions, substitutions or other modifications relative to the native HOP affinity molecule sequence and retains the specificity of the native HOP affinity molecule to bind heat shock proteins;
(b) removing unbound components in the biological sample away from the solid phase;
(c) eluting multichaperone-antigen complexes from the solid phase; and
(d) recovering the eluted multichaperone-antigen complexes.

2. The method of claim 1, wherein the biological sample is a mammalian cell extract, a human cell extract, a tumor cell extract, an infected cell extract, an extract of an engineered cell, or wherein the biological sample is flow-through resulting from contacting a tumor cell extract, a pathogen-infected cell extract or an extract of cells transfected with and expressing a nucleic acid encoding a tumor associated antigen or a tumor specific antigen or infectious disease antigen, containing cellular proteins, with a solid phase to which is bound a binding partner for a heat shock protein.

3. The method of claim 2, wherein said solid phase to which is bound said binding partner is an anti-gp96 immunoaffinity column and said heat shock protein to which said binding partner binds is gp96.

4. The method of any one of claims 1 to 3, wherein the multichaperone-antigen complexes comprise, human heat shock proteins.

5. The method of any one of claims 1 to 4, wherein the solid phase
(i) comprises beads; or
(ii) is a membrane; or
(iii) has a surface comprising polycarbonate, polystyrene, polypropylene, polyethylene, glass, nitrocellulose, dextran, nylon, polyacrylamide or agarose; or
(iv) is a mixed resin bed comprising a first bead/resin to which a HOP affinity molecule comprising HOP TPR1 or a variant thereof is covalently bound and a second bead/resin to which a HOP affinity molecule comprising HOP TPR1/2a or a variant thereof is covalently bound; or
(v) is a mixed resin bed comprising (a) a HOP affinity molecule comprising HOP TPR1 or a variant thereof; and (b) a HOP affinity molecule comprising HOP TPR1/2a or a variant thereof; and wherein the eluting step comprises eluting with a buffered solution containing 20 mM Tris and 500 mM NaCl, at pH 9.

6. The method of claim 5, wherein the beads of (i) are packed in a column or are magnetic.

7. The method of claim 5, wherein the beads of (i) are used in a slurry.

8. The method of any one of claims 1 to 7, wherein said HOP affinity molecules
(i) are attached via a bifunctional crosslinker to the solid phase; or
(ii) comprise a HOP affinity fragment or variant thereof selected from the group consisting of HOP TPR1 or a variant thereof, HOP TPR2a or a variant thereof, HOP TPR1/2a or a variant thereof, and a combination of any one or more of the foregoing; or
(iii) comprise a mammalian HOP affinity fragment or variant thereof; or
(iv) comprise a human HOP affinity fragment or variant thereof; or
(v) comprise a HOP affinity fragment or variant thereof that is present as a concatamer of two or more of HOP TPR1 or a variant thereof, HOP TPR2a or a variant thereof, and/or HOP TPR1/2a or a variant thereof; or
(vi) comprise a HOP affinity fragment or variant thereof that is present as a fusion protein of two or more of HOP TPR1 or a variant thereof, HOP TPR2a or a variant thereof, and/or HOP TPR1/2a or a variant thereof; or
(vii) do not comprise a wild-type HOP protein.

9. The method of any one of claims 1 to 8, wherein
(i) the eluting step comprises eluting with a buffered solution containing 150 mM to 1.5M sodium chloride at pH 3 to pH 11; or
(ii) the HOP affinity molecule comprises HOP TPR1 or a variant thereof and the eluting step comprises eluting with a buffered solution containing 500 mM NaCl at pH 9; or
(iii) the HOP affinity molecule comprises HOP TPR2a or a variant thereof and the eluting step comprises eluting with a buffered solution containing 300 mM NaCl at pH 7.2; or
(iv) the wherein the HOP affinity molecule comprises HOP TPR1/2a or a variant thereof and the eluting step comprises eluting with a buffered solution containing 500 mM NaCl at pH 7.2; or
(v) the HOP affinity molecule comprises HOP TPR1/2a or a variant thereof and the eluting step comprises eluting with a buffered solution containing 500 mM NaCl at pH 9.

10. The method of any one of claims 1 to 9, further comprising combining the recovered multichaperone-antigen complexes with purified heat shock protein-antigen complexes or purified gp96-antigen complexes.

11. A method comprising
(i) contacting a first solid phase to which is bound a binding partner for a heat shock protein with a human tumor cell extract or human infected cell extract or an extract of cells transfected with and expressing a nucleic acid encoding a tumor associated antigen or a tumor specific antigen or infectious disease antigen under conditions such that gp96-antigen complexes in the extract bind the first solid phase, wherein the first solid phase is an anti-gp96 immunoaffinity column or an anti-gp96 scFv column;
(ii) collecting the flow through from said first solid phase;
(iii) washing said first solid phase;
(iv) eluting gp96- antigen complexes from said first solid phase;
(v) contacting said flow through collected in step (ii) with a second solid phase to which HOP affinity molecules are covalently bound, under conditions such that multichaperone-antigen complexes in the biological sample bind said HOP affinity molecules, wherein the multichaperone-antigen complexes comprise a combination of at least two different heat shock proteins selected from the group consisting of HSP40, HSP70, HSP90, HSP110, HIP, BIP, and calreticulin; and wherein the HOP affinity molecules comprise HOP TPR1, having an amino acid sequence as set forth in SEQ ID NO: 1, HOP TPR2a, having an amino acid sequence as set forth in SEQ ID NO: 2, HOP TPR1/2a, having an amino acid sequence as set forth in SEQ ID NO: 3, or a combination or variant of any one or more of the foregoing wherein a variant is a HOP affinity molecule defined above containing deletions, insertions, substitutions or other modifications relative to the native HOP affinity molecule sequence and retains the specificity of the native HOP affinity molecule to bind heat shock proteins;
(vi) removing unbound components in the biological sample away from the second solid phase;
(vii) eluting multichaperone-antigen complexes from the second solid phase; and
(viii) combining said gp96-antigen complexes eluted in step (iv) with the multichaperone-antigen complexes eluted in step (vii).

## Patentansprüche

1. Verfahren zum Erzeugen von Multichaperone-Antigenkomplexen, die eine Kombination aus wenigstens zwei verschiedenen Hitzeschockproteinen umfassen, ausgewählt aus der Gruppe bestehend aus HSP40, HSP70, HSP90, HSP110, HIP, BIP und Calreticulin, wobei das Verfahren die folgenden Schritte umfasst:
(a) Inberührungbringen einer biologischen Probe mit einer festen Phase, an die HOP-Affinitätsmoleküle kovalent gebunden sind, unter Bedingungen, sodass Multichaperone-Antigenkomplexe in der biologischen Probe die HOP-Affinitätsmoleküle binden, wobei die HOP-Affinitätsmoleküle HOP-TPR1 mit einer Aminosäuresequenz nach SEQ ID NO: 1, HOP-TPR2a mit einer Aminosäuresequenz nach SEQ ID NO: 2, HOP-TPR1/2a mit einer Aminosäuresequenz nach SEQ ID NO: 3 oder eine Kombination oder eine Variante eines oder mehrerer der obenstehenden umfassen, wobei eine Variante ein zuvor definiertes HOP-Affinitätsmolekül mit Deletierungen, Insertionen, Substitutionen oder anderen Veränderungen im Vergleich zu der nativen HOP-Affinitätsmolekülsequenz ist, und das die Spezifizität des nativen HOP-Affinitätsmoleküls beibehält, Hitzeschockproteine zu binden;
(b) Entfernen von ungebundenen Komponenten in der biologischen Probe, entfernt von der festen Phase;
(c) Eluieren von Multichaperone-Antigenkomplexen aus der festen Phase; und
(d) Wiedergewinnen der eluierten Multichaperone-Antigenkomplexe.

2. Verfahren nach Anspruch 1, wobei die biologische Probe ein Säugerzellextrakt, ein Extrakt einer humanen Zelle, ein Tumorzellextrakt, ein Extrakt einer infizierten Zelle oder ein Extrakt einer modifizierten Zelle ist, oder wobei die biologische Probe Durchfluss ist, der sich aus dem Inberührungbringen eines Tumorzellextrakts, eines Extrakts einer mit einem Pathogen infizierten Zelle oder eines Extrakts von mit einer für ein tumorassoziiertes Antigen oder ein tumorspezifisches Antigen oder Infektionskrankheitantigen codierenden Nukleinsäure transfizierten und diese exprimierenden Zellen, Zellproteine enthaltend, mit einer festen Phase, an die ein Bindungspartner für ein Hitzeschockprotein gebunden ist, ergibt.

3. Verfahren nach Anspruch 2, wobei die feste Phase, an die der Bindungspartner gebunden ist, eine Anti-gp96-Immunoaffinitätssäule ist, und das Hitzeschockprotein, an welches der Bindungspartner bindet, gp96 ist.

4. Verfahren nach einem der Ansprüche 1 bis 3, wobei die Multichaperone-Antigenkomplexe humane Hitzeschockproteine umfassen.

5. Verfahren nach einem der Ansprüche 1 bis 4, wobei die feste Phase:
(i) Kügelchen umfasst; oder
(ii) eine Membran ist; oder
(iii) eine Oberfläche aufweist, die Polycarbonat, Polystyrol, Polypropylen, Polyethylen, Glas, Nitrocellulose, Dextran, Nylon, Polyacrylamid oder Agarose umfasst; oder
(iv) ein gemischtes Harzbett ist, umfassend ein erstes Kügelchen/Harz, an das ein HOP-Affinitätsmolekül, das HOP-TPR1 oder eine Variante davon umfasst, kovalent gebunden ist, und ein zweites Kügelchen/Harz, an das ein HOP-Affinitätsmolekül, das HOP-TPR1/2a oder eine Variante davon umfasst, kovalent gebunden ist; oder
(v) ein gemischtes Harzbett ist, das Folgendes umfasst: (a) ein HOP-Affinitätsmolekül, umfassend HOP-TPR1 oder eine Variante davon; und (b) ein HOP-Affinitätsmolekül, umfassend HOP-TPR1/2a oder eine Variante davon; und wobei der Eluierungsschritt Eluieren mit einer gepufferten Lösung umfasst, die 20 mM Tris und 500 mM NaCl mit einem pH-Wert von 9 enthält.

6. Verfahren nach Anspruch 5, wobei die Kügelchen aus (i) in einer Säule gepackt sind oder magnetisch sind.

7. Verfahren nach Anspruch 5, wobei die Kügelchen aus (i) in einer Aufschlämmung eingesetzt werden.

8. Verfahren nach einem der Ansprüche 1 bis 7, wobei die HOP-Affinitätsmoleküle:
(i) über einen bifunktionellen Vernetzer an der festen Phase befestigt sind; oder
(ii) ein HOP-Affinitätsfragment oder eine Variante davon umfassen, ausgewählt aus der Gruppe bestehend aus HOP-TPR1 oder einer Variante davon, HOP-TPR2a oder einer Variante davon, HOP-TPR1/2a oder einer Variante davon, und einer Kombination aus einem oder mehreren der obenstehenden; oder
(iii) ein Säuger-HOP-Affinitätsfragment oder eine Variante davon umfassen; oder
(iv) ein humanes HOP-Affinitätsfragment oder eine Variante davon umfassen; oder
(v) ein HOP-Affinitätsfragment oder eine Variante davon umfassen, das/die als ein Konkatamer aus zwei oder mehr HOP-TPR1 oder einer Variante davon, HOP-TPR2a oder einer Variante davon und/oder HOP-TPR1/2a oder einer Variante davon vorliegt; oder
(vi) ein HOP-Affinitätsfragment oder eine Variante davon umfassen, das/die als ein Fusionsprotein aus zwei oder mehr HOP-TPR1 oder einer Variante davon, HOP-TPR2a oder einer Variante davon und/oder HOP-TPR1/2a oder einer Variante davon vorliegt; oder
(vii) kein Wildtyp-HOP-Protein umfassen.

9. Verfahren nach einem der Ansprüche 1 bis 8, wobei:
(i) der Eluierungsschritt Eluieren mit einer gepufferten Lösung umfasst, die 150 mM bis 1,5 M Natriumchlorid mit einem pH-Wert von 3 bis 11 enthält; oder
(ii) das HOP-Affinitätsmolekül HOP-TPR1 oder eine Variante davon umfasst und wobei der Eluierungsschritt das Eluieren mit einer gepufferten Lösung, die 500 mM NaCl mit einem pH-Wert von 9 enthält, umfasst; oder
(iii) das HOP-Affinitätsmolekül HOP-TPR2a oder eine Variante davon umfasst und wobei der Eluierungsschritt das Eluieren mit einer gepufferten Lösung, die 300 mM NaCl mit einem pH-Wert von 7,2 enthält, umfasst; oder
(iv) wobei das HOP-Affinitätsmolekül HOP-TPR1/2a oder eine Variante davon umfasst und wobei der Eluierungsschritt das Eluieren mit einer gepufferten Lösung, die 500 mM NaCl mit einem pH-Wert von 7,2 enthält, umfasst; oder
(v) das HOP-Affinitätsmolekül HOP-TPR1/2a oder eine Variante davon umfasst und wobei der Eluierungsschritt das Eluieren mit einer gepufferten Lösung, die 500 mM NaCl mit einem pH-Wert von 9 enthält, umfasst.

10. Verfahren nach einem der Ansprüche 1 bis 9, ferner umfassend das Kombinieren der wiedergewonnenen Multichaperone-Antigenkomplexe mit bereinigten Hitzeschockprotein-Antigenkomplexen oder bereinigten gp96-Antigenkomplexen.

11. Verfahren, Folgendes umfassend:
(i) Inberührungbringen einer ersten festen Phase, an die ein Bindungspartner für ein Hitzeschockprotein gebunden ist, mit einem humanen Tumorzellextrakt oder einem Extrakt einer humanen infizierten Zelle oder einem Extrakt von mit einer für ein tumorassozüertes Antigen oder ein tumorspezifisches Antigen oder Infektionskrankheitantigen codierenden Nukleinsäure transfizierten und diese exprimierenden Zellen, unter Bedingungen, sodass die gp96-Antigenkomplexe in dem Extrakt an die erste feste Phase binden, wobei die erste feste Phase eine Anti-gp96-Immunoaffinitätssäule oder eine Anti-gp96-scFv-Säule ist;
(ii) Sammeln des Durchflusses aus der ersten festen Phase;
(iii) Waschen der ersten festen Phase;
(iv) Eluieren von gp96-Antigenkomplexen aus der ersten festen Phase;
(v) Inberührungbringen des in Schritt (ii) gesammelten Durchflusses mit einer zweiten festen Phase, an die HOP-Affinitätsmoleküle kovalent gebunden sind, unter Bedingungen, sodass Multichaperone-Antigenkomplexe in der biologischen Probe die HOP-Affinitätsmoleküle binden, wobei die Multichaperone-Antigenkomplexe eine Kombination aus wenigstens zwei verschiedenen Hitzeschockproteinen umfassen, ausgewählt aus der Gruppe bestehend aus HSP40, HSP70, HSP90, HSP110, HIP, BIP und Calreticulin; und wobei die HOP-Affinitätsmoleküle HOP-TPR1 mit einer Aminosäuresequenz nach SEQ ID NO: 1, HOP-TPR2a mit einer Aminosäuresequenz nach SEQ ID NO: 2, HOP-TPR1/2a mit einer Aminosäuresequenz nach SEQ ID NO: 3 oder eine Kombination oder eine Variante eines oder mehrerer der obenstehenden umfassen, wobei eine Variante ein zuvor definiertes HOP-Affinitätsmolekül mit Deletierungen, Insertionen, Substitutionen oder anderen Veränderungen im Vergleich zu der nativen HOP-Affinitätsmolekülsequenz ist, und das die Spezifizität des nativen HOP-Affinitätsmoleküls beibehält, Hitzeschockproteine zu binden;
(vi) Entfernen von ungebundenen Komponenten in der biologischen Probe, entfernt von der zweiten festen Phase;
(vii) Eluieren von Multichaperone-Antigenkomplexen aus der zweiten festen Phase; und
(viii) Kombinieren der in Schritt (iv) eluierten gp96-Antigenkomplexe mit den in Schritt (vii) eluierten Multichaperone-Antigenkomplexen.

## Revendications

1. Procédé de préparation de complexes multichapérone-antigène comprenant une combinaison d'au moins deux protéines de choc thermique différentes choisies dans le groupe constitué par HSP40, HSP70, HSP90, HSP110, HIP, BIP et la calréticuline, ledit procédé comprenant les étapes de :
(a) mise en contact d'un échantillon biologique avec une phase solide à laquelle les molécules d'affinité HOP sont liées de façon covalente, dans des conditions telles que les complexes multichapérone-antigène dans l'échantillon biologique se lient auxdites molécules d'affinité HOP, dans lequel les molécules d'affinité HOP comprennent HOP TPR1, ayant une séquence d'acides aminés telle que décrite dans SÉQ ID n° : 1, HOP TPR2a, ayant une séquence d'acides aminés telle que décrite dans SÉQ ID n° : 2, HOP TPR1/2a, ayant une séquence d'acides aminés telle que décrite dans SÉQ ID n° : 3, ou une combinaison ou variante de l'une quelconque ou de plusieurs des précédentes, dans lequel une variante est une molécule d'affinité HOP définie ci-dessus contenant des délétions, des insertions, des substitutions ou d'autres modifications par rapport à la séquence de molécule d'affinité HOP native et conserve la spécificité de liaison de la molécule d'affinité HOP native aux protéines de choc thermique ;
(b) élimination des composants non liés dans l'échantillon biologique de la phase solide ;
(c) élution des complexes multichapérone-antigène de la phase solide ; et
(d) récupération des complexes multichapérone-antigène élués.

2. Procédé selon la revendication 1, dans lequel l'échantillon biologique est un extrait de cellule mammifère, un extrait de cellule humaine, un extrait de cellule tumorale, un extrait de cellule infectée, un extrait de cellule génétiquement modifiée, ou dans lequel l'échantillon biologique est un flux continu résultant de la mise en contact d'un extrait de cellule tumorale, d'un extrait de cellule infectée par un pathogène ou d'un extrait de cellules transfectées avec et exprimant un acide nucléique codant pour un antigène associé à une tumeur ou un antigène spécifique d'une tumeur ou un antigène de maladie infectieuse, contenant des protéines cellulaires, avec une phase solide à laquelle est lié un partenaire de liaison pour une protéine de choc thermique.

3. Procédé selon la revendication 2, dans lequel ladite phase solide à laquelle est lié ledit partenaire de liaison est une colonne d'immunoaffinité anti-gp96 et ladite protéine de choc thermique à laquelle est lié ledit partenaire de liaison est gp96.

4. Procédé selon l'une quelconque des revendications 1 à 3, dans lequel les complexes multichapérone-antigène comprennent des protéines de choc thermique humaines.

5. Procédé selon l'une quelconque des revendications 1 à 4, dans lequel la phase solide
(i) comprend des billes ; ou
(ii) est une membrane ; ou
(iii) a une surface comprenant du polycarbonate, du polystyrène, du polypropylène, du polyéthylène, du verre, de la nitrocellulose, du dextrane, du nylon, du polyacrylamide ou l'agarose ; ou
(iv) est un lit de résine mixte comprenant une première bille/résine à laquelle une molécule d'affinité HOP comprenant HOP TPR1 ou une variante de celle-ci est liée de façon covalente et une deuxième bille/résine à laquelle une molécule d'affinité HOP comprenant HOP TPR1/2a ou une variante de celle-ci est liée de façon covalente ; ou
(v) est un lit de résine mixte comprenant (a) une molécule d'affinité HOP comprenant HOP TPR1 ou une variante de celle-ci ; et (b) une molécule d'affinité HOP comprenant HOP TPR1/2a ou une variante de celle-ci ; et dans lequel l'étape d'élution comprend l'élution avec une solution tamponnée contenant 20 mM de Tris et 500 mM de NaCl, à pH 9.

6. Procédé selon la revendication 5, dans lequel les billes de (i) sont empilées dans une colonne ou sont magnétiques.

7. Procédé selon la revendication 5, dans lequel les billes de (i) sont utilisées dans une barbotine.

8. Procédé selon l'une quelconque des revendications 1 à 7, dans lequel lesdites molécules d'affinité HOP
(i) sont liées à la phase solide par le biais d'un agent de réticulation bifonctionnel ; ou
(ii) comprennent un fragment d'affinité HOP ou une variante de celui-ci, choisi dans le groupe constitué par HOP TPR1 ou une variante de celui-ci, HOP TPR2a ou une variante de celui-ci, HOP TPR1/2a ou une variante de celui-ci, et une combinaison de l'une quelconque ou de plusieurs des précédents ; ou
(iii) comprennent un fragment d'affinité HOP mammifère ou une variante de celui-ci ; ou
(iv) comprennent un fragment d'affinité HOP humain ou une variante de celui-ci ; ou
(v) comprennent un fragment d'affinité HOP ou une variante de celui-ci qui est présent sous forme de concatémère de deux ou plus de HOP TPR1 ou d'une variante de celui-ci, de HOP TPR2a ou d'une variante de celui-ci et/ou de HOP TPR1/2a ou d'une variante de celui-ci ; ou
(vi) comprennent un fragment d'affinité HOP ou une variante de celui-ci qui est présent sous forme de protéine de fusion de deux ou plus de HOP TPR1 ou d'une variante de celui-ci, de HOP TPR2a ou d'une variante de celui-ci et/ou de HOP TPR1/2a ou d'une variante de celui-ci ; ou
(vii) ne comprennent pas de protéine HOP de type sauvage.

9. Procédé selon l'une quelconque des revendications 1 à 8, dans lequel
(i) l'étape d'élution comprend l'élution avec une solution tamponnée contenant 150 mM à 1,5 M de chlorure de sodium à pH 3 à pH 11 ; ou
(ii) la molécule d'affinité HOP comprend HOP TPR1 ou une variante de celle-ci et l'étape d'élution comprend l'élution avec une solution tamponnée contenant 500 mM de NaCl à pH 9 ; ou
(iii) la molécule d'affinité HOP comprend HOP TPR2a ou une variante de celle-ci et l'étape d'élution comprend l'élution avec une solution tamponnée contenant 300 mM de NaCl à pH 7,2 ; ou
(iv) la molécule d'affinité HOP comprend HOP TPR1/2a ou une variante de celle-ci et l'étape d'élution comprend l'élution avec une solution tamponnée contenant 500 mM de NaCl à pH 7,2 ; ou
(v) la molécule d'affinité HOP comprend HOP TPR1/2a ou une variante de celle-ci et l'étape d'élution comprend l'élution avec une solution tamponnée contenant 500 mM de NaCl à pH 9.

10. Procédé selon l'une quelconque des revendications 1 à 9, comprenant en outre la combinaison des complexes multichapérone-antigène récupérés avec des complexes protéine thermique-antigène purifiés ou des complexes gp96-antigène purifiés.

11. Procédé comprenant
(i) la mise en contact d'une première phase solide à laquelle est lié un partenaire de liaison pour une protéine de choc thermique avec un extrait de cellule tumorale humaine ou un extrait de cellule infectée humaine ou un extrait de cellules transfectées avec et exprimant un acide nucléique codant pour un antigène associé à une tumeur ou un antigène spécifique d'une tumeur ou un antigène de maladie infectieuse dans des conditions telles que les complexes gp96-antigène dans l'extrait se lient à la première phase solide, dans lequel la première phase solide est une colonne d'immunoaffinité anti-gp96 ou une colonne scFv anti-gp96 ;
(ii) la récupération du flux continu provenant de ladite première phase solide ;
(iii) le rinçage de ladite première phase solide ;
(iv) l'élution des complexes gp96-antigène de ladite première phase solide ;
(v) la mise en contact dudit flux continu récupéré dans l'étape (ii) avec une deuxième phase solide à laquelle les molécules d'affinité HOP sont liées de façon covalente, dans des conditions telles que les complexes multichapérone-antigène dans l'échantillon biologique se lient auxdites molécules d'affinité HOP, dans lequel les complexes multichapérone-antigène comprennent une combinaison d'au moins deux protéines de choc thermique différentes choisies dans le groupe constitué par HSP40, HSP70, HSP90, HSP110, HIP, BIP et la calréticuline ; et dans lequel les molécules d'affinité HOP comprennent HOP TPR1, ayant une séquence d'acides aminés telle que décrite dans SÉQ ID n° : 1, HOP TPR2a, ayant une séquence d'acides aminés telle que décrite dans SÉQ ID n° : 2, HOP TPR1/2a, ayant une séquence d'acides aminés telle que décrite dans SÉQ ID n° : 3, ou une combinaison ou variante de l'une quelconque ou de plusieurs des précédentes, dans lequel une variante est une molécule d'affinité HOP définie ci-dessus contenant des délétions, des insertions, des substitutions ou d'autres modifications par rapport à la séquence de molécule d'affinité HOP native et conserve la spécificité de liaison de la molécule d'affinité HOP native aux protéines de choc thermique ;
(vi) l'élimination des composants non liés dans l'échantillon biologique de la deuxième phase solide ;
(vii) l'élution des complexes multichapérone-antigène de la deuxième phase solide ; et
(viii) la combinaison desdits complexes gp96-antigène élués dans l'étape (iv) avec les complexes multichapérone-antigène élués dans l'étape (vii).
